# EUROPÄISCHE PATENTANMELDUNG

(11) **EP 1 602 930 A2**
(43) Veröffentlichungstag der Anmeldung: **07.12.2005**
(21) Anmeldenummer: 04090322.1
(22) Anmeldetag: 20.08.2004
(51) Int. Cl.: G01N 33/574, G01N 33/566

(54) **Verwendungen von an GPR49 bindenden Substanzen zur Diagnose und Behandlung von Krebs**

(30) Priorität: 22.08.2003 DE 10339820
(71) Anmelder: Hinzmann, Bernd, Dr., 13127 Berlin (DE); Stein, Dr., Anke, 14974 Ludwigsfelde (DE); Staub, Dr., Eike, 13189 Berlin (DE); Heiden, Esmeralda, Dr., 10589 Berlin (DE); Klaman, Dr., Irina, 10318 Berlin (DE); Dahl, Dr., Edgar, 4851 Gemmenich (BE)
(72) Erfinder: Stein, Anke, 14974 Ludwigsfelde (DE); Staub, Eike, 13189 Berlin (DE); Weber, Birgit, Dr., 81927 München (DE); Heiden, Esmeralda, Dr., 10589 Berlin (DE); Klaman, Irina, 10318 Berlin (DE); Dahl, Edgar Institut für Pathologie Uni-Klinikum, 52074 Aachen (DE); Hinzmann, Bernd, 13127 Berlin (DE)

(57) **Zusammenfassung**

Die Erfindung betrifft Verwendungen von GPR49 zur Diagnose und Behandlung von Krebs, insbesondere des Colon-, Uterus- und/oder Rectumkarzinoms, sowie zum Screenen nach Substanzen für solche Zwecke.

## Beschreibung

### Gebiet der Erfindung

Die Erfindung betrifft neue Verwendungen von GPR49 oder daraus abgeleiteten Sequenzen zum Screenen nach daran bindenden Substanzen, sowie die Verwendung von an GPR49 bindenden Substanzen zur Diagnose und/oder Behandlung von Krebs.

### Hintergrund der Erfindung und Stand der Technik

GPR49, auch als LGR5 oder HG38 bezeichnet, wurde ursprünglich als ein "orphan" G-Protein gekoppelter 7 transmembraner Rezeptor isoliert. Obwohl der GPR49 spezifische Ligand noch nicht identifiziert werden konnte, lassen Sequenzanalysen auf eine Zugehörigkeit zur Glycoprotein Hormone Rezeptor Familie schließen. Merkmal dieser Subfamilie ist eine große N-terminale Ektodomäne. Die sich wiederholenden LRR Motive werden für die Ligandenerkennung verantwortlich gemacht. Die 17 LRR in GPR49, verglichen mit den 9 der anderen Familienmitglieder, lassen ein noch größeres Glycoprotein als TSH, FSH oder LH als Liganden vermuten.

Die C-terminale Endodomäne ist im Cytoplasma lokalisiert und weist sowohl potentielle Phosphorylierungsstellen als auch mögliche Erkennungssequenzen für Proteine mit SH2 und SH3 Domänen auf. Diese sind möglicherweise an einer Verbindung zu einer von G-Proteinen verschiedenen Signaltransduktion beteiligt (Heu et al. 1998, Molecular Endocrinology 12(12):1830-1845; McDonald et al. 1998, Biochem. & Biophys. Research Communications 247:266-270).

In der Literaturstelle WO99/15660 wird nicht nur die Klonierung der cDNA aus humaner Placenta mRNA beschrieben, sondern auch die Verteilung der mRNA in normalem Gewebe über Multiple Tissue Northern Hybridisierung. Ein deutlicher Nachweis erfolgte für Placenta, Skelettmuskelgewebe und spezielle Subtypen des Gehirns, insbesondere Corpus callosum.

Yamamoto et al. (Hepatology 37(3):528-533 (2003)) konnten eine Hochregulierung der GPR49 mRNA in Lebergeweben in Zusammenhang mit mutiertem β-Catenin stellen. β-Catenin ist sowohl an Cadherin-vermittelter Zell-Zell-Adhäsion als auch an der Wnt-Signalkaskade beteiligt. Die Authoren schliessen auf eine Genaktivierung von GRP49 durch die Wnt-Signalkaskade und sprechen von einem möglichen Potential als therapeutisches Target in der Behandlung von Leberzell-Karzinoma.

Krebs ist eine meist letal verlaufende Erkrankung mit zunehmender Inzidenz. Daher ist es wünschenwert, verbesserte Ansätze zur Diagnose und Therapie von Krebserkrankungen zur Verfügung zu stellen.

### Technisches Problem der Erfindung

Der Erfindung liegt daher das technische Problem zugrunde, pharmazeutische Zusammensetzungen zur Diagnose, auch zur Verlaufs- bzw. Progressionsprognose, und/oder zur Behandlung von Krebs, insbesondere von Colon-, Uterus- und/oder Rectumtumoren, anzugeben sowie Mittel zu deren Identifizierung.

Grundzüge der Erfindung und bevorzugte Ausführungsformen.

Zur Lösung dieses technischen Problems lehrt die Verwendung einer für GPR49 codierenden Nukleinsäure und/oder eines GPR49 Peptids oder Proteins zur Detektion von Krebs, insbesondere von Colon-, Uterus- und/oder Rectumtumoren oder zur Detektion eines Risikos der Erkrankung an einem solchen Karzinom oder zur Detektion eines Risikos einer Progression eines solchen Karzinoms, wobei eine Gewebeprobe, insbesondere eine Colon-, Uterus und/oder Rectum-Gewebeprobe, auf Transkription oder Übertranskription von GPR49 RNA oder auf Expression oder Überexpression eines GPR49 Proteins untersucht wird. Eine an für GPR49 codierende Nukleinsäure oder eine an GPR49 Protein oder Peptid bindende Detektorsubstanz, vorzugsweise enthaltend eine Reportergruppe, kann verwendet werden, wobei Bindung besagter Nukleinsäure und/oder besagten Proteins oder Peptids an die Detektorsubstanz halbquantitativ oder quantitativ detektiert wird. In diesem Zusammenhang lehrt die Erfindung weiterhin ein Testsystem zur (in vitro) Detektion eines vorstehend genannten Karzinoms oder eines Risikos der Erkrankung hieran oder der Progressionsprognose, enthaltend Mittel zur quantitativen Messung der Expression von GPR49 in Gewebeproben, wobei diese Mittel beispielsweise Mittel zur Amplifikation und spezifischen Detektion von GPR49 RNA und/oder eine Detektorsubstanz, insbesondere spezifisch für GPR49 Protein, sein können.

Die Erfindung lehrt weiterhin die Verwendung einer GPR49 RNA oder eines GPR49 Proteins oder Peptids zum Screenen nach daran bindenden Substanzen, insbesondere prospektiven Wirkstoffen zur Modulierung, insbesondere Inhibierung, von besagter RNA oder besagtem Protein oder Peptid, oder prospektiven Detektorsubstanzen, wobei eine prospektive Substanz oder eine Mischung solcher prospektiver Substanzen mit besagter RNA oder besagtem Protein oder Peptid kontaktiert wird, wobei mit einem Bindungsassay Bindungsereignisse festgestellt werden, und wobei eine bindende prospektive Substanz, ggf. nach Dekonvolutierung, selektiert wird. In diesen Zusammenhängen lehrt die Erfindung weiterhin ein Screeningsystem zur Ermittlung von für die Behandlung von vorstehenden Tumorerkrankungen geeigneten Wirksubstanzen enthaltend eine GPR49 Nukleinsäure oder ein GPR49 Protein bzw. Peptid, Mittel zur Bestimmung von (in vitro) Bindungsereignissen an die GPR49 Nukleinsäure oder an das GPR49 Protein bzw. Peptid, und/oder Mittel zur Bestimmung der (in vitro) Aktivität von GPR49 Protein. Hierbei kann GPR49 in einem zellfreien oder einem zellbasierten System, letzteres insbesondere aufweisend Zellen aus vorstehend genannten Geweben bzw. eine hieraus entwickelte Zelllinie, vorliegen. Mittel zur Bestimmung von Bindungsereignissen können beispielsweise natürlicherweise in normalen oder in Tumorzellen z.B. an GPR49 Protein bindende Substanzen bzw. Assoziationspartner umfassen, wobei über deren (freie) Konzentration bzw. Konzentrationsänderung bei Zugabe prospektiver Wirksubstanzen und/oder Detektorsubstanzen eine kompetitive Bindung einer bindenden Wirk- oder Detektorsubstanz bestimmt wird. Solche Mittel können aber auch physikalische bzw. physikalisch-chemische Methoden umfassen, wie beispielsweise Röntgenstrukturanalyse und/oder NMR, insbesondere zweidimensionale 1H/1H oder 15N/1H oder 14C/1H Korrelationsspektroskopie. Hierbei werden Spektren vor und nach der Zugabe einer prospektiven Wirk- oder Detektorsubstanz miteinander verglichen und im Falle von Änderungen ist ein Bindungsereignis festgestellt. Es kann mit Spektren oder dergleichen entweder von GPR49 oder der prospektiven Substanz oder mit einer Kombination aus beidem gearbeitet werden. Selbstverständlich sind auch alle anderen fachüblichen Methoden der Bestimmung von Bindungsereignissen und/oder Proteinaktivitäten einsetzbar. Beispielsweise kann eine prospektive Substanz (oder mehrere Substanzen, räumlich voneinander getrennt) immobilisiert sein, wobei dann markiertes GPR49 aufgetragen wird. Ein Bindungsereignis wird dann nach Auftrag und folgender Spülung durch Detektion, ggf. ortlich aufgelöst, der Markierung gebundenen FABP4s festgestellt. Bindungsereignisse können auch ohne Markierung eines der Bindungspartner mittels der dem Fachmann geläufigen Biacore Technologie (Oberflächenplasmonen Resonanz)detektiert werden. Umgekehrt kann GPR49 immobilisiert sein und es wird eine markierte prospektive Substanz oder eine Mischung hieraus aufgetragen. Bindungsereignisse werden analog der vorstehenden Variante festgestellt.

Die Erfindung lehrt schließlich die Verwendung einer GPR49 inhibierenden oder daran bindenden Substanz zur Herstellung einer pharmazeutischen Zusammensetzung zur Behandlung und/oder Diagnose von Krebs, insbesondere Colon-, Uterus- und/oder Rectumtumoren.

Die Substanz kann ein Antikörper sein, welcher durch Immunisierung eines nicht-menschlichen Säugetiers mit einem GPR49 Peptid oder Protein, mit hierfür codierender cDNA transfizierte Zellen, cDNA Immunisierung (Genovac), mit endogen ein solches Peptid oder Protein exprimierenden Tumorzellen, oder mit rekombinant hergestellten GPR49 Peptiden oder Proteinen, erhältlich ist, oder ein Phage-Display-Antikörper sein. Die Substanz kann aber auch eine Mimikryverbindung eines Antikörpers gegen ein GPR49 Peptid oder Protein sein. Die Substanz kann schließlich ein Aptamer, eine antisense RNA, ein Ribozym oder eine siRNA gegen GPR49 Nukleinsäuren sein. Die Substanz kann zusätzlich eine zytotoxische und/oder immunstimulierende Komponente tragen.

Bevorzugt ist es, wenn die vorstehenden, an GPR49 Protein bindenden Substanzen in der Verwendung zu therapeutischen Zwecken spezifisch an das GPR49 Protein binden und es in seiner biologischen Aktivität modulieren. Dies ist nicht erforderlich im Falle der Fusion bzw. Verbindung der Substanz mit einer zytotoxischen Komponente. Dies ist weiterhin nicht erforderlich, wenn die Substanz der Gewinnung eines anti-idiotypischen Antikörpers dient, welcher vom Immunsystem eines Patienten aufgrund seiner nicht-humanisierten Form als körperfremd erkannt wird und dem Immunsystem ansonsten ein GPR49-Antigen präsentiert.

Die pharmazeutische Zusammensetzung kann zur beliebigen Applikation, beispielsweise i.v. oder i.p. Injektion, hergerichtet sein. Eine Herrichtung zur lokalen Applikation in Tumorzellen enthaltendem Gewebe wird sich empfehlen im Falle des Einsatzes einer zytotoxischen Komponente.

Die Erfindung läßt sich im Rahmen eines Verfahrens zur Diagnose bzw. (Progressions-) Prognose einer Tumorerkrankung verwenden, wobei eine Detektorsubstanz in einer Ausführungsform mit einer Reportergruppe in zu untersuchendes Gewebe, ggf. in vitro nach Gewebeentnahme, appliziert wird, wobei das zu untersuchende Gewebe dann einer Detektionsverfahrenstufe unterworfen wird, welche sensitiv für die Reportergruppe ist, und wobei im Fall der Detektion eines definierten Mindestwertes der Reportergruppe im Gewebe das Gewebe als Tumorzellen enthaltend qualifiziert bzw. als progressionsgefährdet oder nicht progressionsgefährdet eingestuft wird, sowie eines Verfahrens zur Behandlung einer Krebserkrankung, wobei eine erfindungsgemäße pharmazeutische Zusammensetzung in einer physiologisch wirksamen Dosis einem Patienten dargereicht wird. Im Falle der Diagnose bzw. Progressionsprognose kann zusätzlich oder alternativ eine Gewebeprobe mit einem erfindungsgemäßen Testsystem auf GPR49 Expression untersucht werden.

Die Erfindung beruht insbesondere auf der Erkenntnis, daß GPR49 in Colon-, Rectum- und/oder Uterusgeweben unterschiedlich exprimiert wird, i.e. in besagten Tumorgeweben ist die Expression im Falle solcher Karzinome höher, verglichen mit normalen Zellen gleichen Gewebes und der daraus herleitbaren technische Lehre, daß GPR49 als Zielmolekül bei der Diagnostik und Therapie bzw. Prophylaxe besagter Tumorerkrankungen eingesetzt werden kann. GPR49 kann also als spezifischer Marker zur Identifizierung von Tumorzellen in den besagten Tumorgeweben dienen. Es ist erkannt worden, dass pathologisch normale Zellen mit einer erhöhten Expression ein erhöhtes Risiko aufweisen, zu Krebszellen zu transformieren. Auf der anderen Seite bietet die Inhibierung von GPR49 die Möglichkeit, in die Tumor-spezifischen GPR49 Assoziationen mit anderen Prozessen in den Tumorzellen einzugreifen und somit letztendlich den tumorzellenspezifisch veränderten Stoffwechsel zu stören und zu einem Absterben oder zumindest einer Wachstumshemmung der Tumorzellen, insbsondere aber einer Hemmung der Transformation zu Tumorzellen, beizutragen.

Im Rahmen der Erfindung kann es sich empfehlen, im Vorfeld einer Behandlung mit einer erfindungsgemäßen pharmazeutischen Zusammensetzung eine Probe aus einem Gewebe, welches als Tumorgewebe mit anderen Methoden identifiziert ist, zu entnehmen und die Gewebeprobe auf Expression bzw. Überexpression von GPR49 zu untersuchen. Alternativ kann mit einer erfindungsgemäßen Detektorsubstanz zur Diagnose in vivo auf GPR49 Abhängigkeit getestet werden. Wird eine Expression bzw. Überexpression von GPR49 gegenüber Normalgewebe gleichen Typs festgestellt, so ist die Anwendung der erfindungsgemäßen pharmazeutischen Zusammensetzung indiziert.

Generell ist es im Rahmen der Erfindung möglich, patientenspezifisch auf differentielle Expression zu untersuchen, wobei Normalgewebeprobe und Tumorgewebeproben bzw. tumorverdächtige Gewebeproben dem (gleichen) Patienten entnommen und vergleichend auf Werte der GPR49 Expression untersucht werden.

Handelt es sich bei dem Tumor um einem Typus, bei welchem Tumorzellen GPR49 exprimieren, Normalzellen gleichen Gewebetyps jedoch nicht, so ist es besonders bevorzugt, wenn die an GPR49 bindende Substanz zusätzlich eine zytotoxische und/oder immunstimulierende Komponente trägt. Dies führt dann letztendlich dazu, dass praktisch ausschließlich Tumorzellen getötet werden, sei es durch die Zytotoxizität, sei es durch Angriff durch das stimulierte Immunsystem, während Normalzellen in dem Gewebe praktisch vollständig erhalten bleiben. In dieser Ausführungsform braucht die bindende Substanz selbst nicht inhibierend auf GPR49 zu wirken, da die bindende Substanz dann lediglich als Marker funktionieren muß, welcher die Komponenten zu Ziel-Tumorzellen trägt. Im Falle des Einsatzes einer zytotoxischen Komponente wird es sich besonders empfehlen, wenn die pharmazeutische Zusammensetzung zur lokalen Applikation in Tumorzellen enthaltendem Gewebe hergerichtet ist, beispielsweise zur Injektion.

Definitionen und weitere Ausführungsformen der Erfindung.

GPR49 Sequenzen, insbesondere Teilsequenzen, welche in im Rahmen der Erfindung nutzbare Nukleinsäuren oder Peptide oder Proteine enthalten sind, sind in den Seq.-ID 1 bis 82 (siehe auch die Figuren) dargestellt. Die im Rahmen der Erfindung nutzbaren Nukleinsäuren oder Peptide oder Proteine können auch aus diesen Sequenzen bestehen. Im Falle der Seq.-ID 82 bzw. Teilsequenzen hieraus sind auch Mutationen V569A und/oder V666A möglich (siehe beispielsweise Seq.-ID 84). Mit umfasst sind für besagte Mutationen codierende Nukleinsäuren (beispielsweise Seq.-ID 83).

Im Rahmen dieser Beschreibung wird die Bezeichnung GPR49 für alle humanen Isoformen, bekannt oder neu, auf Nukleinsäuren- oder Aminosäurenbasis, verwendet. Mit diesen Begriffen mit umfaßt sind auch die im Rahmen dieser Beschreibung offenbarten kurzen Sequenzen, beispielsweise Immunisierungssequenzen. Weiterhin mit umfaßt sind auch Homologe, wobei die Homologie zumindest 80%, vorzugsweise mehr als 90%, höchstvorzugsweise mehr als 95%, beträgt, berechnet mit dem Programm MEGALIGN (DNASTAR LASERGENE) in der zum Zeitpunkt der vorliegenden Anmeldung aktuellen Fassung. Im Falle der Nukleinsäuresequenzen sind auch komplementäre oder allelische Varianten mit umfaßt. Weiterhin sind Sequenzen umfaßt, welche lediglich Teilsequenzen der explizit offenbarten Sequenzen, beispielsweise ein Exon oder mehrere Exons, oder komplementärer Sequenzen hierzu darstellen, mit der Maßgabe, daß diese Teilsequenzen im Falle der Nukleinsäuren eine für eine Hybridisierung mit einer erfindungsgemäßen Nukleinsäure hinreichende Länge, zumindest 50 Basen, aufweisen und im Falle der Proteine bzw. Peptide mit zumindest gleicher Affinität an ein protein- oder peptidspezifisches Zielmolekül binden. Weiterhin sind alle mit erfindungsgemäßen Nukleinsäuren hybridisierende Nukleinsäuren umfaßt, nämlich solche, die unter stringenten Bedingungen (5°C bis 25°C unterhalb der Aufschmelztemperatur; siehe ergänzend J.M. Sambrook et al., A laboratory manual, Cold Spring Harbor Laboratory Press (1989) und E.M. Southern, J Mol Biol, 98:503ff (1975)) hybridisieren. Es versteht sich, daß die Erfindung auch Expressionskassetten umfaßt, i.e. eine oder mehrere der erfindungsgemäßen Nukleinsäuresequenzen mit mindestens einer operativ verbundenen Kontroll- oder regulatorischen Sequenz. Eine solche Expressionskassette kann auch eine Sequenz für ein bekanntes Protein umfassen, wobei im Zuge der Translation ein Fusionsprotein aus einem bekannten Protein und einem erfindungsgemäßen Protein oder Peptid entsteht. Ebenso sind auch antisense Sequenzen zu den vorstehenden Nukleinsäuresequenzen umfaßt. Schließlich sind RNA sowie damit korrelierende DNA und umgekehrt umfaßt, ebenso wie genomische DNA als auch korrelierte cDNA und umgekehrt.

Im Zusammenhang mit erfindungsgemäßen Verwendungen umfassen die Begriffe der GPR49 Nukleinsäuren oder Protein bzw. Peptide neben den Volllängen der offenbarten Sequenzen (siehe auch vorstehender Absatz) auch Teilsequenzen hieraus, und zwar mit einer Mindestlänge von 12 bis 30 Nukleotiden, vorzugsweise 30 bis 90 Nukleotiden, im Falle der Nukleinsäuren und einer Mindestlänge von 4 bis 10 Aminosäuren, vorzugsweise 10 bis 30 Aminosäuren, im Falle der Peptide oder Proteine. Diese Teilsequenzen können in ansonsten von GPR49 verschiedene Nukleinsäuren- oder Protein- bzw. Peptidsequenzen eingebaut sein.

Der Begriff der Behandlung umfaßt auch die Prophylaxe, insbesondere die Prophylaxe der Progression zu Tumoren.

Als Inhibitor ist eine Verbindung oder Substanz bezeichnet, welche entweder die Bildung von GPR49 Protein inhibiert oder gebildetes GPR49 Protein in der Aktivität reduziert, bezogen auf die GPR49 Aktivität in Abwesenheit des Inhibitors. Insofern kann ein Inhibitor einerseits eine Substanz sein, welche in der Entstehungskaskade von GPR49 inhibierend eingreift. Auf der anderen Seite kann ein Inhibitor eine Substanz sein, welche mit gebildetem GPR49 eine Bindung eingeht, und zwar dergestalt, dass weitere physiologische Wechselwirkungen mit endogenen Substanzen zumindest reduziert sind.

Mimikry-Moleküle sind Verbindungen, die den variablen Bereich, insbesondere den Bindungsbereich eines Antikörpers, nachbilden und an gleicher Stelle eines Zielmoleküls binden, wie der zu Grunde liegende Antikörper.

Der Begriff der Antikörper umfaßt polyklonale Antikörper, monoklonale Antikörper, nicht-humane, humane und humanisierte Antikörper, sowie Phage-Display-Antikörper, aber auch chimäre Antikörper sowie spezifische Fragmente der leichten und/oder der schweren Kette des variablen Bereiches zu Grunde liegender Antikörper vorstehender Art sowie anti-idiotypische Antikörper. Die Herstellung bzw. Gewinnung solcher Antikörper mit vorgegebenen Immunogenen ist dem Durchschnittsfachmann wohl vertraut und braucht nicht näher erläutert zu werden. Weiterhin umfaßt der Begriff der Antikörper bispezifische Antikörper. Bispezifische Antikörper kombinieren eine definierte Immunzellaktivität mit einer spezifischen Tumorzellerkennung, wodurch Tumorzellen getötet werden. Ein bispezifischer Antikörper bindet einerseits an ein Auslösemolekül der Immun-Effektorzelle (z.B. CD3, CD16, CD64) und andererseits an Antigene der Tumorzielzelle.

Die galenische Herrichtung einer erfindungsgemäßen pharmazeutischen Zusammensetzung kann in fachüblicher Weise erfolgen. Als Gegenionen für ionische Verbindungen kommen beispielsweise Na⁺, K⁺, Li⁺ oder Cyclohexylammonium infrage. Geeigente feste oder flüssige galenische Zubereitungsformen sind beispielsweise Granulate, Pulver, Dragees, Tabletten, (Mikro-) Kapseln, Suppositorien, Sirupe, Säfte, Suspensionen, Emulsionen, Tropfen oder injizierbare Lösungen (i.v., i.p., i.m.) sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als Hilfsstoffe sei Magnesiumcarbonat, Titandioxyd, Lactose, Mannit und andere Zucker, Talcum, Milcheiweiß, Gelatine, Stärke, Zellulose und ihre Derivate, tierische und pflanzliche Öle wie Lebertran, Sonnenblumen-, Erdnuss- oder Sesamöl, Polyethylenglycole und Lösungsmittel, wie etwa steriles Wasser und ein- oder mehrwertige Alkohole, beispielsweise Glycerin, genannt. Eine erfindungsgemäße pharmazeutische Zusammensetzung ist dadurch herstellbar, dass mindestens ein erfindungsgemäß verwendeter Inhibitor in definierter Dosis mit einem pharmazeutisch geeigneten und physiologisch verträglichen Träger und ggf. weiteren geeigneten Wirk-, Zusatz- oder Hilfsstoffen mit definierter Inhibitordosis gemischt und zu der gewünschten Darreichungsform hergerichtet ist.

Tumorzellen exprimieren GPR49 differenziell, wenn Normalzellen des gleichen Gewebetyps (des gleichen oder verschiedener Probanden) dieses nicht exprimieren. Tumorzellen überexprimieren GPR49 spezifisch bzw. differenziell, wenn GPR49 im Vergleich zu Normalzellen des gleichen Gewebetyps zumindest in doppelter Menge exprimiert wird.

Zytotoxische Komponenten bzw. Gruppen sind Verbindungen, welche direkt oder indirekt Apoptose einleiten bzw. zu Nekrose führen oder zumindest wachstumshemmend wirken. Solche Gruppen bzw. Verbindungen können neben Radioisotopen (z.B. 188Re, 213Bi, 99mTc, 90Y, 131J, 177Lu) insbesondere Zytostatika sein, welche in der Tumortherapie eingesetzt werden. Beispiele hierfür sind: Alkylantien (z.B. Mechlorethamin, Ifosfamid, Chlorambucil, Cyclophosphamid, Melphalan, Alkylsulfonate, Busulphan, Nitrosoharnstoffe, Carmustin, Lomustin, Semustin, Triazene, Dacarbazin), Antimetaboliten (z.B. Folsäure-Antagonisten, Methotrexat, Pyrimidin-Analoga, Fluoruracil, Fluordesoxyuridin, Cytarabin, Gemcitabin, Purin-Analoga, Mercaptopurin), Mitosehemmer (z.B. Vincaalkaloide, Voncristin, Vinblastin, Paclitaxal, Docetaxel, Protaxel), Epipodophyllotoxine (z.B. Etoposid, Teniposid), Antibiotika (z.B. Dactinomycin, Daunorubicin, Idarubicin, Anthracycline, Bleomycin, L-Asparaginase), Platinkomplexverbindungen (z.B. Cisplatin), Hormone und verwandte Verbindungen (z.B. Nebennierenrindensteroide, Aminogluthetimid, Gestagene, Östrogene, Androgene, Antiöstrogene, Tamoxifen, Steriodanaloga, Flutamid). Bei Bindung einer solchen Verbindung mit einer an GPR49 bindenden Substanz erfolgt die Kopplung dergestalt, daß die Affinität zu GPR49 um nicht mehr als 90%, vorzugsweise 50%, bezogen auf die Substanz ohne zytostatische Gruppe, reduziert ist und die zytostatische Wirkung der Gruppe um nicht mehr als 90%, vorzugsweise 50%, bezogen auf die Verbindung ohne Substanz, reduziert ist.

Eine immunstimulierende Komponente ist meist ein Protein oder ein wirksamer Bestandteil hiervon, welches Zellen des Immunsystems stimuliert. Beispiele hierfür sind: Zytokine, wie M-CSF, GM-CSF, G-CSF, Interferone, wie IFN-alpha, -beta, -gamma, Interleukine wie IL-1 bis -16 (außer -8), human LIF, Chemokine wie Rantes, MCAF, MIP-1-alpha, -beta, NAP-1 und IL-8.

Eine Reportergruppe ist ein Atom, Molekül oder eine Verbindung, welche in Verbindung mit einem hierauf abgestellten Assay den Nachweis der Reportergruppe und der somit mit der Reportergruppe verbundenen Verbindung oder Substanz ermöglicht. Beispiele für Reportergruppen und hiermit assoziierte Detektionsmethoden sind: 32P-Labeling und Intensitätsmessung mittels Phosphoimager. Viele weitere Beispiele sind dem Durchschnittsfachmann bekannt und bedürfen nicht der detaillierten Aufzählung.

Eine an GPR49 bindende Substanz kann eine Substanz sein, welche an ein GPR49 Protein oder eine GPR49 RNA bindet.

Im Rahmen der vorstehenden Definition gegenüber dem engen Wortsinn erweiterte Begriffsbestimmungen umfassen auch die bestimmten Begriffe im engen Wortsinn.

### Beispiele.

Im Folgenden wird die Erfindung anhand von lediglich bevorzugte Ausführungsformen darstellenden Beispielen und Figuren näher erläutert. Es zeigen:
- Fig. 1:: Chipanalyse zur differentiellen Expression von GPR49 in Colontumorgewebe aus 28 Patienten,
- Fig. 2:: Northern-Blot-Verfahren des Cancer-Profiling-Arrays zur diffenrtiellen Expression von GPR49 in Colon-, Uterus- und Rectumtumorgewebe,
- Fig. 3:: Taqman-Analyse zur differentiellen Expression von GPR49 in Colontumorgewebe (nach erfolgter Laser-gestützter Microdissektion der Normalepithelien und Tumorzellen),
- Fig. 4:: Taqman-Analyse zur spezifischen Expression von GPR49 im Gewebe des Dünndarms (Small Intestine), der Plazenta, Rückenmark (Spinal Cord), Nebenniere (Adrenal Gland), Gehirn (Brain), Magen (Stomach) und Skelettmuskulatur (Skeleton Muscle) aus 26 Normalgeweben eines Patienten,
- Fig. 5:: Verwendete Peptidsequenzen zur Immunisierung von Kanninchen und zur Gewinnung von Antikörpern gegen GPR49,
- Fig. 6:: Verwendete cDNA Sequenz zur Immunisierung in Ratten mit der Ektodomäne des N-Terminus von GPR49,

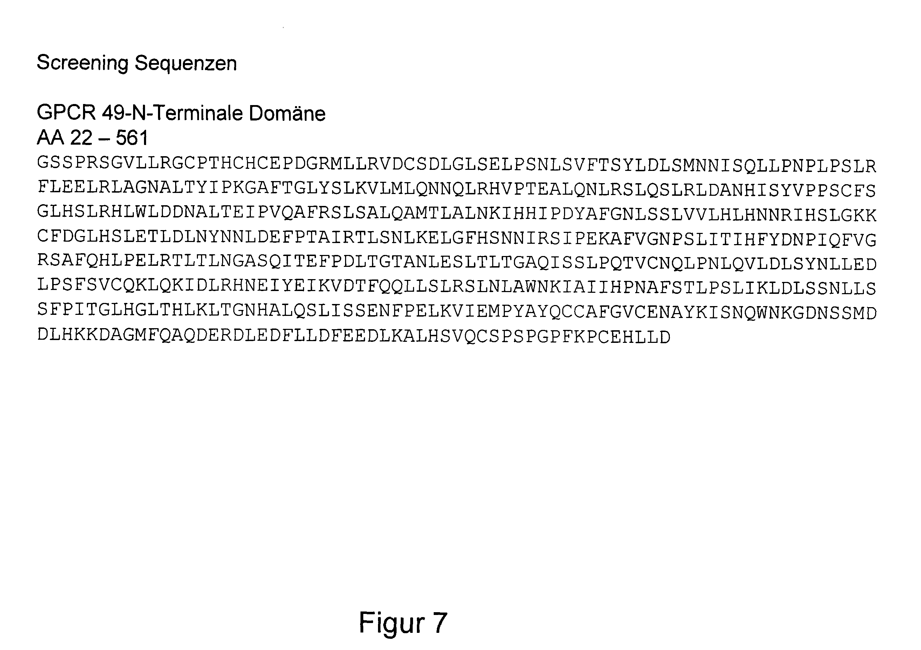

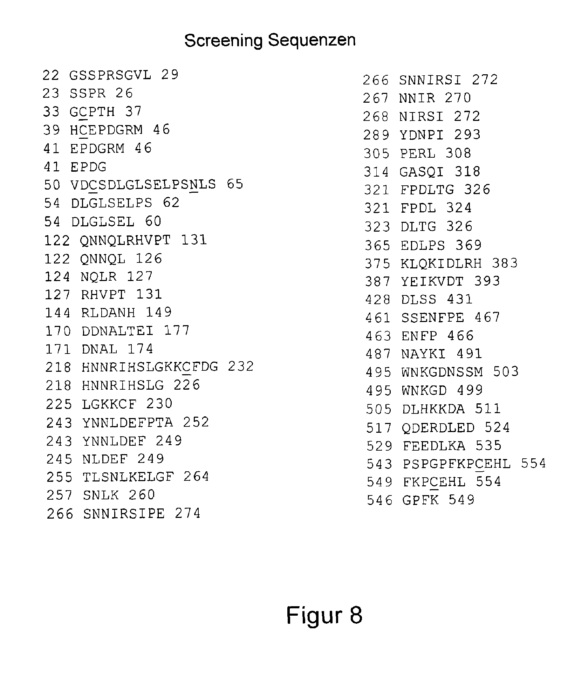

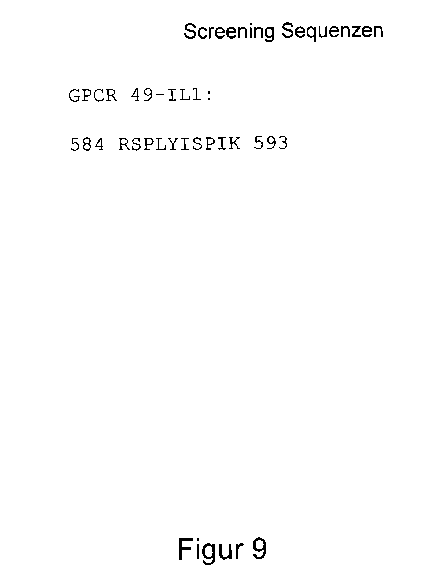

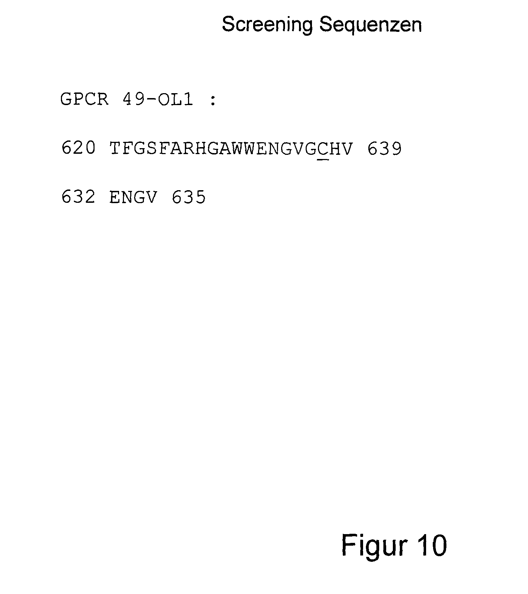

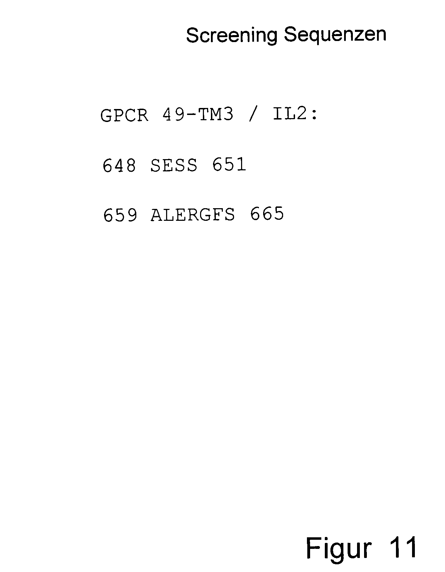

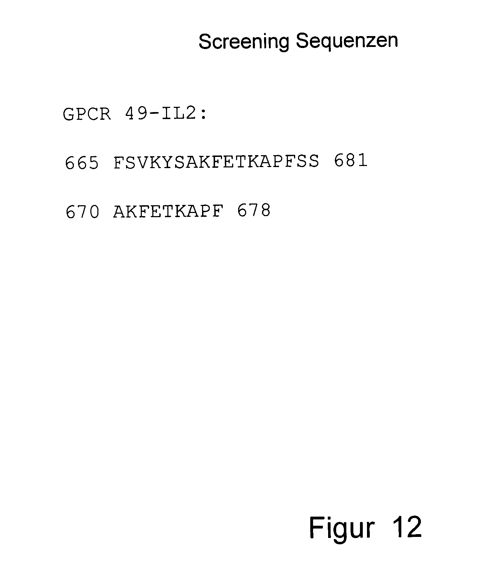

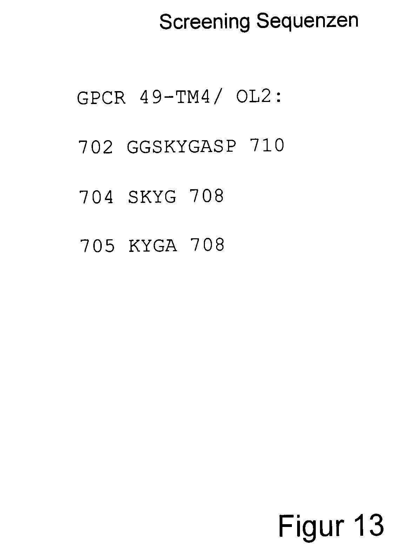

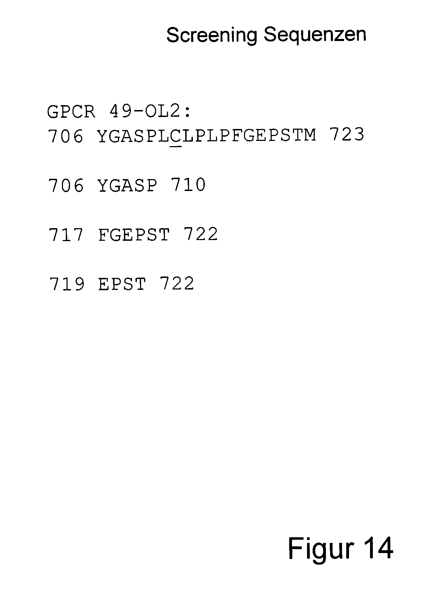

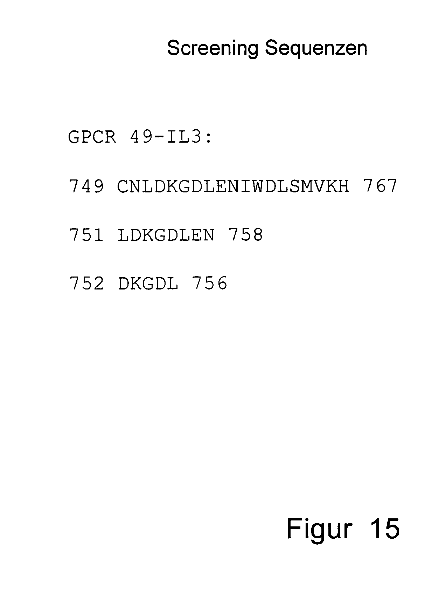

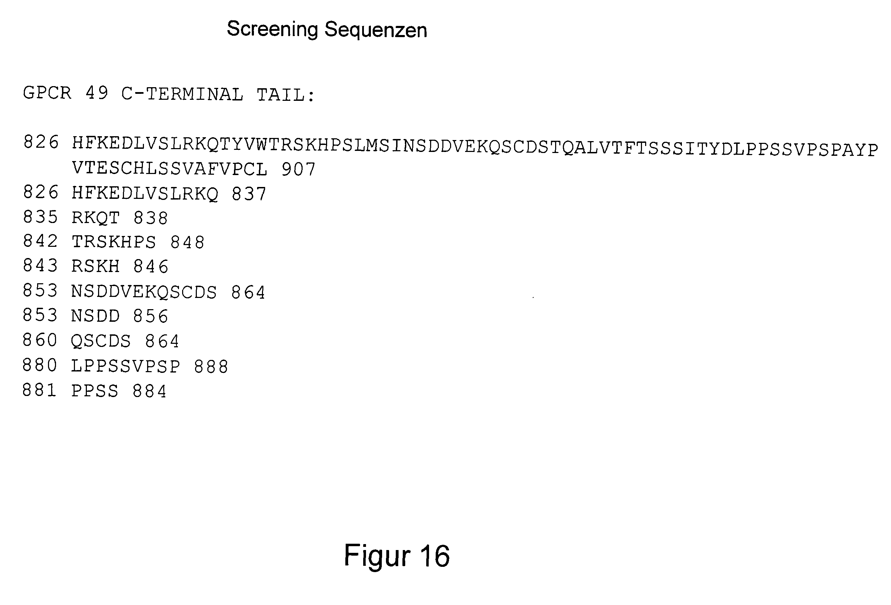

- Figuren 7 bis 16:: Teilsequenzen aus GPR49, welche zum Einsatz in Screening Verfahren geeignet sind,
- Fig. 17:: Nukleinsäuresequenz von GPR49,

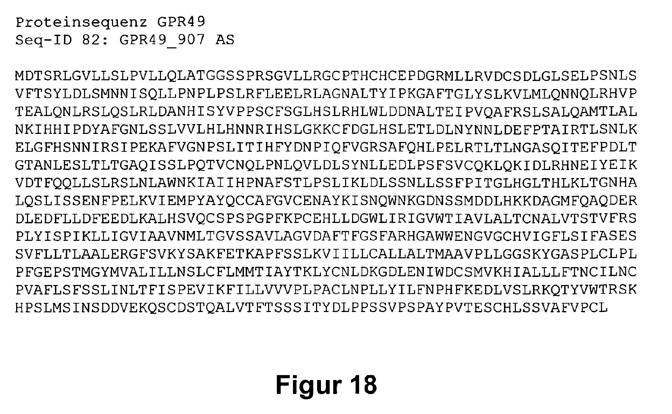

- Fig. 18:: Aminosäurensequenz von GPR49,

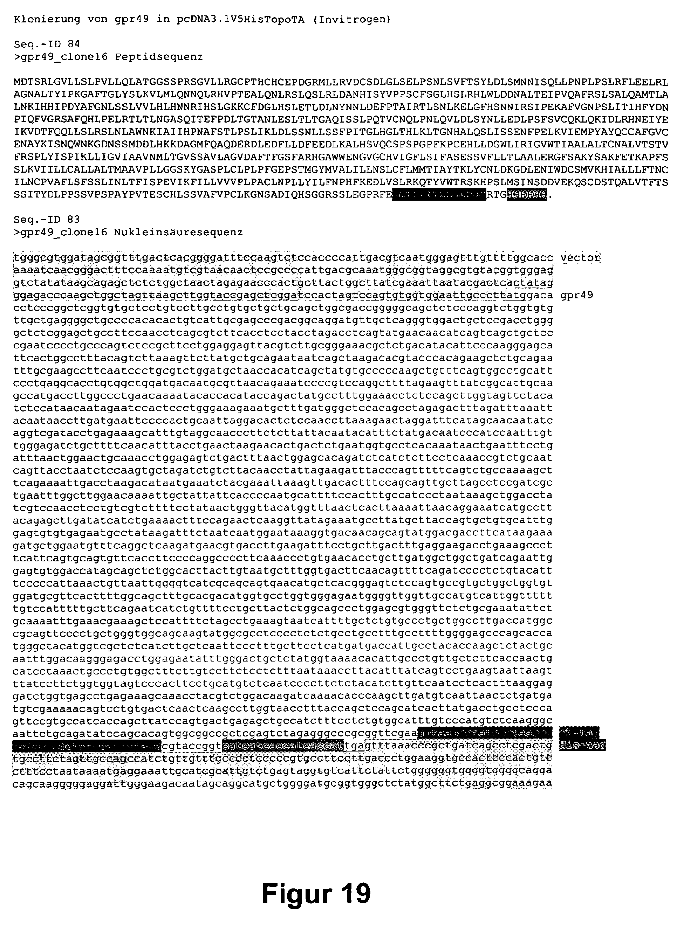

- Fig. 19:: alternative verwendete cDNA Sequenzen (Volllängen) zur Immunisierung in Ratten, und
- Fig. 20:: in situ Hybridisierungen in Colon Tumorgewebe.

### Beispiel 1: Untersuchte Gewebeproben

Es wurde Colontumorgewebe von 28 Patienten mit zum Zeitpunkt der Erhebung invasiven Tumoren entnommen, und zwar einerseits aus dem Zentraltumor und andererseits aus der Invasionsfront. Zu Vergleichszwecken wurde zugleich Normalgewebe den Patienten entnommen. Die drei Gewebeprobentypen (Kerntumor, Invasionsfront, Normalgewebe) aus jeweils einem Patienten wurden einander zugeordnet. Im Einzelnen wurde wie folgt verfahren. Die Tumorund -normalgewebeproben wurden gefroren und in 10µm Proben geschnitten. Aus jedem Patienten wurden zumindest 30 Proben gewonnen. Normale und maligne Bereiche wurden durch einen Pathologen mit Hilfe eines Mikroskopes identifiziert und markiert. Hierbei wird ggf. auch die Verlaufsform identifiziert und der Probe zugeordnet. Die jeweiligen Bereiche wurden unter dem Mikroskop resektiert unter Verwendung einer Nadel und jeweils separat auf -80°C eingefroren in 150µl GTC Puffer enthaltend 2% β-Mercaptoethanol.

Für das Cancer-Profiling-Array wurden Tumor- und Normalgewebeproben aus Brust, Uterus, Darm, Magen, Ovar, Lunge, Niere Rektum und Schilddrüse verwendet.

### Beispiel 2: Expressionsprofile der untersuchten Gewebe

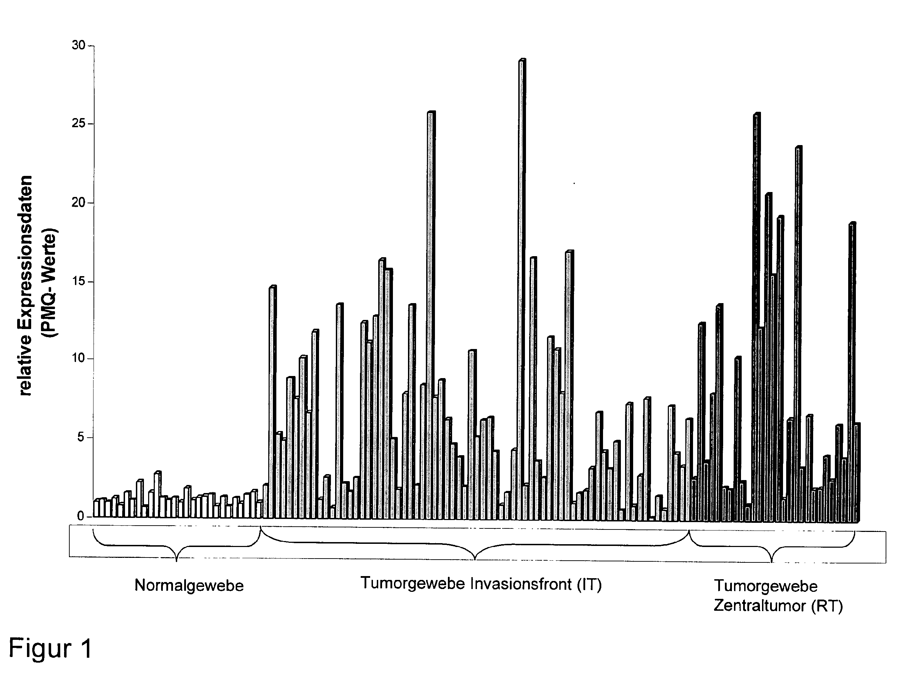

Die Proben aus Beispiel 1 wurden einer Expressionsanalyse auf GPR49 mittels der GeneChip-Technologie (Affimetrix) unterworfen. Dabei wird aus Proben aus Beispiel 1 RNA isoliert, amplifiziert und markiert. Die so erhaltene RNA wird einem Genchip aufgegeben, welcher eine Vielzahl von verschiedenen Oligonukleotiden enthält, wobei jeweils eines (oder auch mehrere, zu Kontrollzwecken) für ein definiertes Gen repräsentativ ist, i.e. eine charakteristische Teilsequenz hieraus aufweist. Man erhält sowohl qualitative, wie auch quantitative Information, ob eine betreffende Normal- und/oder Tumorprobe ein betreffendes Gen exprimiert, und zwar auch im Verhältnis Tumor/Normal. Die Ergebnisse sind in der Figur 1 dargestellt. Man erkennt eine deutliche Überexpression von GPR49 sowohl in der Invasionsfront des Tumors als auch im Zentraltumor selbst.

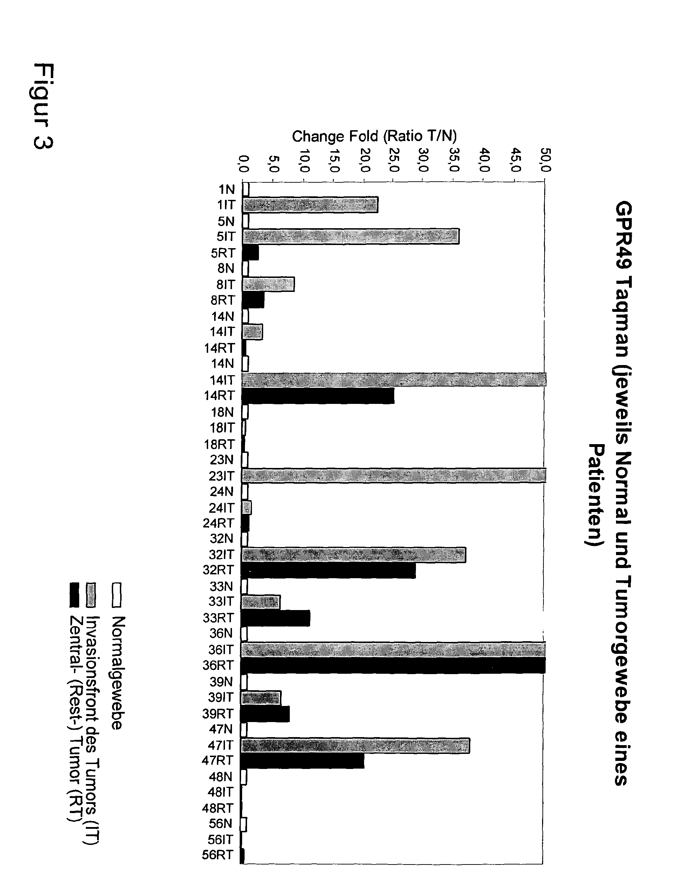

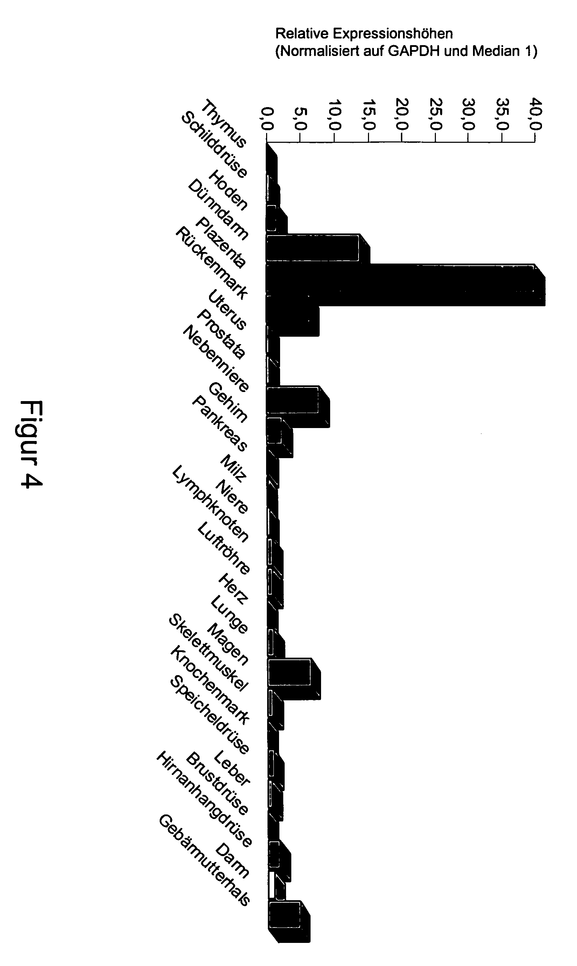

Diese Ergebnisse werden auch durch GPR49 Taqman Ergebnisse gestützt, welche in der Figur 3 dargestellt sind. Bei diesen Experimenten wird im Einzelnen wie folgt verfahren. Eine Poly-A+-RNA Präparation erfolgt unter Verwendung eines modifizierten Protokolls gemäß dem Poly-A-Tract 1000 Kit (Amersham, Freiburg, Deutschland). Gewebeproben, erhalten gemäß Beispiel 1, werden langsam auf Eis aufgetaut, zerkleinert und mit 300µl vorgewärmten Verdünnungspuffer, enthaltend 1% β-Mercaptoethanol, sowie 10 pmol biotinyliertem Oligo-dT Primer versetzt, und für 5 min. auf 70°C erhitzt. Die Proben werden dann für 5 min. bei 20°C gehalten und anschließend bei 20000g für 10 min. zentrifugiert. Dem Überstand werden 120µl gewaschener Streptavidin-gekoppelter paramagnetischer Partikel (SA-PMP) zugebenen und es wurde bei 20°C für 5 min. inkubiert. Die mRNA wurde dann durch magnetische Trennung isoliert. Nach drei Waschschritten mit 0,5x SSC Lösung wird die mRNA in Nuklease-freiem Wasser eluiert, in der Speed-Vac einrotiert, um dann in 11µl DEPC Wasser eluiert zu werden. Es folgt eine RNA-Konzentrationsbestimmung durch RiboGreen Messung. Anschließend erfolgt die cDNA Synthese. 1µl T7-dT24-(GGCCAG) Primer (100 pmol/µl) wird zu den 10µl mRNA gegeben und auf 70°C für 5 min. erhitzt. Dann wurde die Probe auf Eis gelegt und es werden 4µl 5x first strand buffer (Invitrogen), 2µl DTT (0,1M), 1µl dNTP's (10mM), und 14U anti-RNAse (Ambion) zugegeben, gefolgt von einer Inkubation für 2 min. bei 37°C. Dann werden 200nl Superscript II Reverse Transkriptase (Invitrogen) zugegeben, gefolgt von einer Inkubation für 1 h bei 37°C. Anschließend erfolgt die Zweitstrangsynthese und DNA Reinigung. Sofort nach der Synthese des ersten Stranges, wie vorstehend, werden 91µl Wasser, 30µl 5x second strand buffer, 3µl dNTP's (10mM), 10U E. coli DNA-ligase, 40U DNA Polymerase I und 2U RNAse H (alle von Invitrogen) zugegeben und die Mischung wird für 2 h bei 16°C inkubiert. Dann werden 10U T4 DNA Polymerase (Invitrogen) zugegeben und weitere 5 min. inkubiert. Die Reaktion wird durch Zugabe von 10 µl 0,5mM EDTA abgebrochen. Die Reinigung der DNA erfolgt gemäß den Vorschriften des GFX PCR DNA and Gel Band Purification Kits (Amersham). Die gereinigte DNA wird unter Vakuum eingedampft, in 9 µl DEPC Wasser aufgenommen und bei -20°C gelagert. Dann erfolgt die in vitro Transkription und cRNA Reinigung. Die in vitro Transkription wird gemäß dem Herstellerprotokoll von Ambion (Huntigdon, UK) durchgeführt. Zu 8 µg der cDNA werden 7,5µl dNTP's (75mM), 2µl 10x reaction buffer (Ambion), 2µl 10 T7 Enzymmix (Ambion) und 14U anti-RNAse (Ambion) zugegeben, gefolgt von einer Inkubation von 6 h bei 37°C. Die Reinigung der erhaltenen cRNA erfolgt gemäß dem Herstellerprotokoll zum Rneasy Mini Kit (Qiagen, Hilden, Deutschland). Nach Elution von der Säule wird die CRNA (unter Vakuum) eingedampft, in Wasser aufgenommen und bei -80°C eingelagert. Anschließend wird die zweite cDNA Synthese durchgeführt. Die Synthese des ersten Stranges erfolgt mit random hexamer primer (250ng/µl). Nach Inkubation über 60 min. wird das cRNA-cDNA Hybrid für 20 min. mit 2U RNase H inkubiert, gefolgt von einem 2-minütigen Inaktivierungsschritt bei 37°C. Schließlich erfolgt die quantitative PCR und Auswertung. 1ng cDNA werden für die Amplifikation eingesetzt mit 2,5µl 10x SYBR®Green PCR Puffer, 3µl Magnesiumchlorid (25mM), 2µl dNTP's (mit dUTP; 12,5 mM) und 0,625U Ampli Taq Gold in einem Reaktionsvolumen von 25µl. Die Reaktion wird in einem GeneAmp 5700 Sequence Detection System (Applied Biosystems, Weiterstadt, Deutschland) durchgeführt. Die Bedingungen sind: 2 min. 50°C, 10 min. 95°C, 15 s 95°C, 1 min. 60°C, die letzten beiden Phasen in 40 Zyklen. Für die jeweiligen Gene werden die geeigneten Vorwärts- bzw. Rückwärtsprimer verwendet. Die Auswertung erfolgt nach der ΔΔCt Methode nach Herstellervorschrift. Der Ct Wert von beta actin wurde bei einer Grenze von 0,1 gemessen. Zur Normalisierung wird der Ct Wert des beta actin vom Ct Wert des untersuchten Gens abgezogen. Dieser normalisierte Ct Wert wird im Falle der Tumorgewebe auf die Normalgewebe bezogen bzw. normalisiert, wodurch der ΔΔCt erhalten wird. Wird dieser Wert als Potenz zur Basis 2 eingesetzt, so wird eine relative Größe der Überexpression in Tumorgewebe gegenüber dem Normalgewebe des gleichen Patienten erhalten. Auch in diesen Experimenten erkennt man eine vielfache Überpression des GPR49 in der Invasionsfront sowie dem Zentraltumor, verglichen mit den korrespondierenden Normalgewebe. Der Figur 4 ist zu entnehmen, dass in der überwiegenden Anzahl untersuchter Normalgewebe die endogene Expression von GPR49 gerade messbar bis allenfalls sehr gering ist.

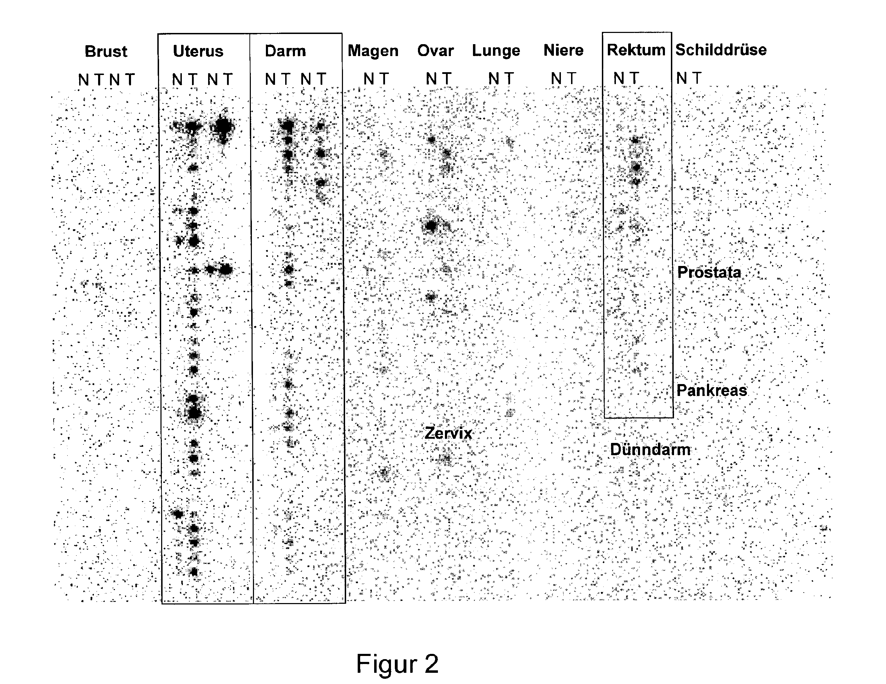

Der Figur 2, welche einen Northern Blot anhand des Cancer-Profiling-Arrays darstellt, entnimmt man, dass eine differenzielle Expression nicht nur in Colon festzustellen ist, sondern auch in Uterus und Rectum.

### Beispiel 3: Nachweis eines überexprimierten Gens mittels Antikörpern.

In diesem Beispiel wird die Markierung von Tumorzellen durch einen gegen ein erfindungsgemäß verwendetes Protein gerichteten Antikörper in vivo (Mausmodell) beschrieben. Ein solcher erfindungsgemäßer Antikörper wird mit einem Markermolekül (z.B. Radioisotop) markiert. In NMRI-Nacktmäuse werden mit einem erfindungsgemäßen Gen transfizierte humane Zellen transplantiert. Nach einem definierten Zeitraum, beispielsweise 30 Tage, wird den Mäusen der markierte Antikörper injiziert. Die Kontrolltiere werden mit einem nicht relevanten Antikörper behandelt. Wenige Stunden nach der Antikörperapplikation werden die Tiere getötet und aus allen Organen Gewebeschnitte angefertigt. Diese Schnitte werden auf die Gegenwart von markiertem Antikörper untersucht.

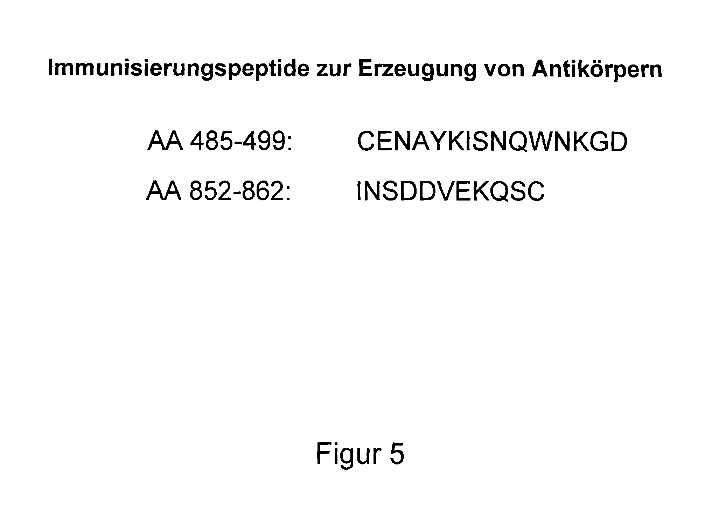

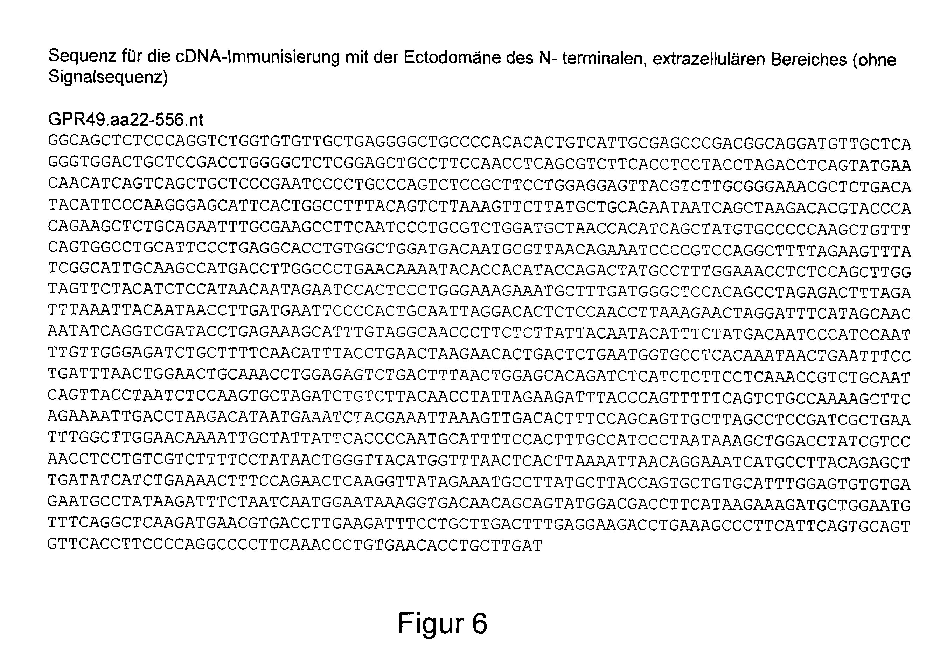

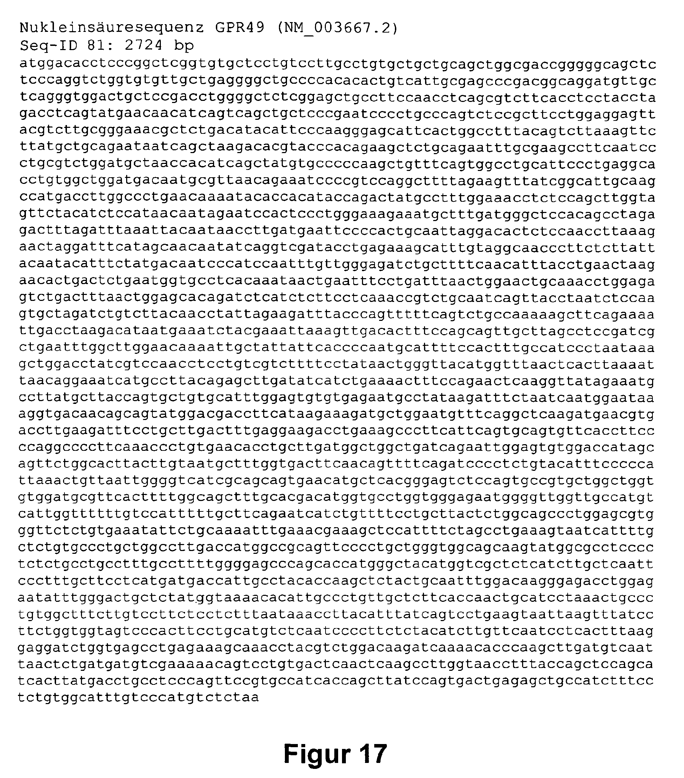

Bei den Antikörpern kann es sich im einfachsten Fall um polyklonale Antikörper gegen humanes Protein, konjugiert mit einem Trägerprotein, in Kaninchen gezogen und mit den spezifischen immobilisierten Peptiden affinitätsgereinigt, handeln. Geeignete Immunisierungspeptide sind beispielsweise aus Teilsequenzen eines erfindungsgemäßen Proteins gebildet, wozu auch auf die Fig. 5 verwiesen wird. Bei der Sequenz AA 852-862 dieser Figur kann das endständige Cystein auch entfallen, da es sich dabei um ein Aminosäure zur Koppelung an ein Trägerprotein handelt. Als Immunogene können ebenso mit cDNA des Gens, oder Teilsequenzen hiervon transfizierte Zellen, wie beispielsweise COS-Zellen oder NIH3T3-Zellen, eingesetzt werden, wozu beispielhaft auf die Fig. 6 oder 17 verwiesen wird. Ebenso sind Tumorzellen, die endogen das Protein exprimieren, geeignet. Weiterhin kann auch rekombinant hergestelltes Protein bzw. Teilsequenzen hieraus, die in Producerzellen, wie E. coli oder Insektenzellen oder Säugerzellen exprimiert werden, zur Immunisierung eingesetzt werden. Selbstverständlich können stattdessen auch entsprechende monoklonale Antikörper oder Fragmente hiervon eingesetzt werden. Die Herstellung von monoklonalen Antikörpern ausgehend von vorstehenden Immunisierungssequenzen und beispielsweise über die Genereirung und Selektion von Hybridomzellen ist dem Durchschnittsfachmann wohl vertraut und bedarf keiner detaillierten Darstellung.

### Beispiel 4: Immunhistochemischer Nachweis von Tumorzellen oder Nachweis mittels in situ Hybridisierung.

Gewebe wird aus einem Patienten mit Krebs oder dem Verdacht auf Krebs isoliert und als Paraffin- bzw. Gefrierschnitte präpariert. Diese Schnitte werden für den immunhistochemischen Nachweis mit einem gegen ein erfindungsgemäßes Protein gerichteten Antikörper auf die Überexpression des Proteins in Zellen untersucht. Die immunhistologische Untersuchung mit dem Antikörper zeigt bei heraufregulierten Genen höhere Expression des Proteins in den Tumorzellen im Vergleich zu umliegenden Normalgewebe. Die Untersuchung erfolgt im Einzelnen beispielsweise durch Inkubation mit dem Antikörper als primärem Antikörper, einem biotinyliertem sekundären anti-Kaninchen Antikörper und einer Streptavidingekoppelten Meerrettich- peroxidase. Die Färbung erfolgt mit mit DAB als chromogenen Substrat (braune Färbung). Die Gegenfärbung erfolgt mit Hemalaun-Lösung (blaue Färbung). Es sind maligne und nichtmaligne Zellen unterscheidbar, wobei die malignen Zellen eine starke Färbung, i.e. hohen Gehalt an erfindungsgemäßem Protein, aufweisen, während die nichtmalignen Zellen nur moderat gefärbt sind.

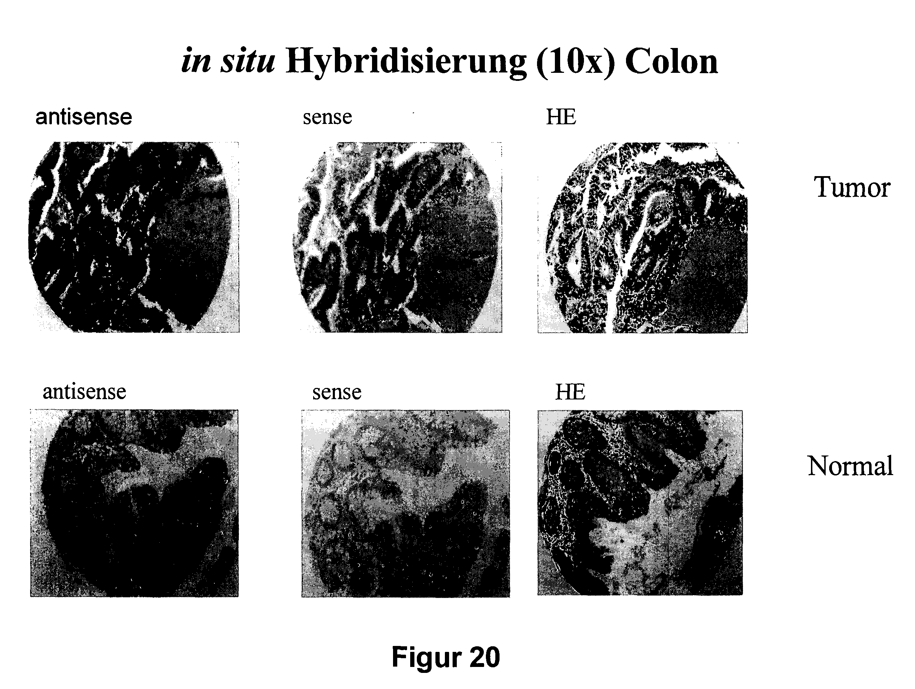

Ergebnisse eine in situ Hybridisierung sind in der Figur 20 dargestellt. Gewebeproben aus Colongewebe wurden mit einer GPR49 spezifischen antisense Sonde inkubiert und mit BM-Purple gefärbt. Die Gegenfärbung erfolgte mit Kernecht-Rot. Es sind maligne und nichtmaligne Epithelzellen voneinander zu unterscheiden, von denen die malignen Zellen eine starke Anfärbung der RNA in der in situ Hybridisierung zeigen. Man erkennt, dass GRP49 im Colontumorgewebe (links) auf RNA Ebene deutlich überexprimiert ist, verglichen zur Kontrolle (Mitte) und zum Colonnormalgewebe (rechts) .

### Beispiel 5: Erzeugung von anti-idiotypischen monoklonalen Antikörpern zu therapeutischen Zwecken

Ausgehend von einem erfindungsgemäß verwendeten Protein wird in fachüblicher Weise ein monoklonaler Antikörper Ab1 erzeugt, welcher in der Lage ist, das Protein spezifisch zu erkennen und daran zu binden. Dabei ist es unwesentlich, ob eine funktionale Domäne oder ein anderer zugänglicher Bereich erkannt wird. Mit Hilfe des erzeugten Antikörpers Ab1 wird in ebenso fachüblicher Weise ein zweiter anti-idiotypischer nicht humanisierter, beispielsweise Maus, monoklonaler Antikörper aAB1 erzeugt, welcher zur Herstellung einer pharmazeutischen Zusammensetzung zur Behandlung von Krebs, insbesondere Colon-, Uterus- oder Rectumtumoren, geeignet ist. Die Funktion des Antikörpers aAB1 beruht dabei darauf, dass dieser dem humanen Immunsystem ein Image des (humanen) Protein-Antigens gleichsam vortäuscht, wobei das Immunsystem den Antikörper aAB1 aufgrund seiner mangelnden Humanisierung als körperfremd erkennt. Der humane Körper bildet folglich eigene Antikörper, die gegen aAB1 und somit auch gegen das humane Protein bzw. dieses exprimierende Tumorzellen gerichtet sind.

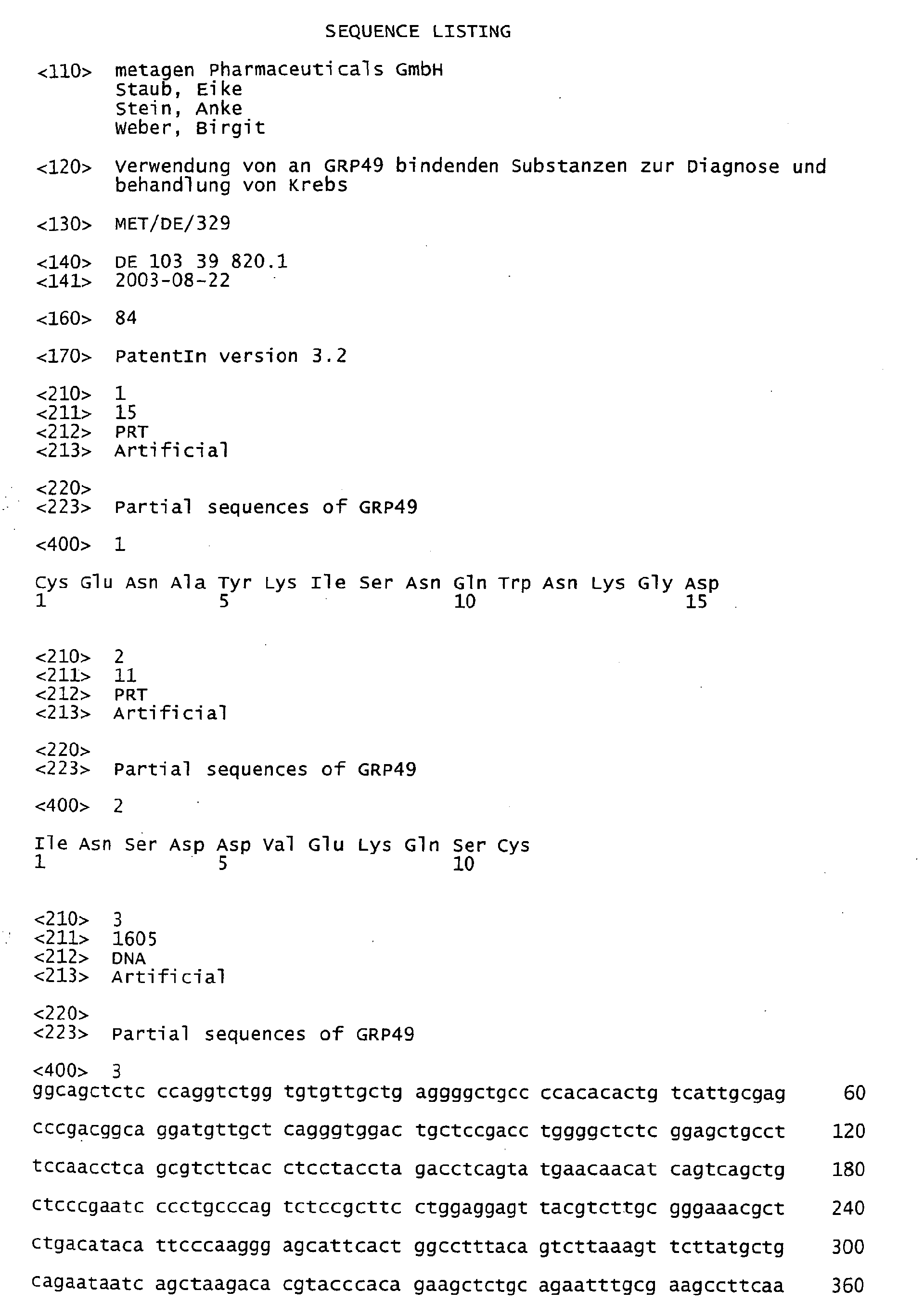

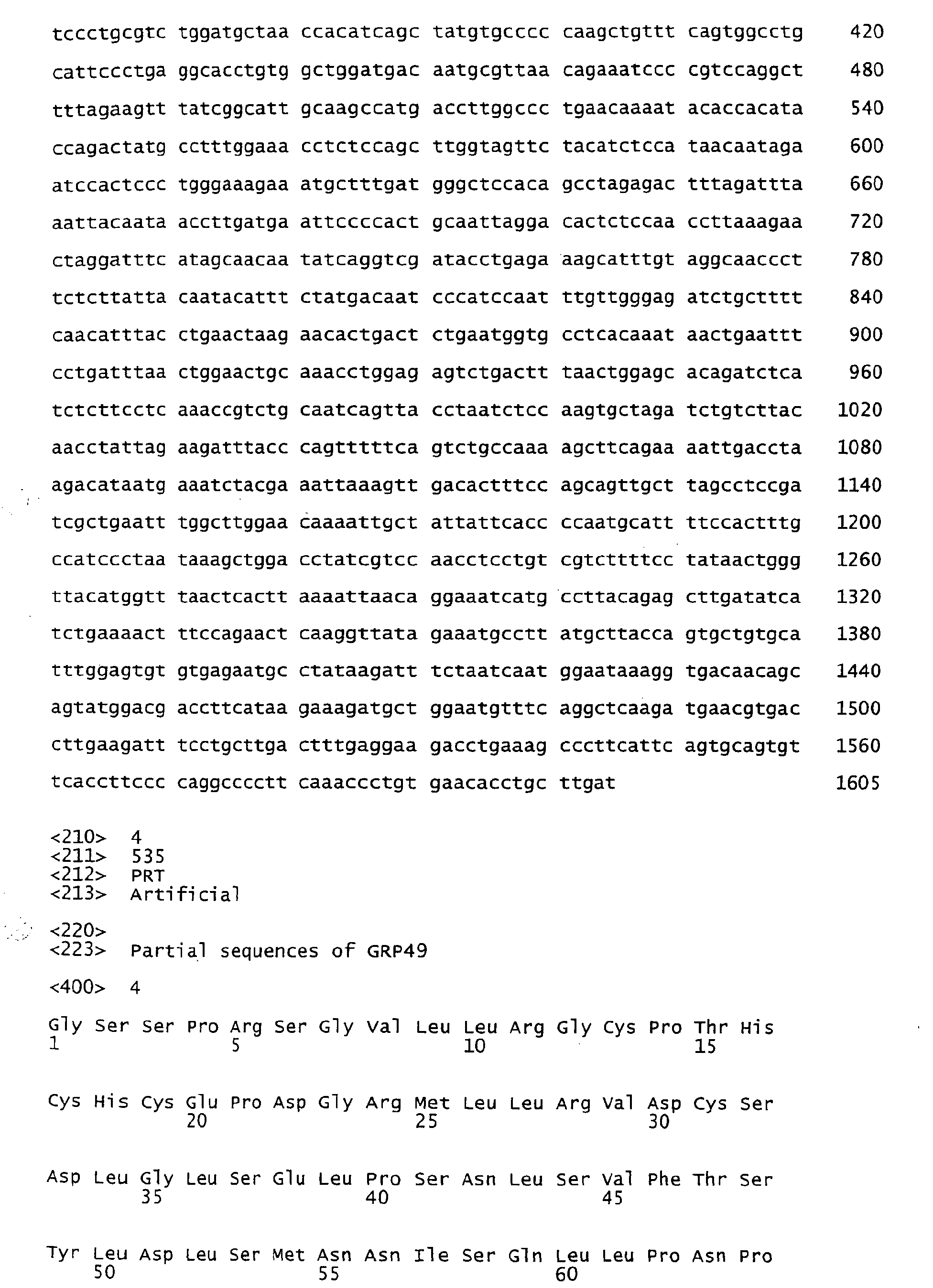

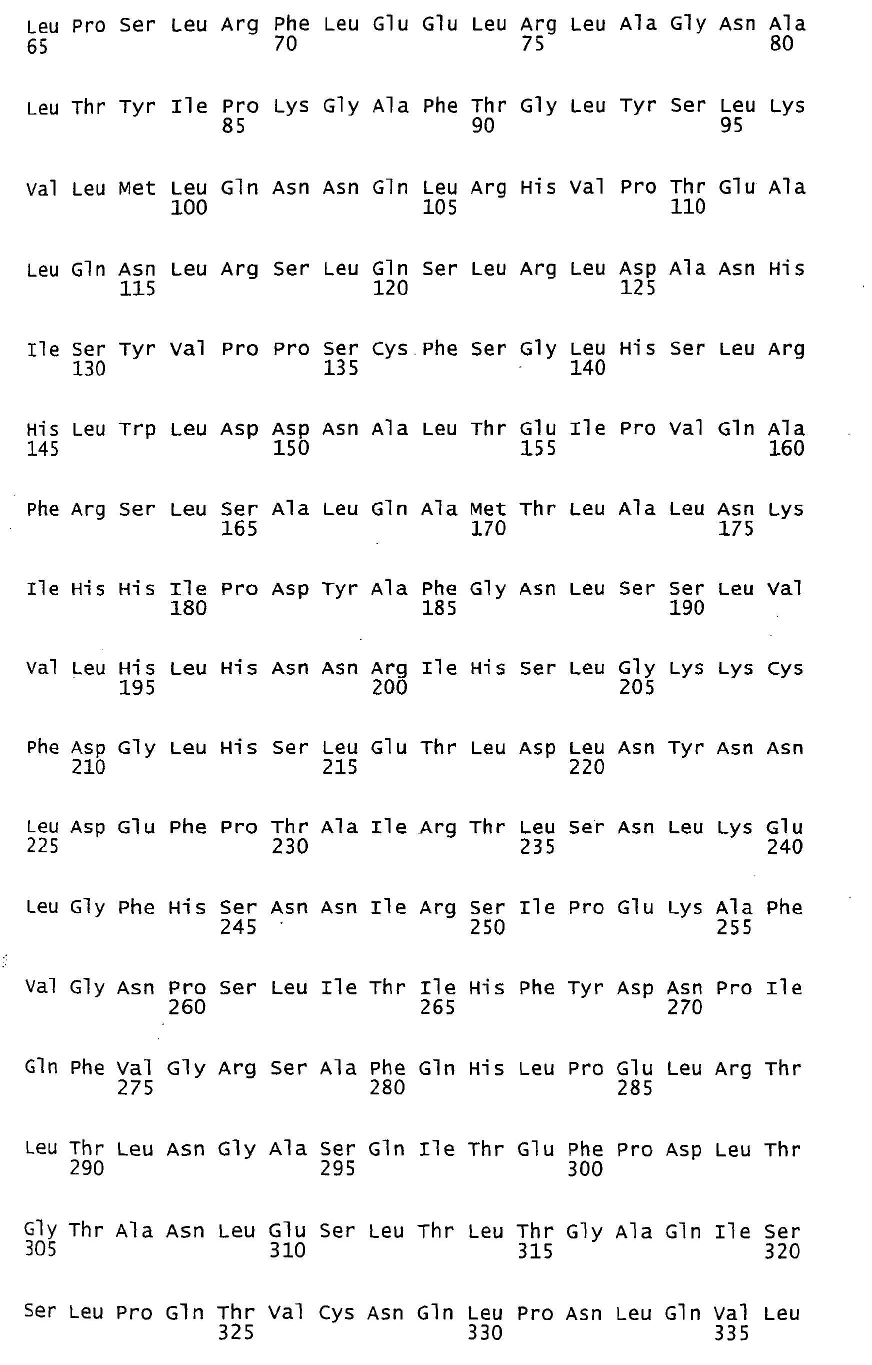

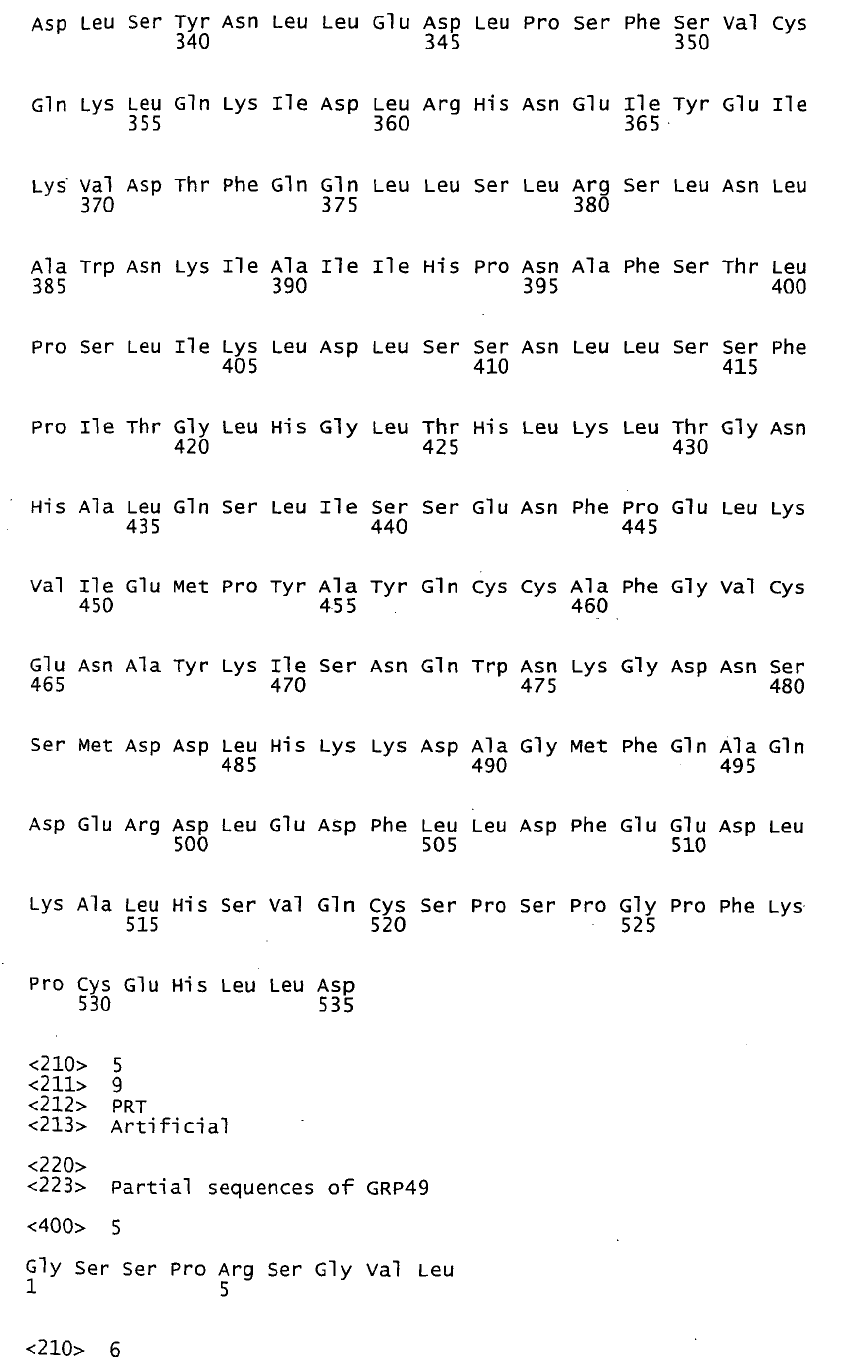

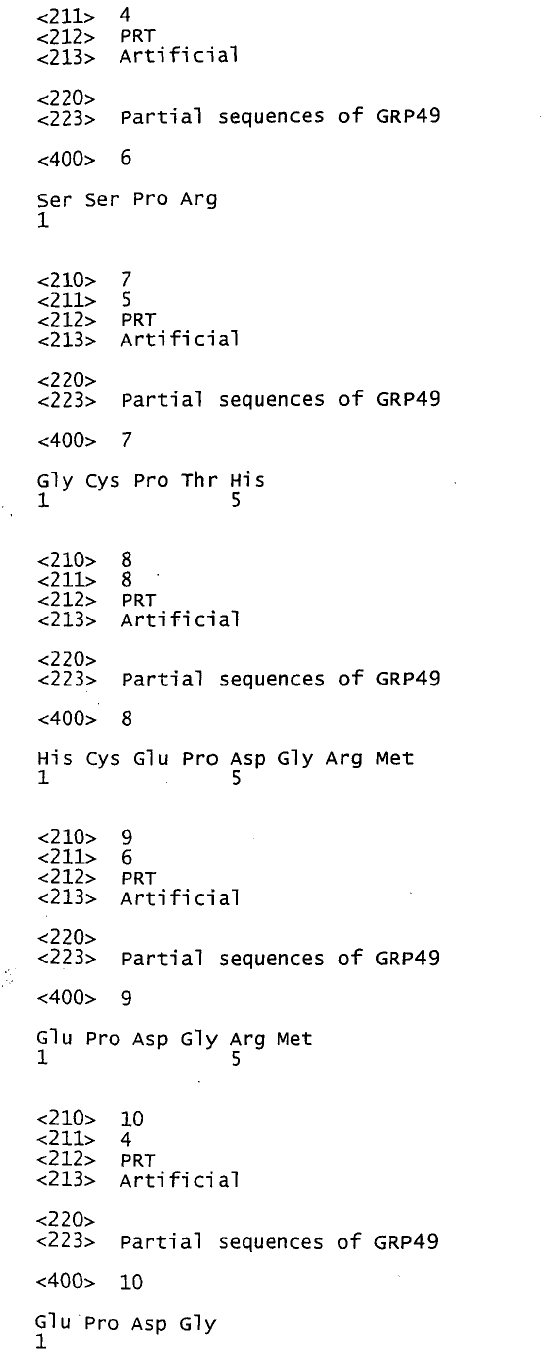

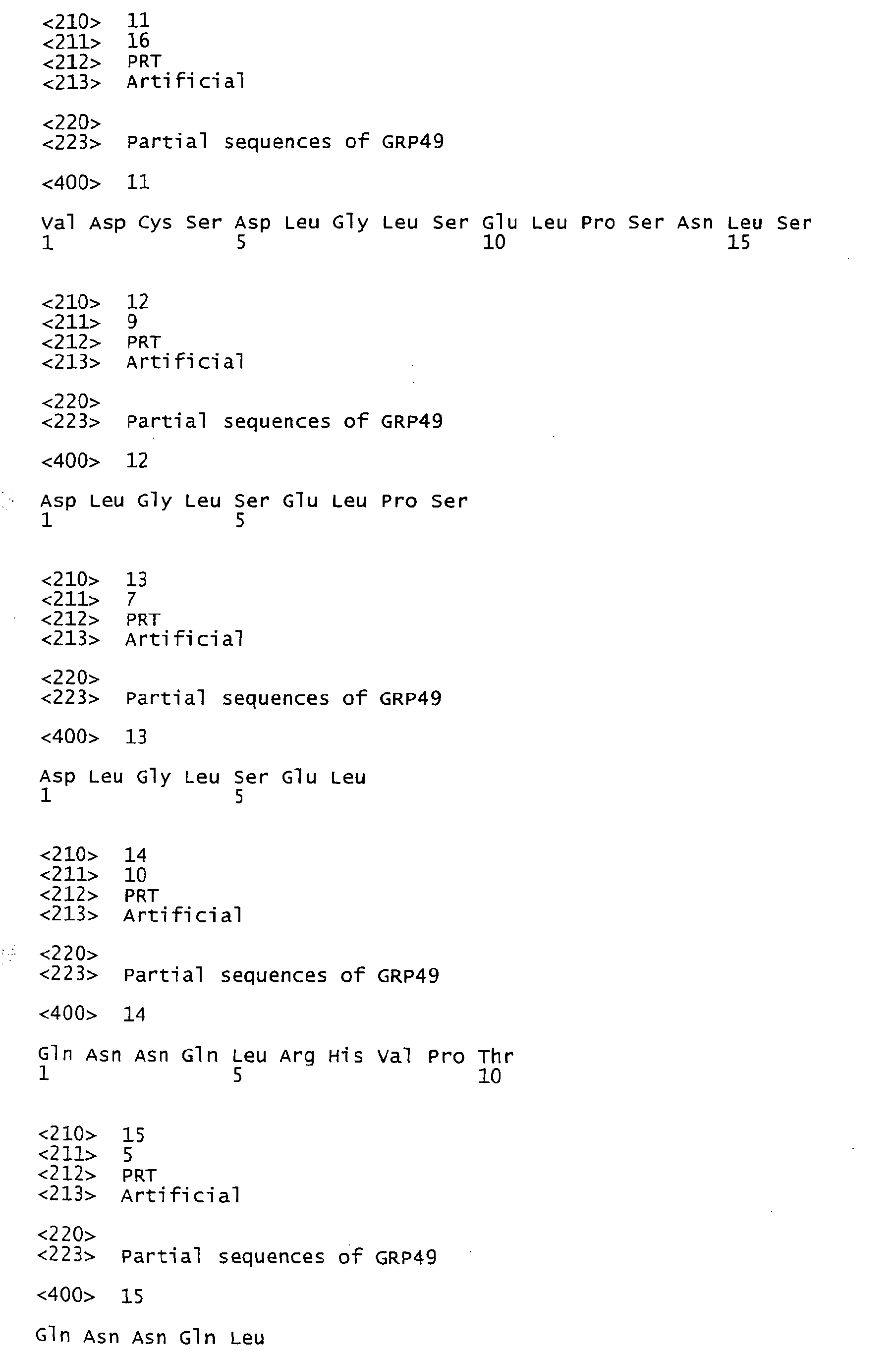

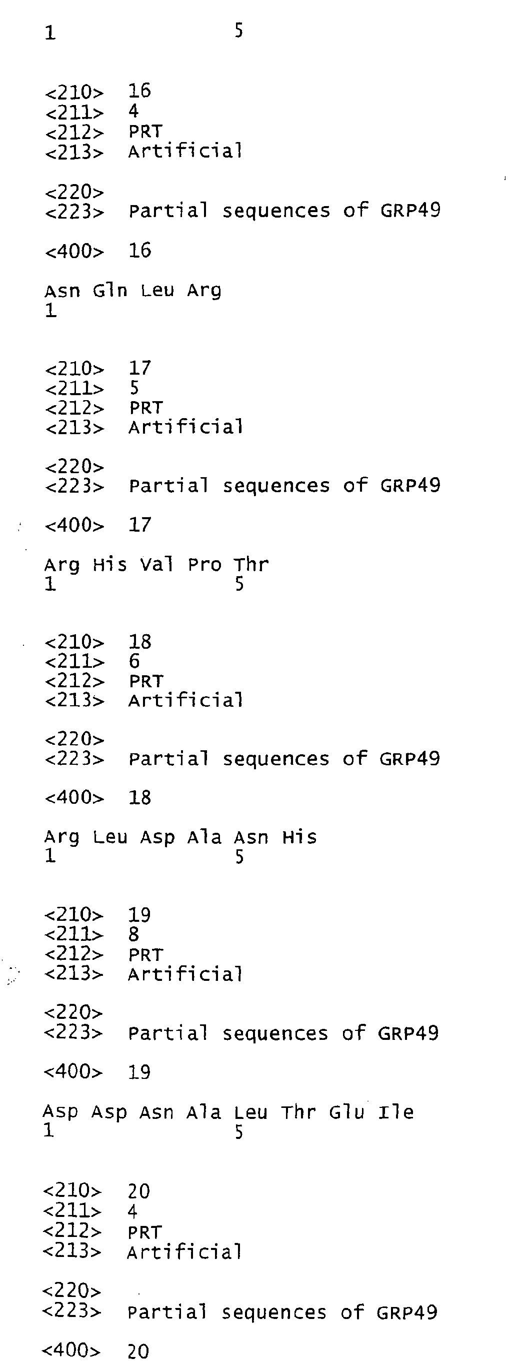

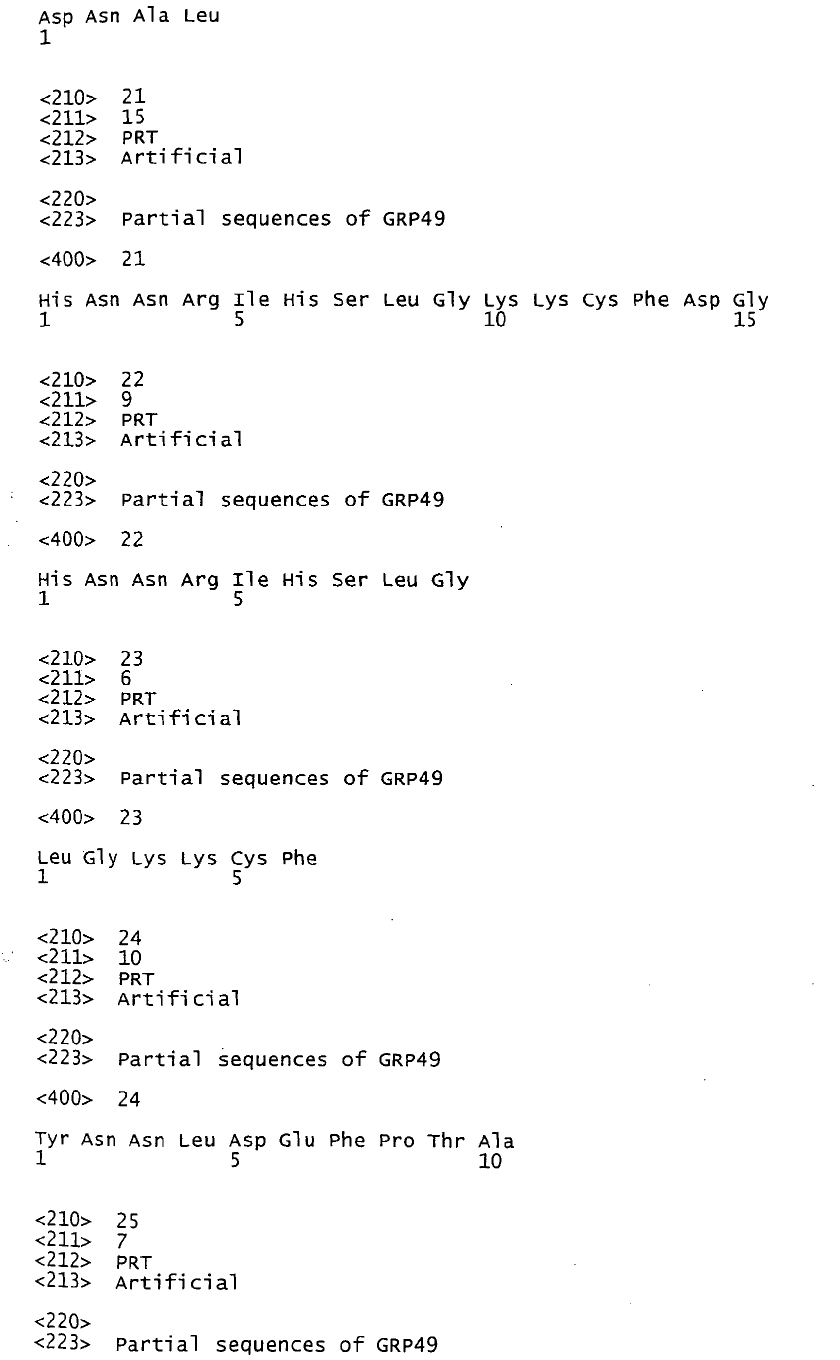

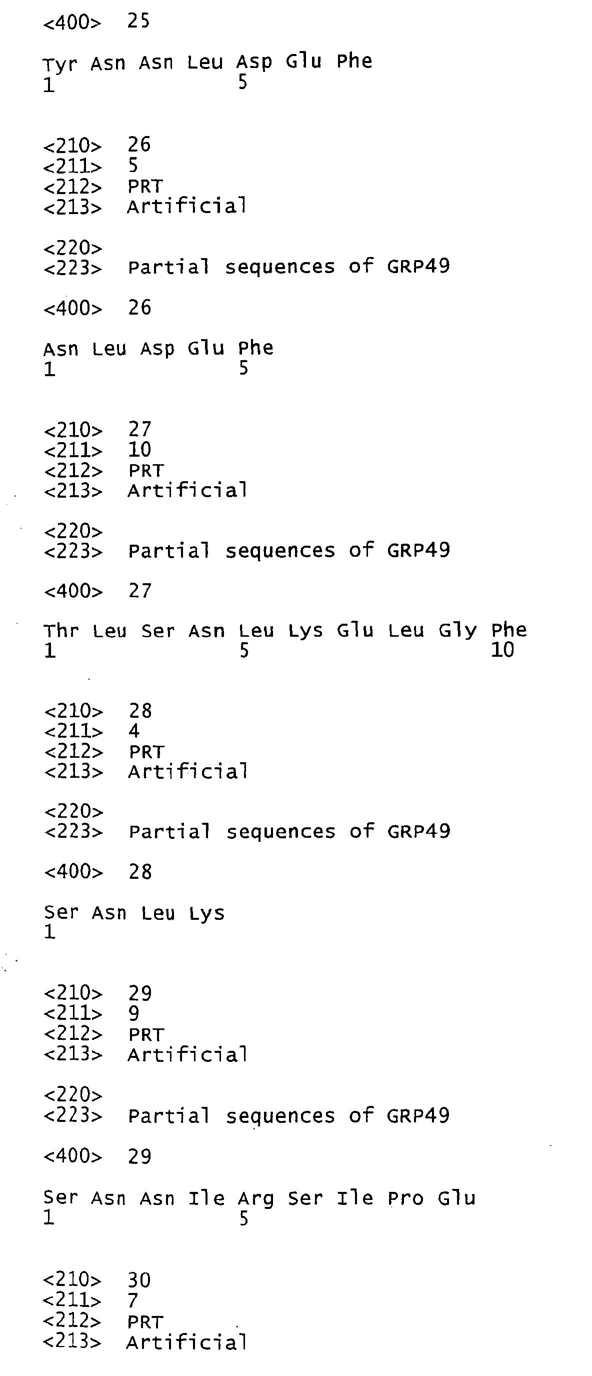

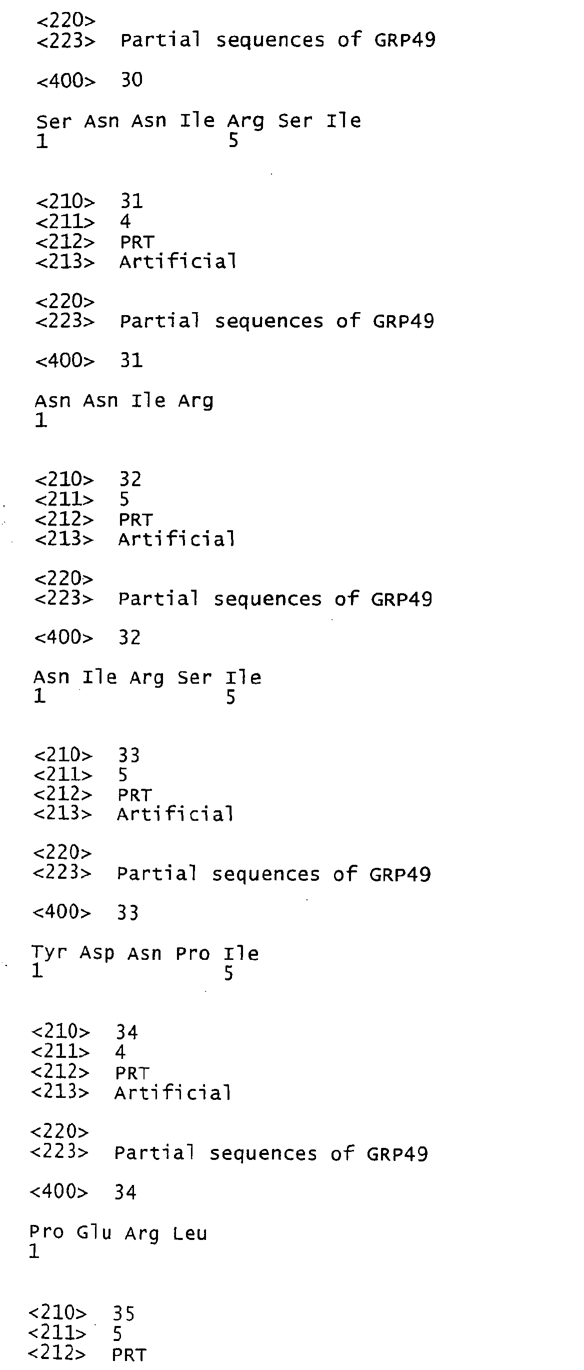

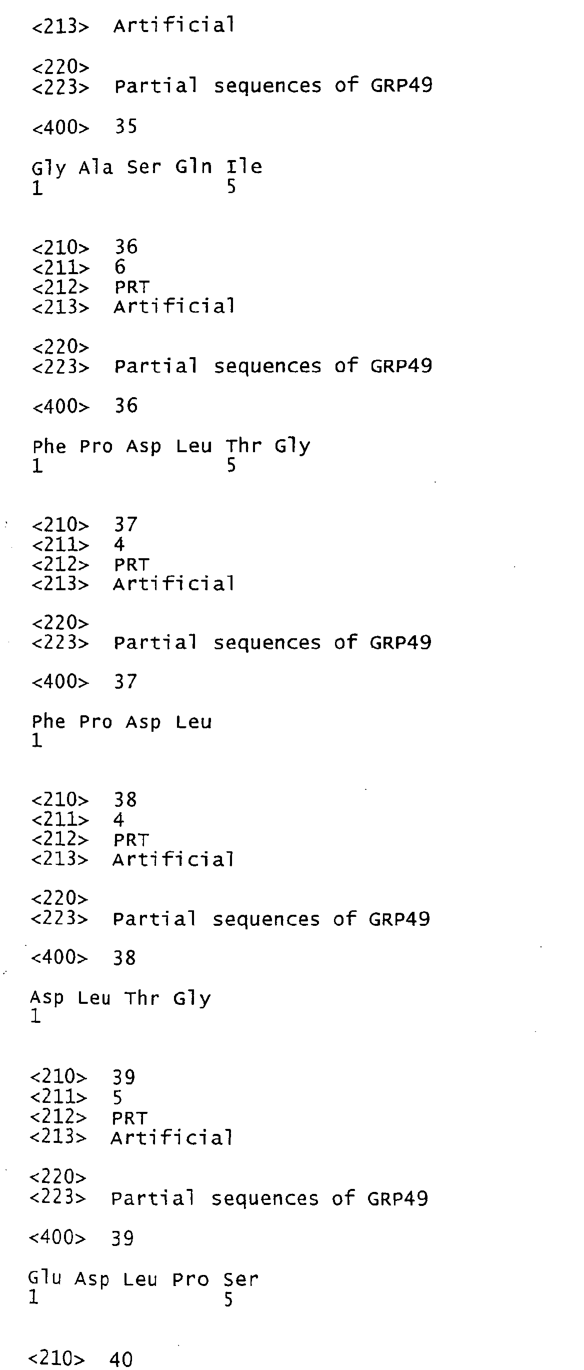

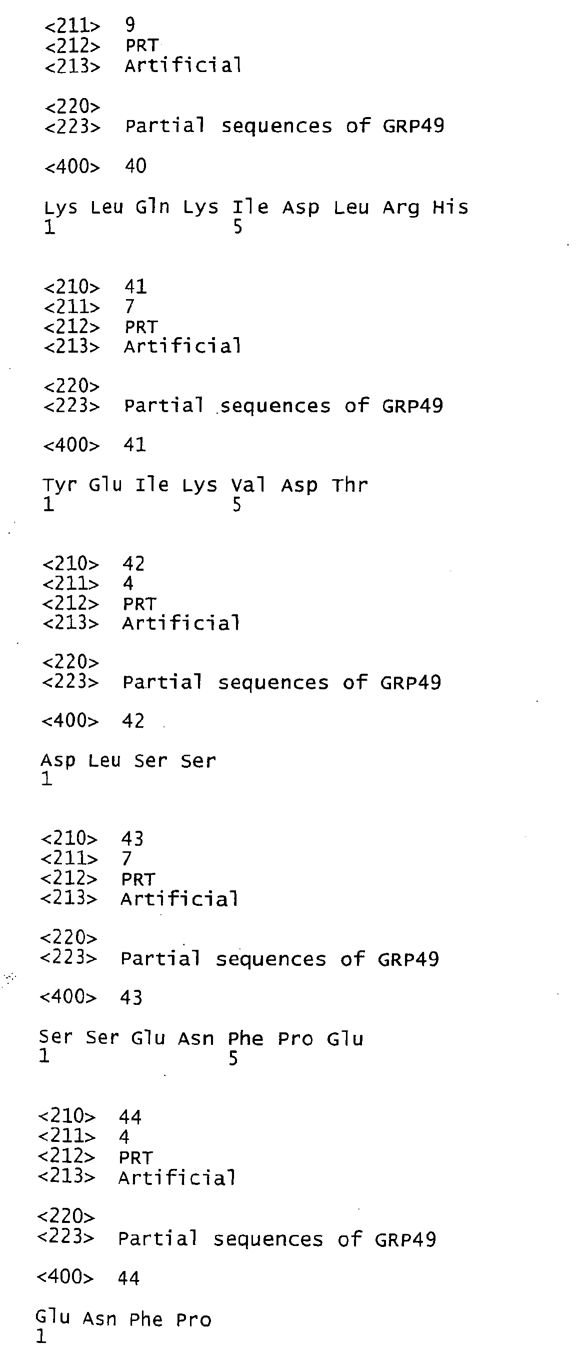

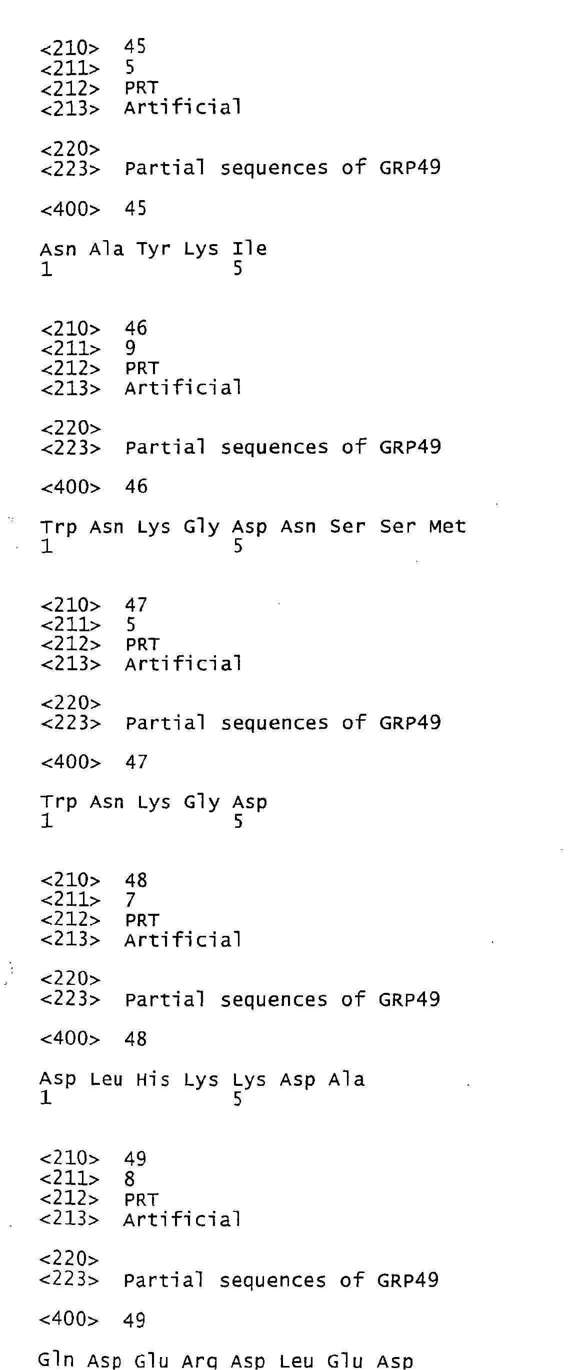

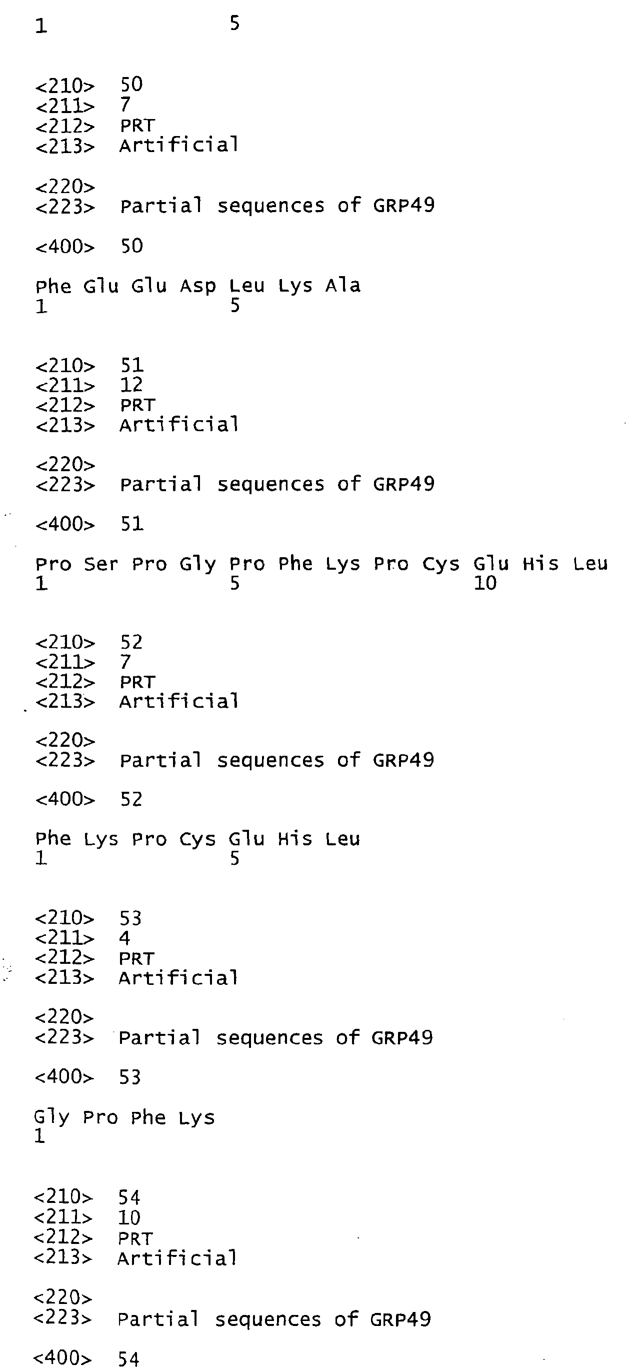

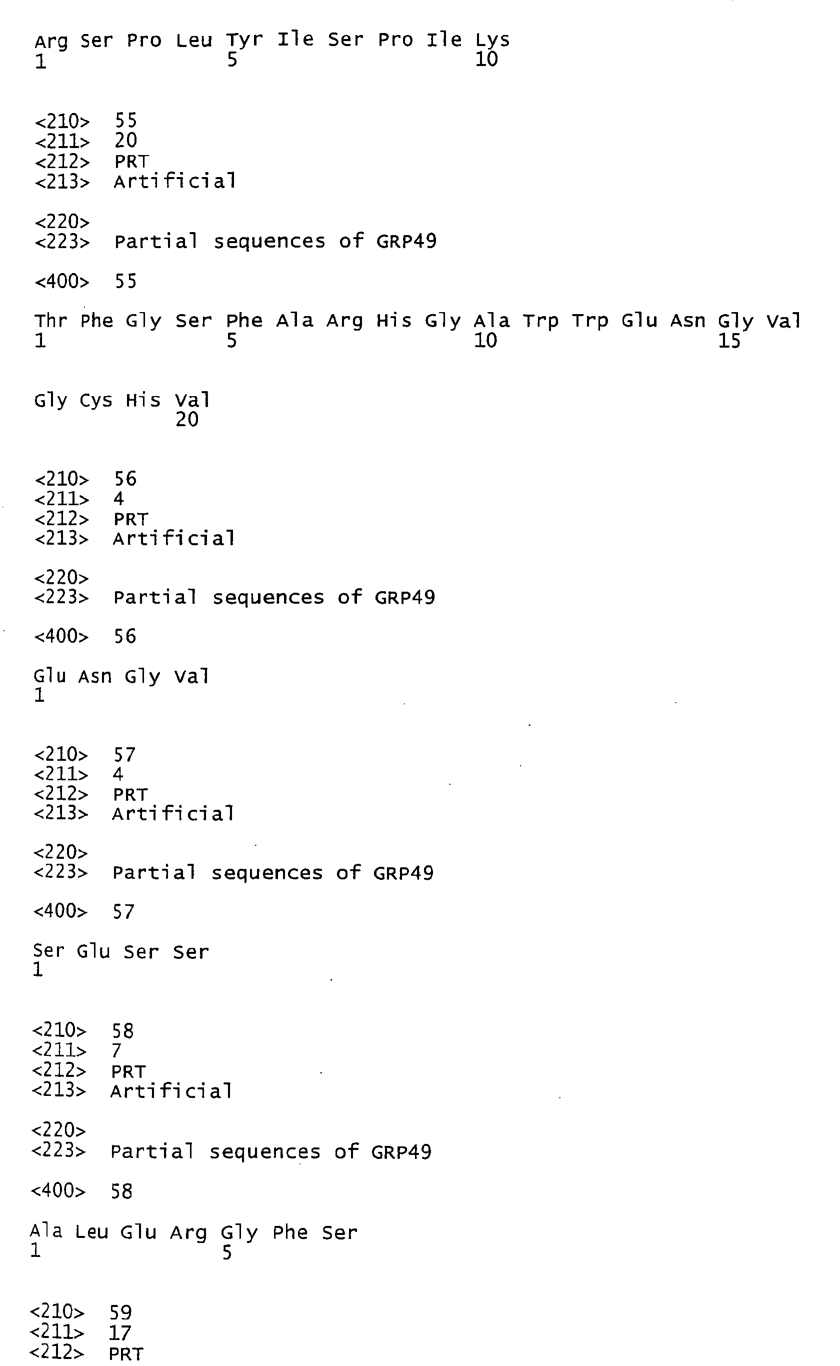

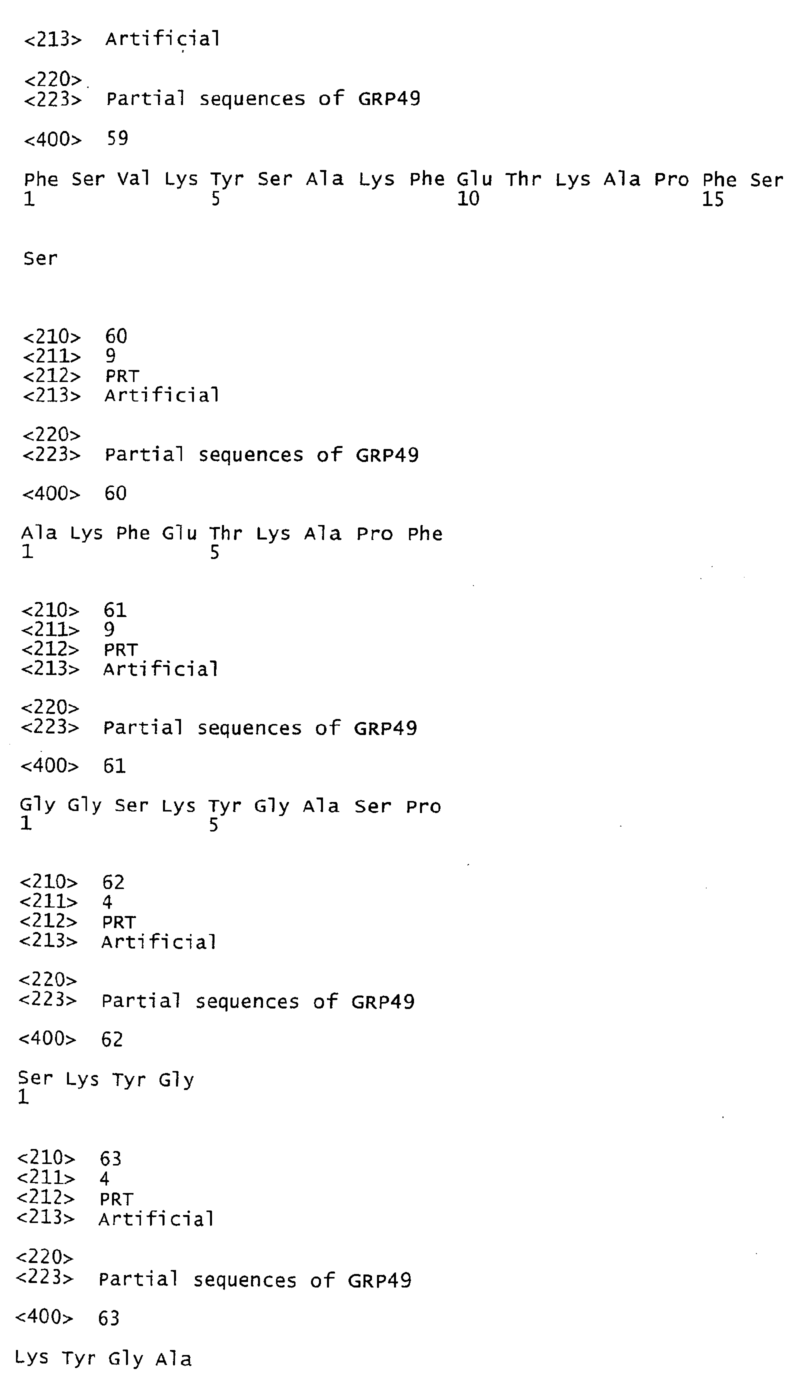

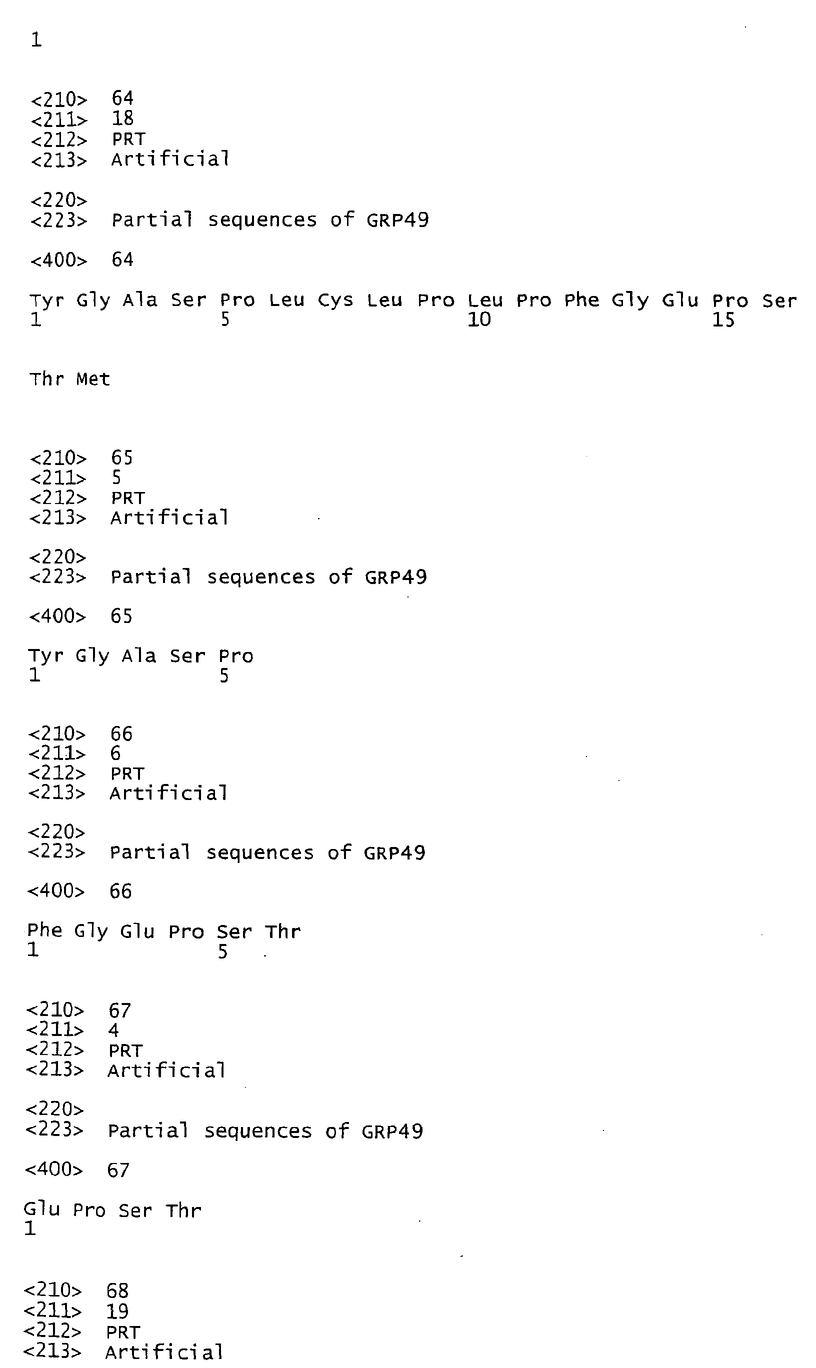

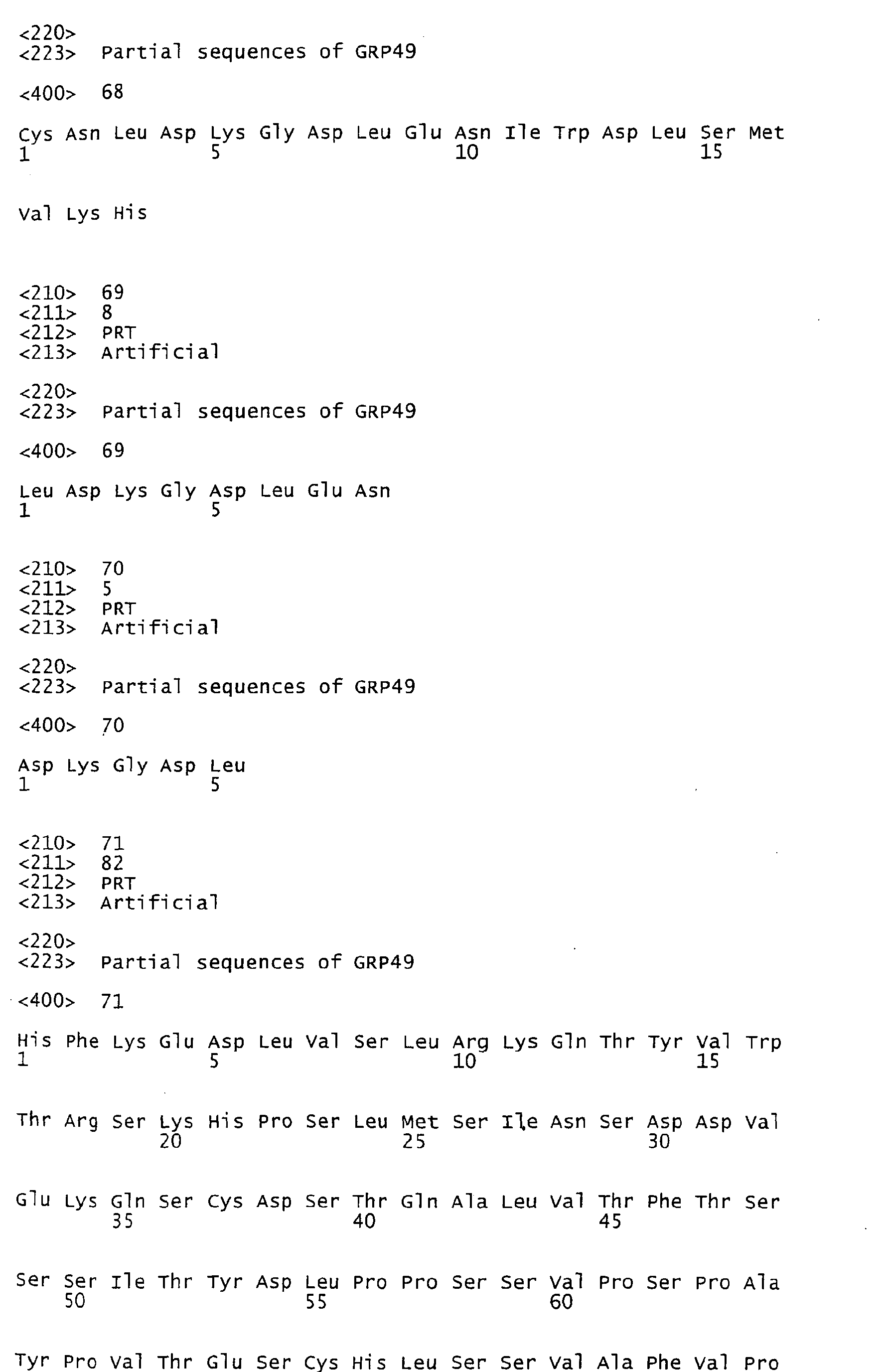

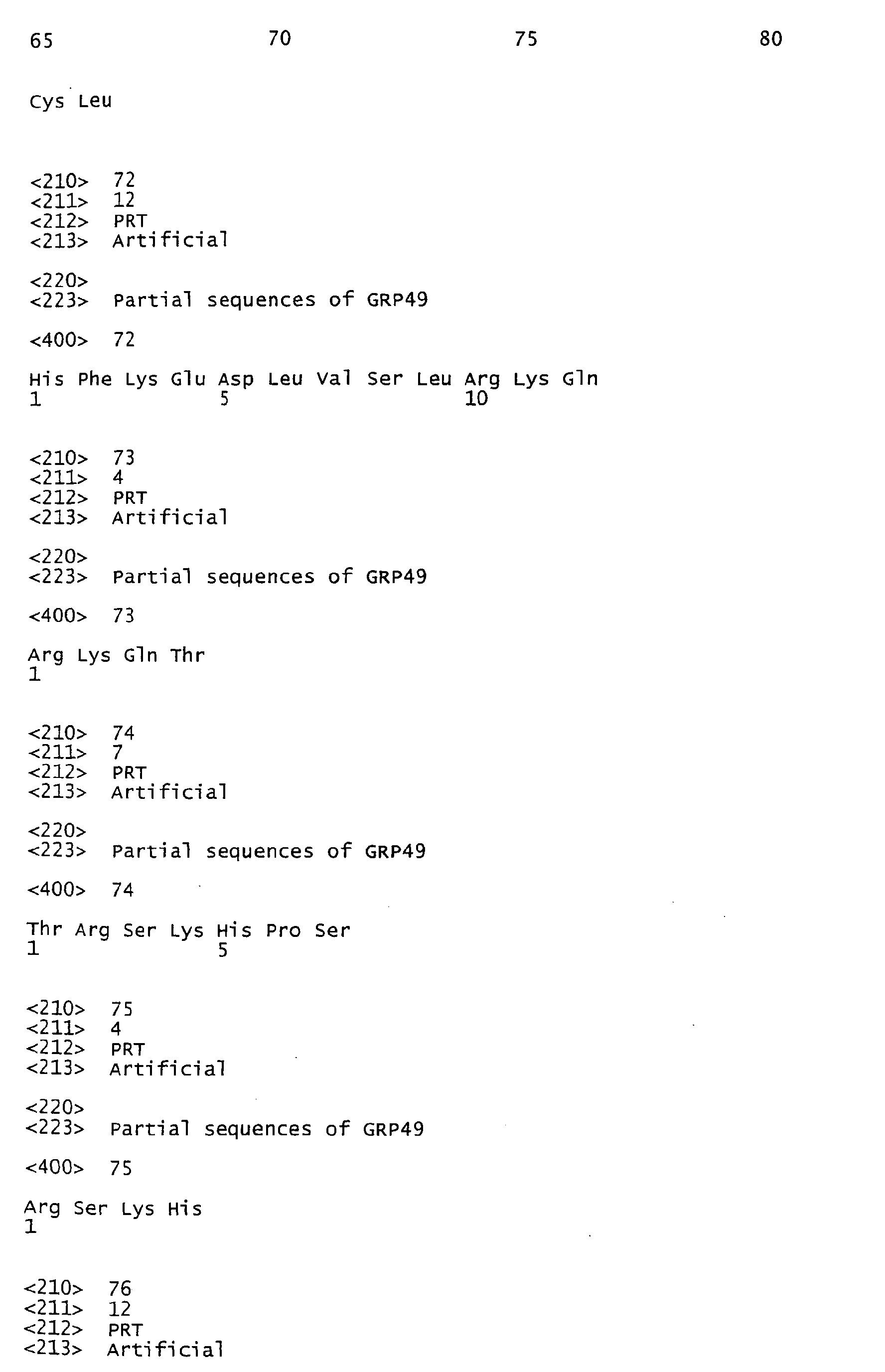

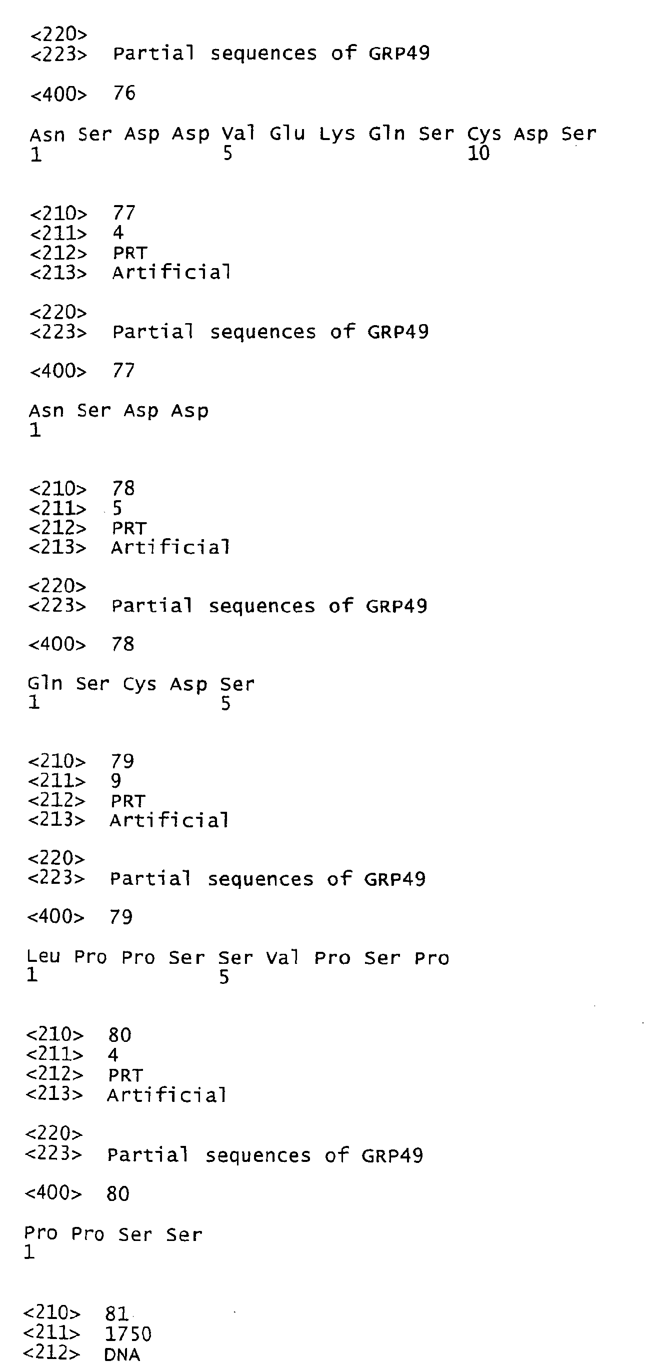

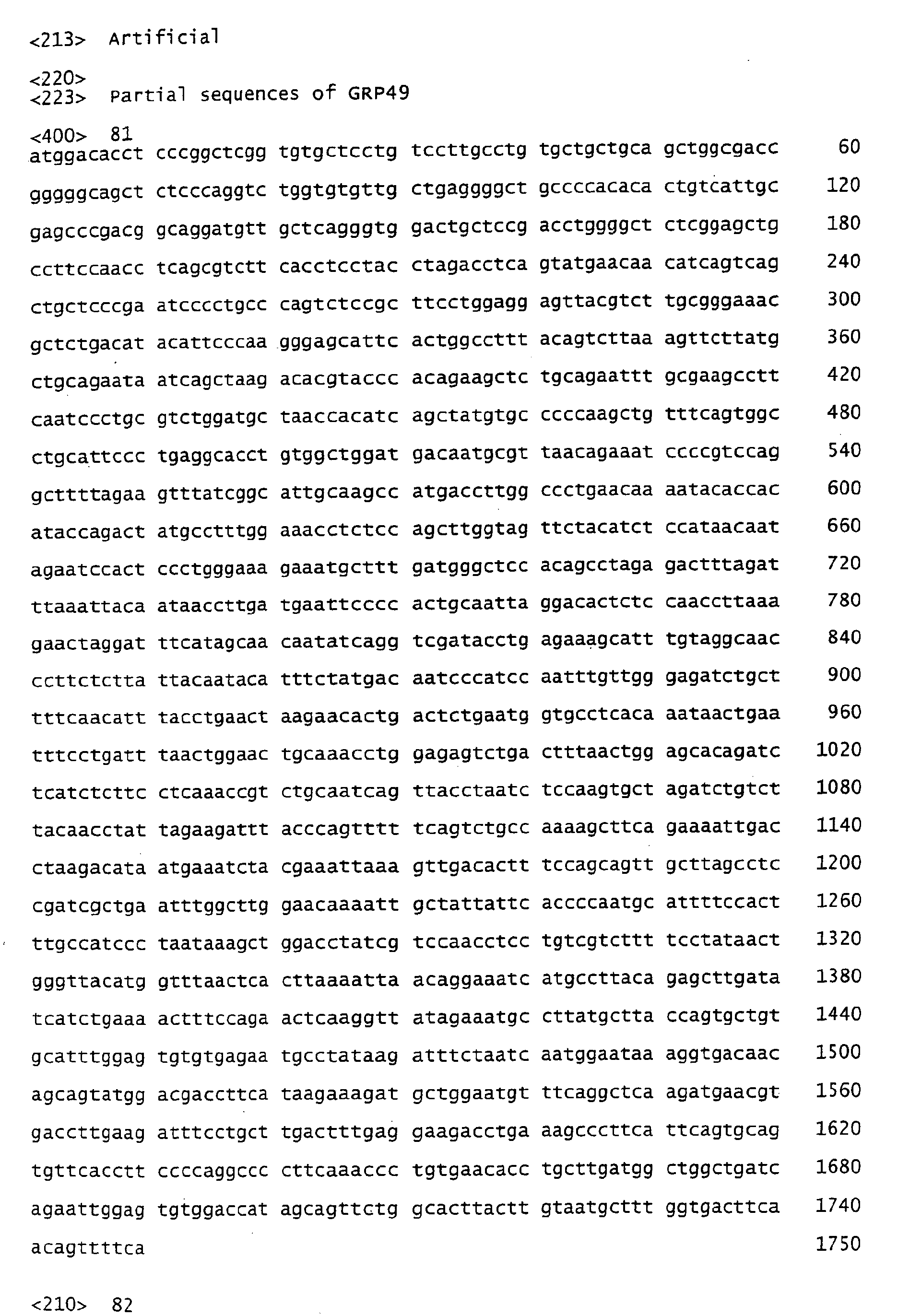

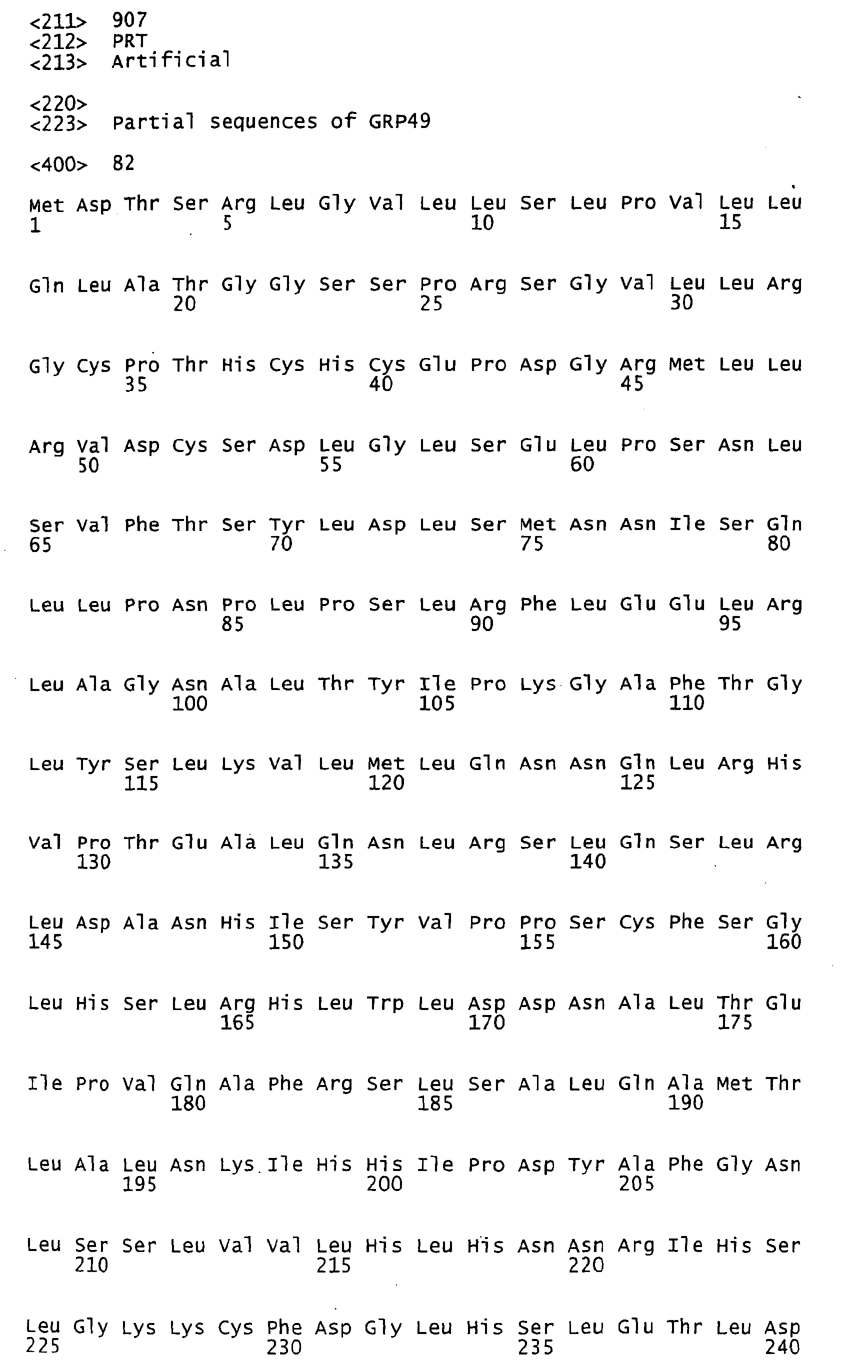

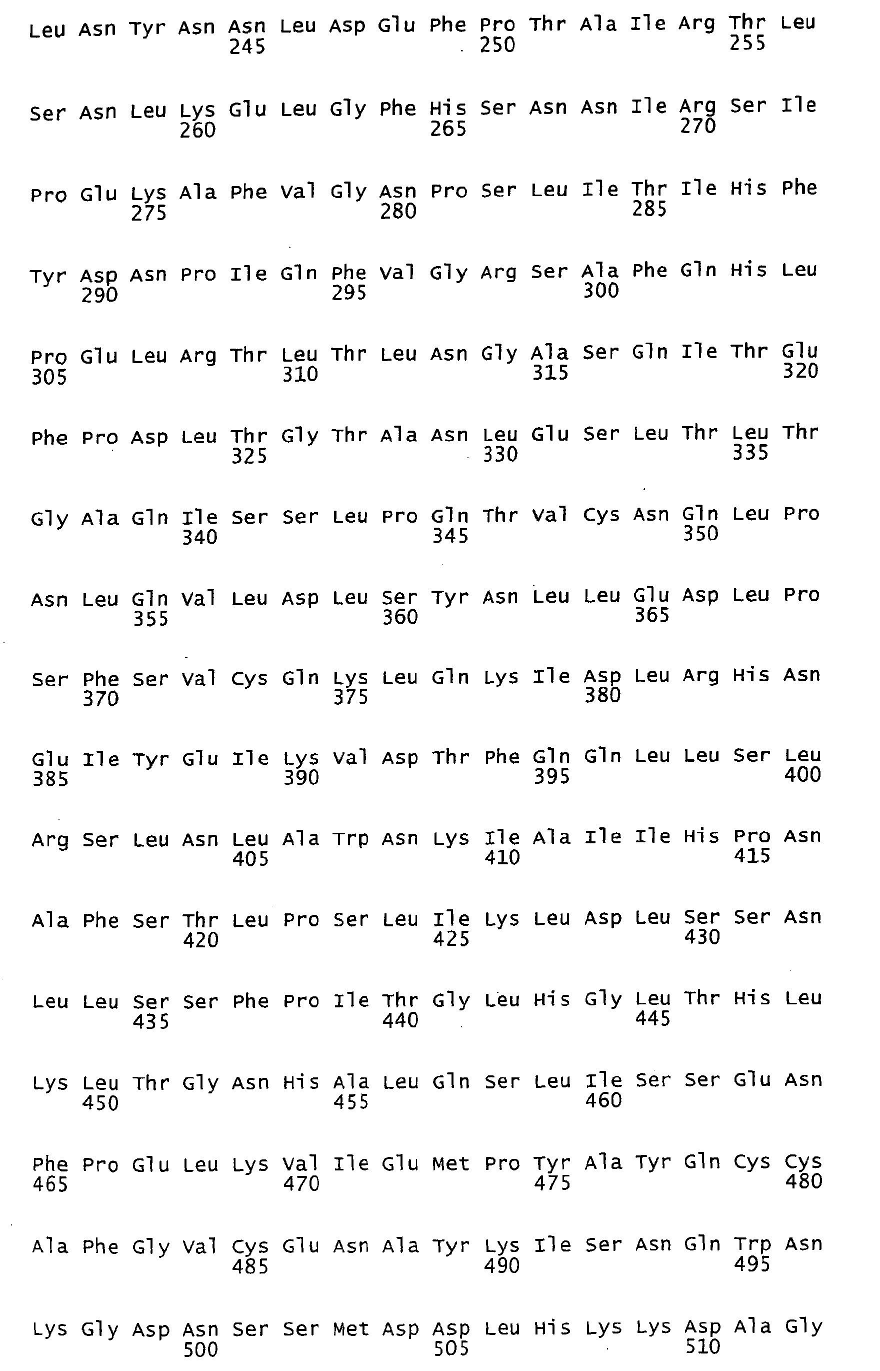

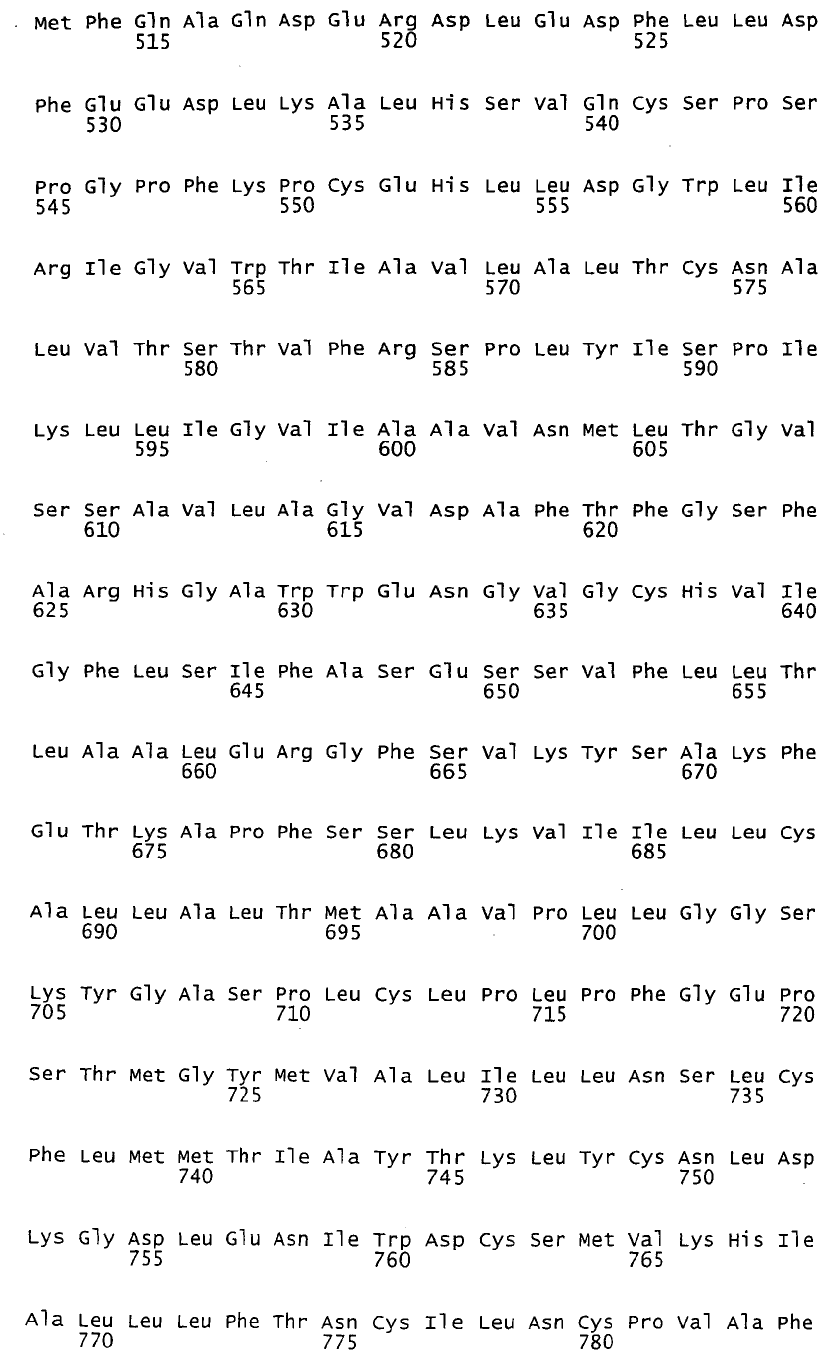

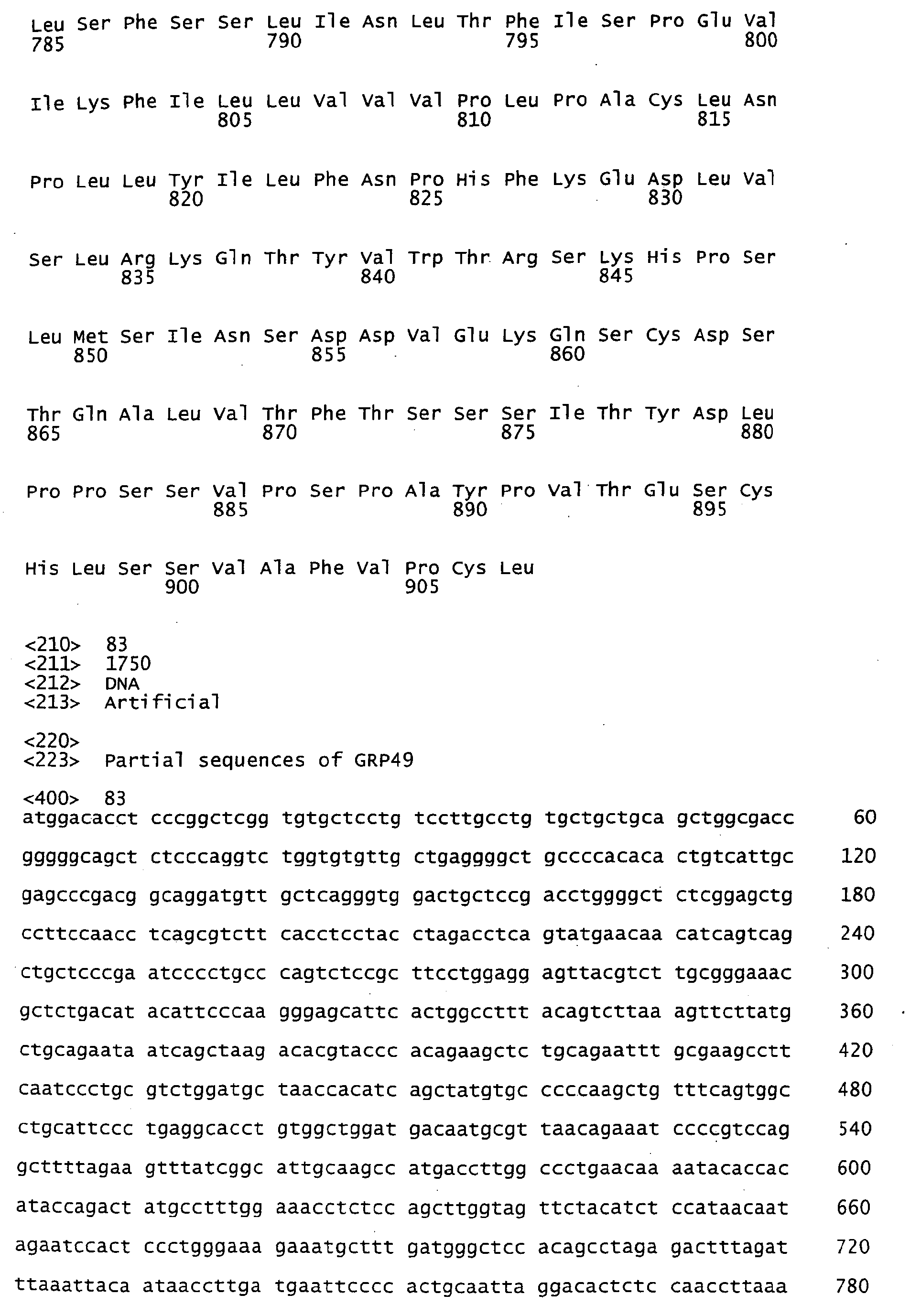

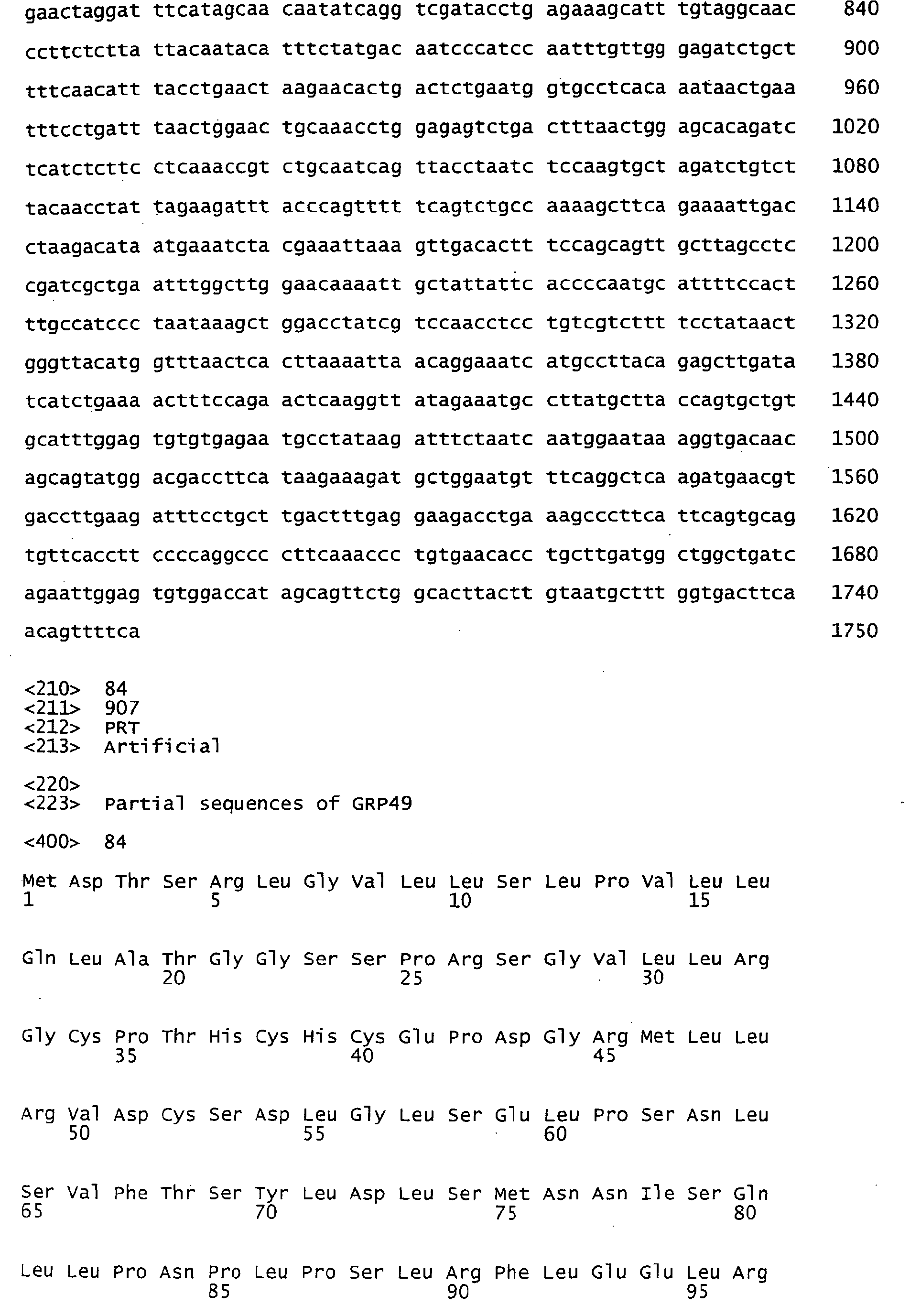

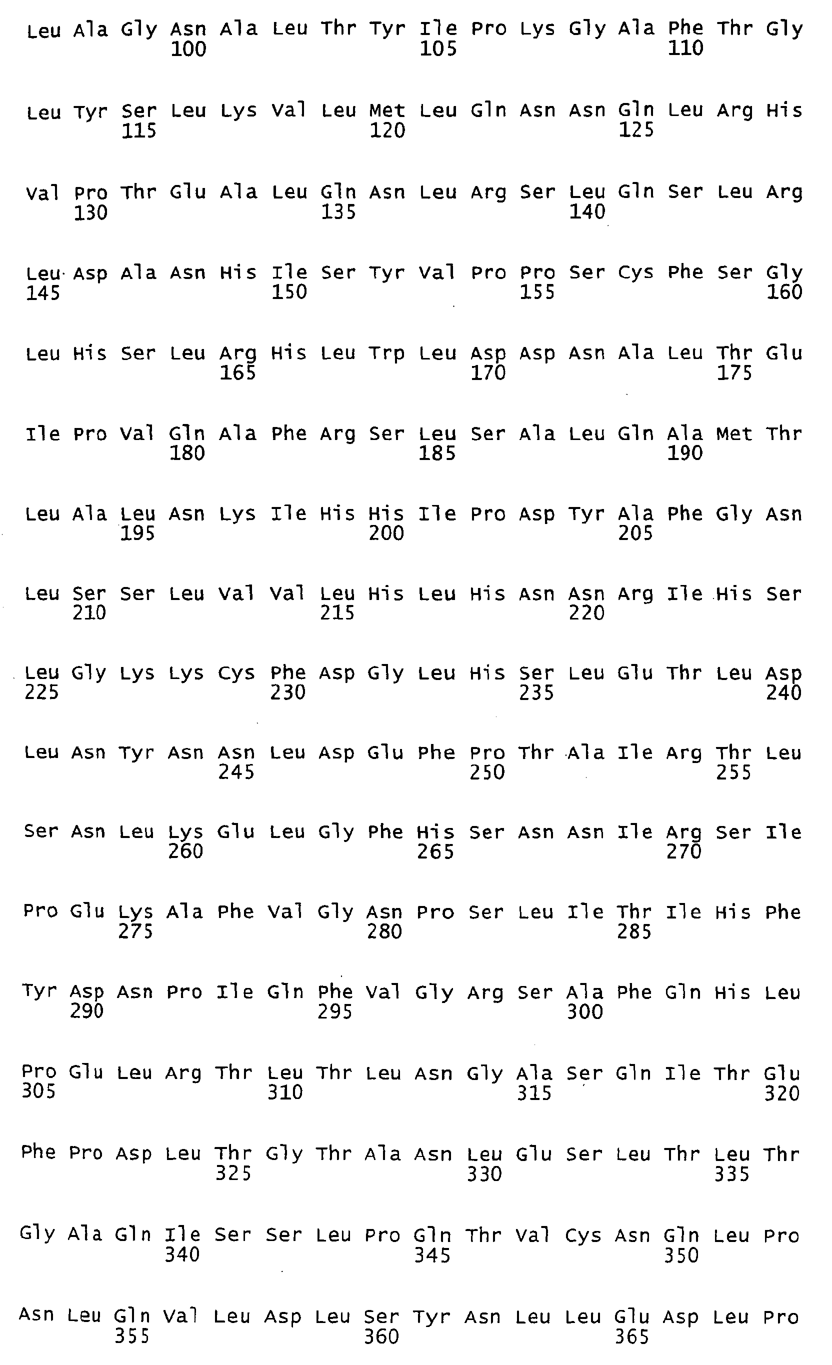

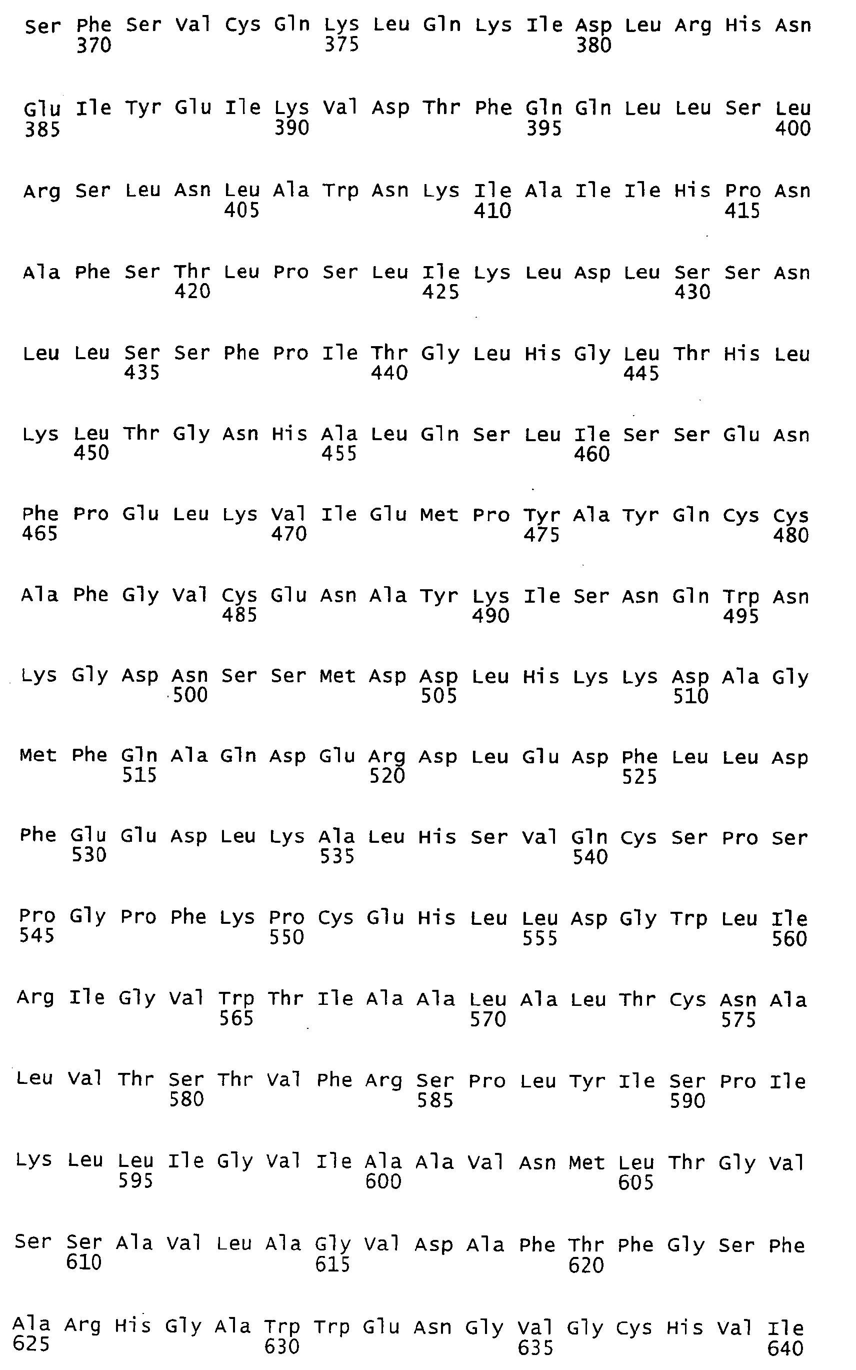

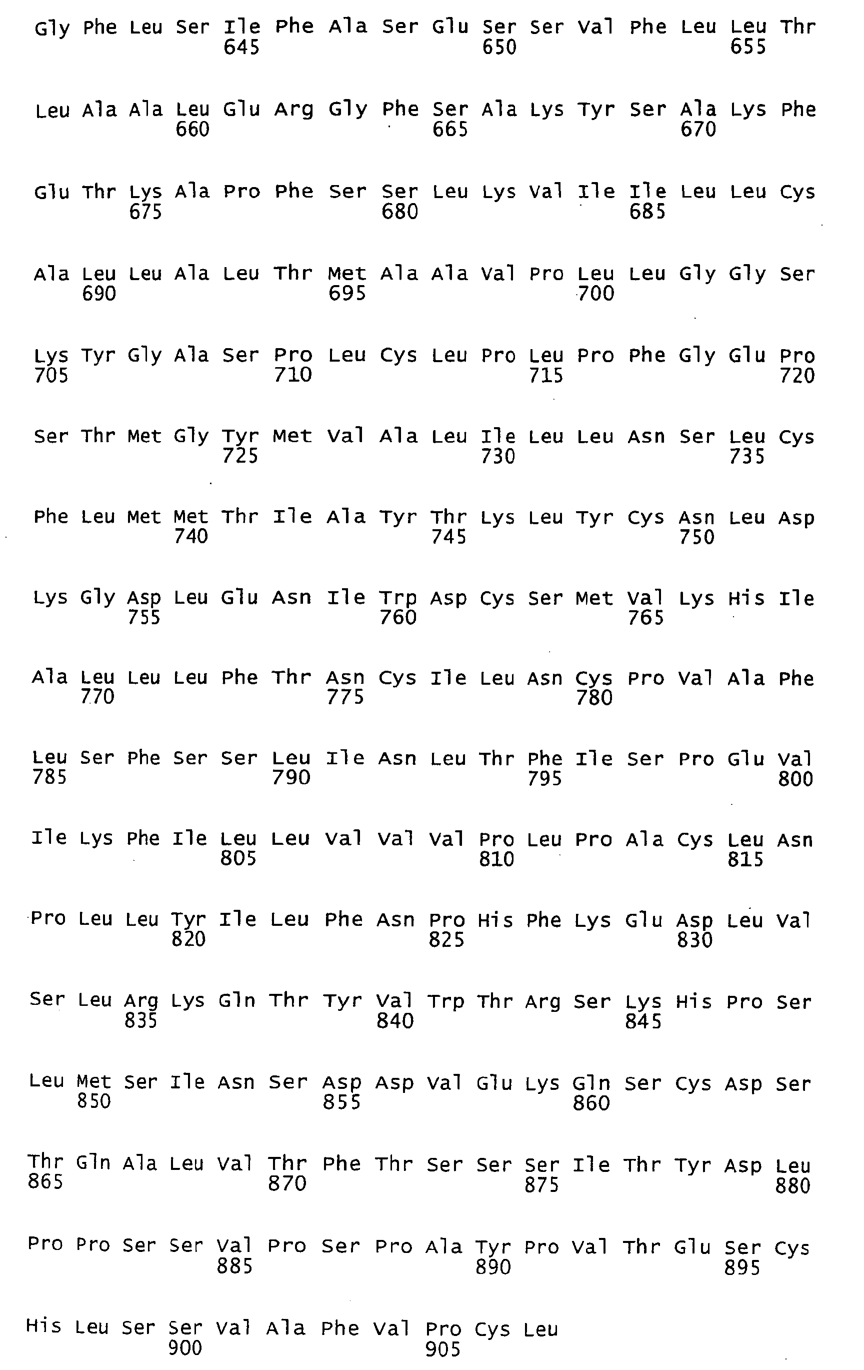

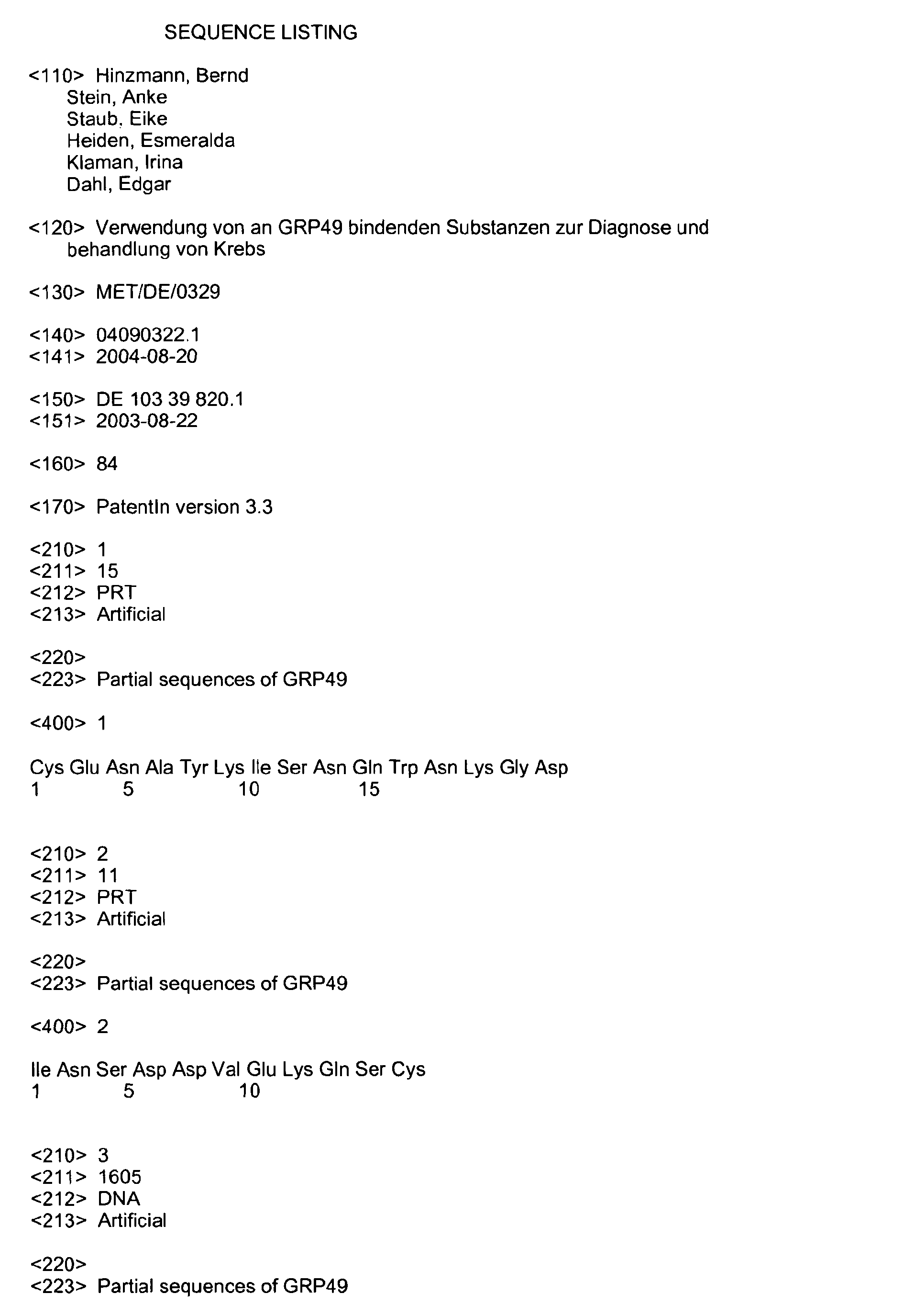

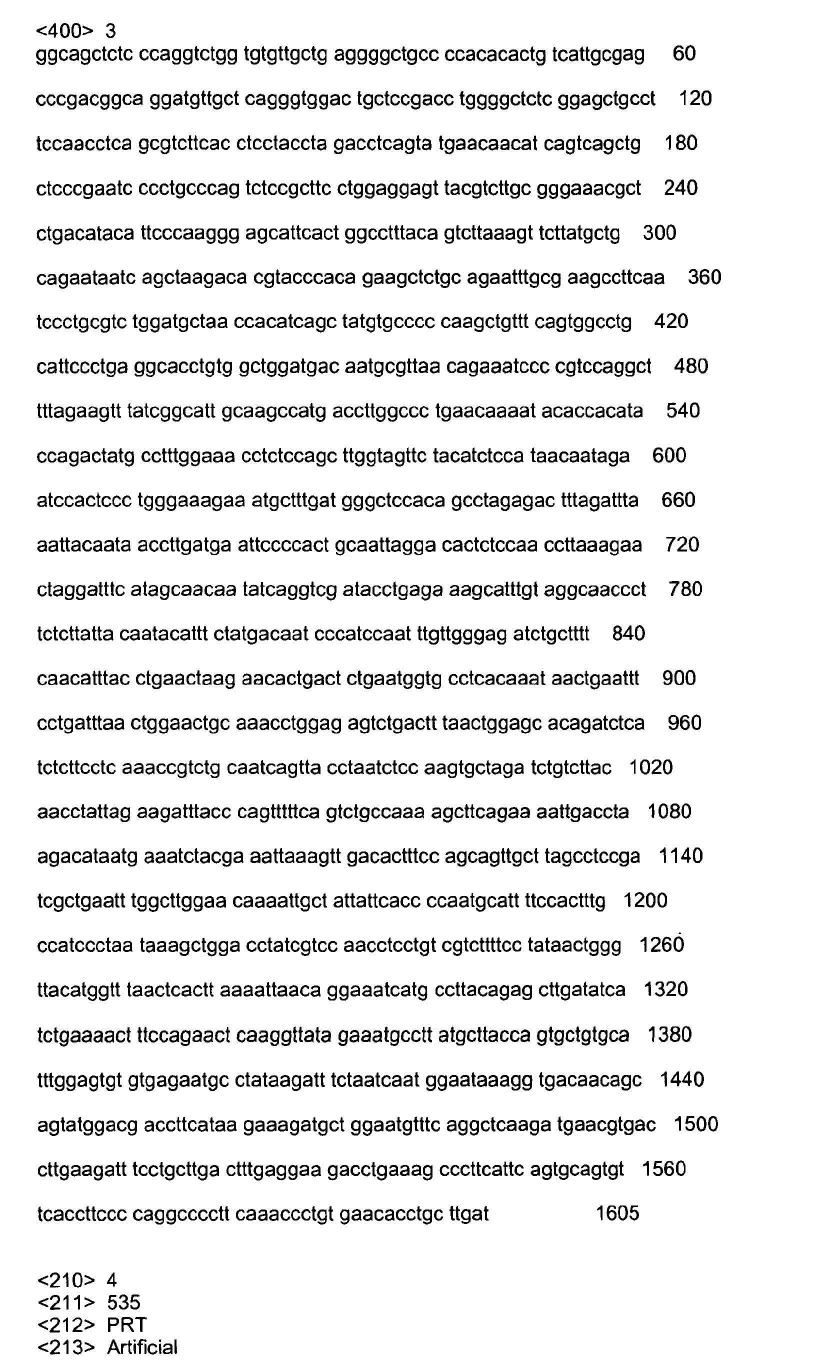

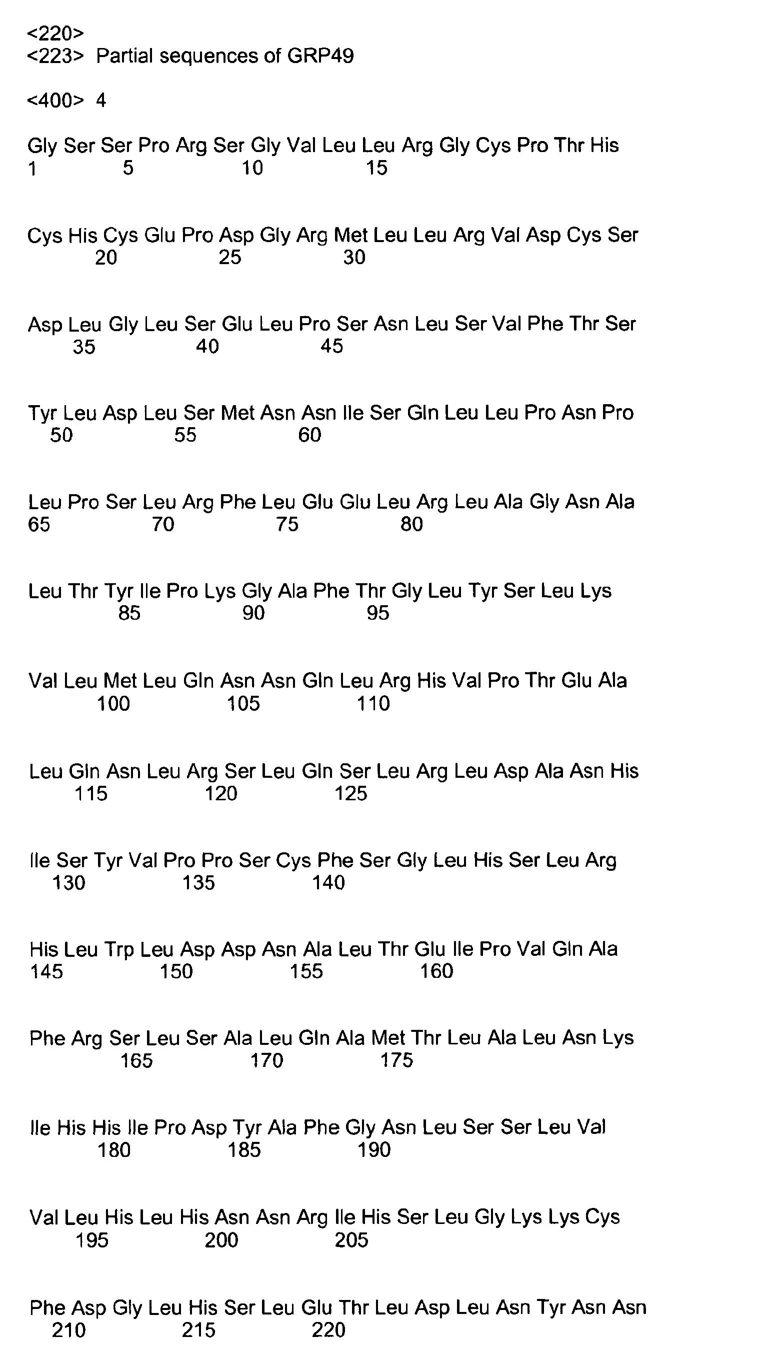

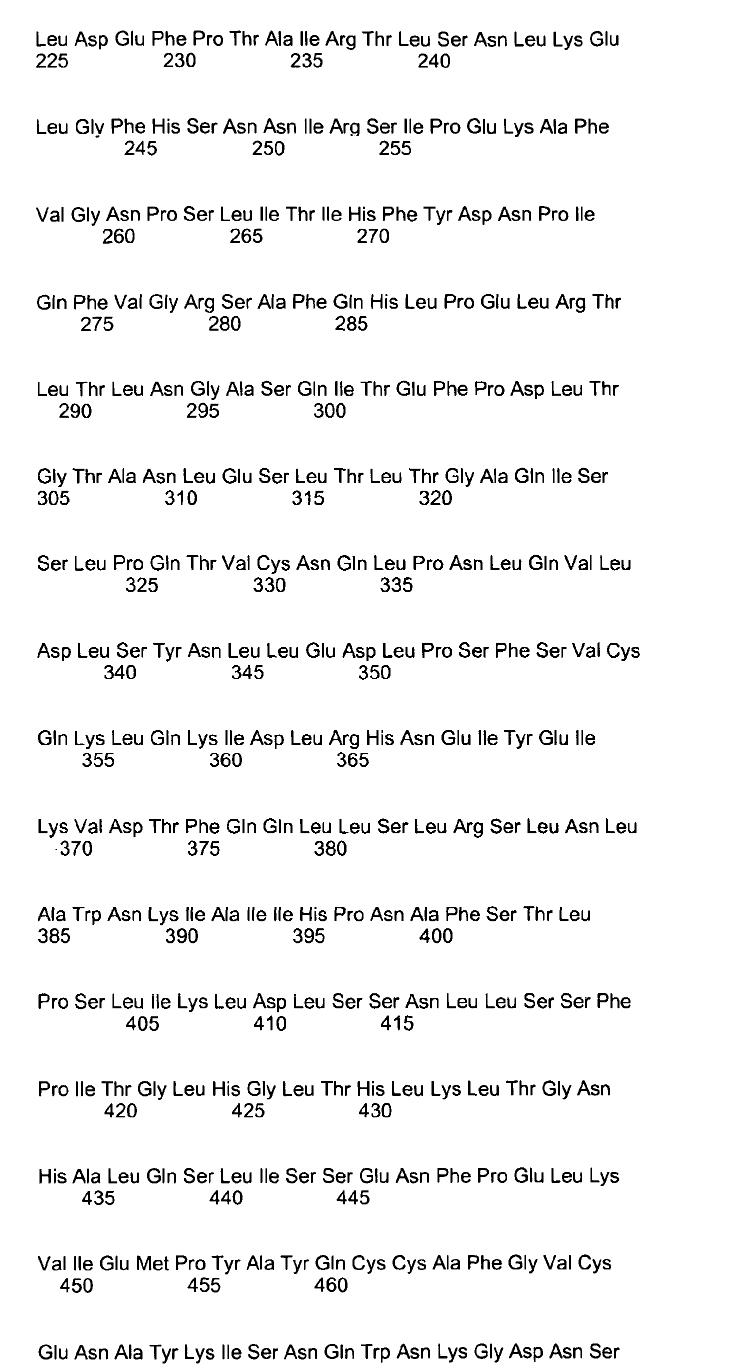

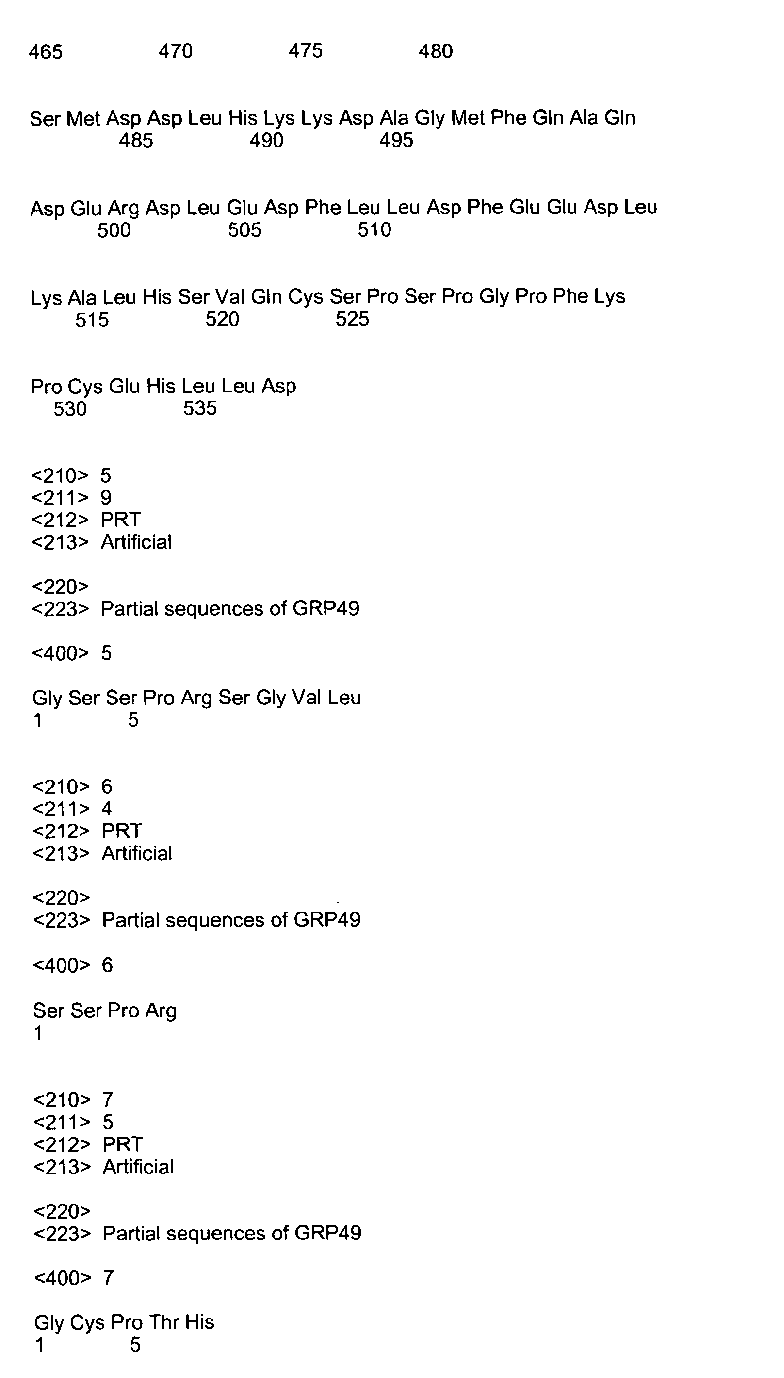

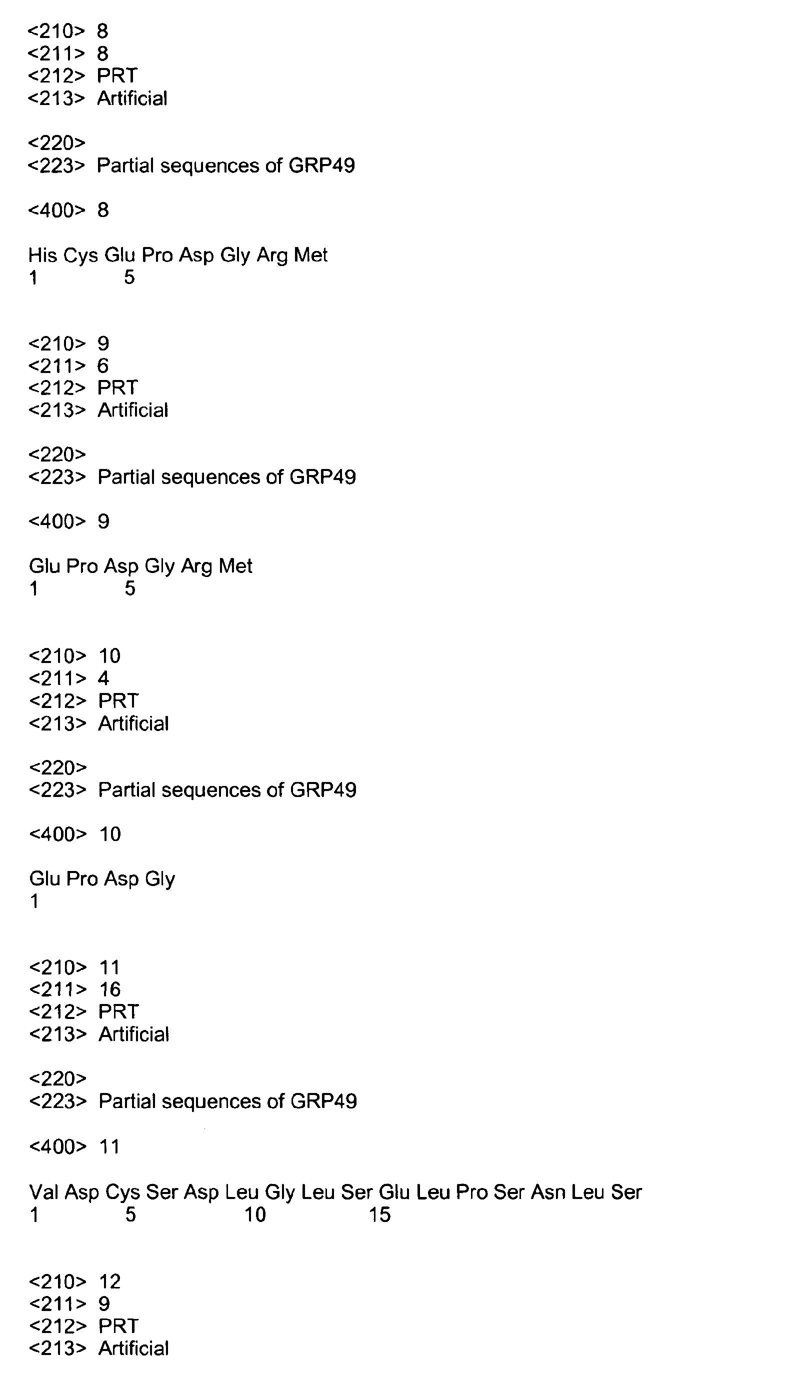

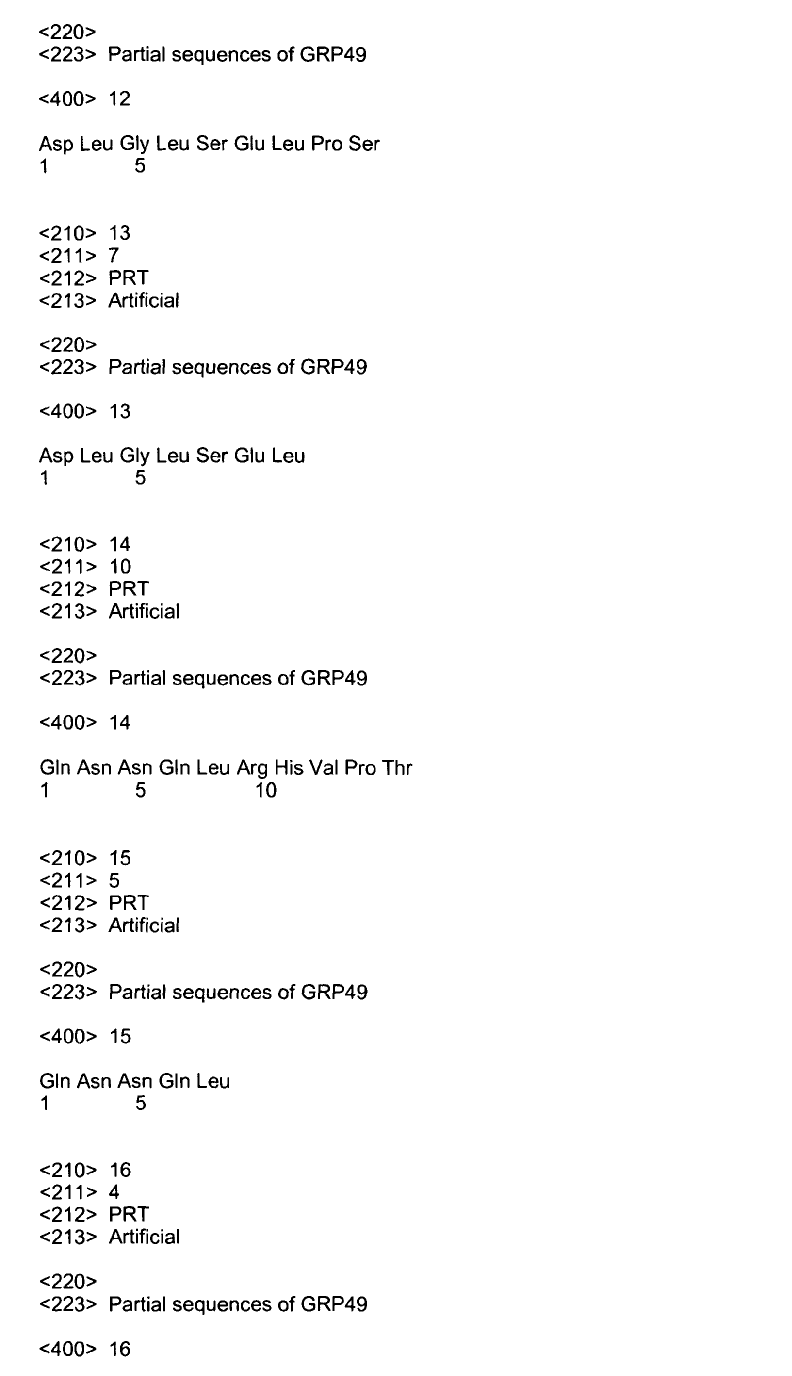

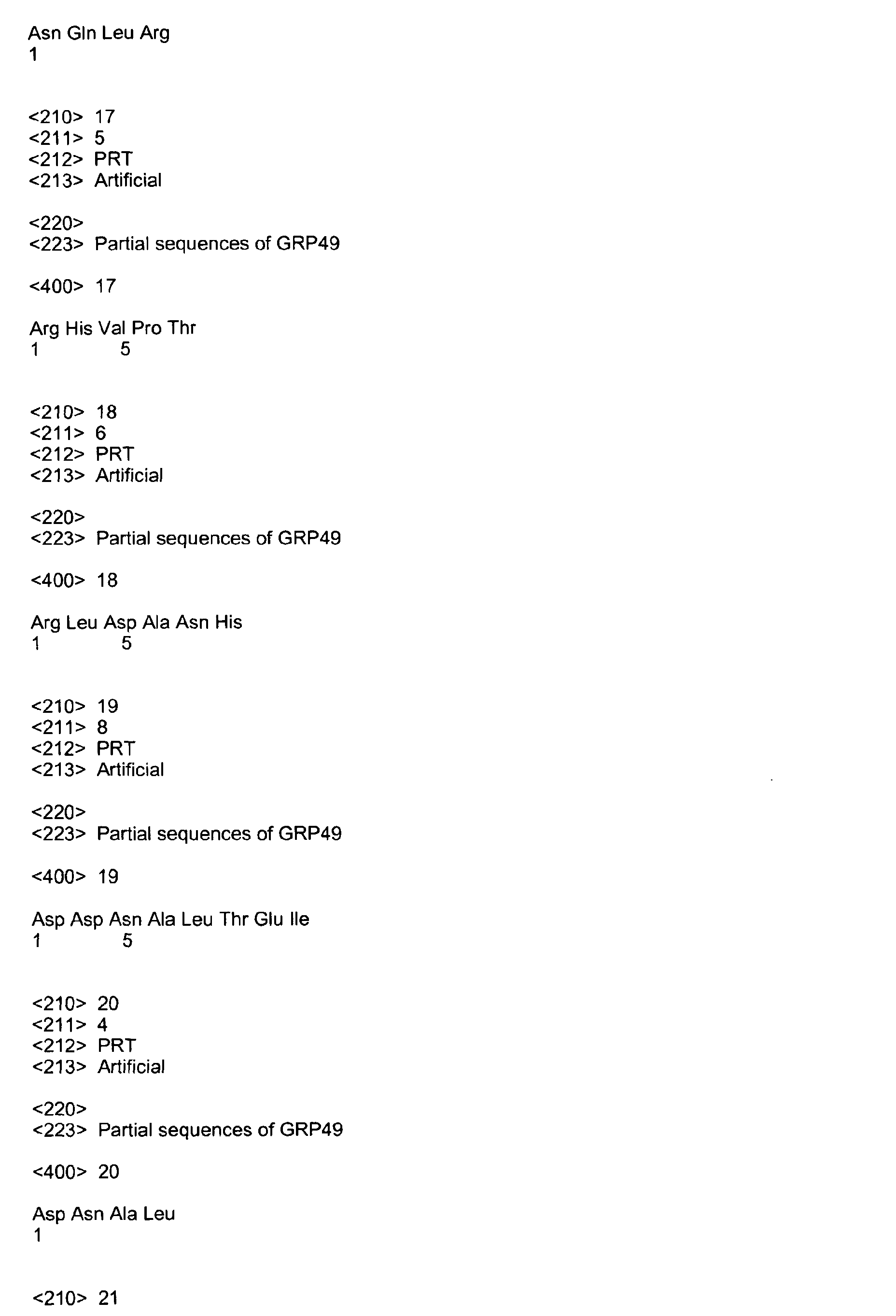

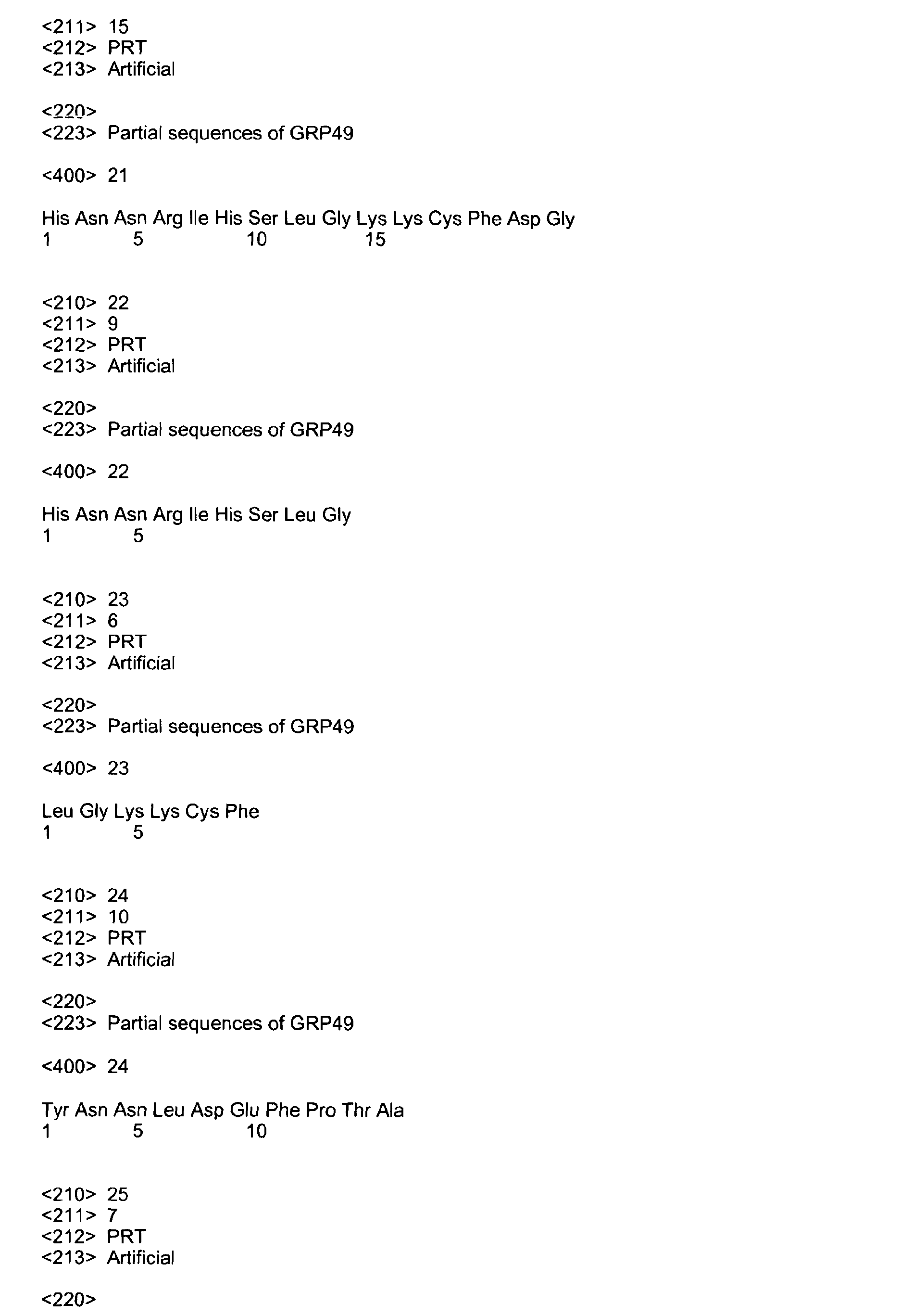

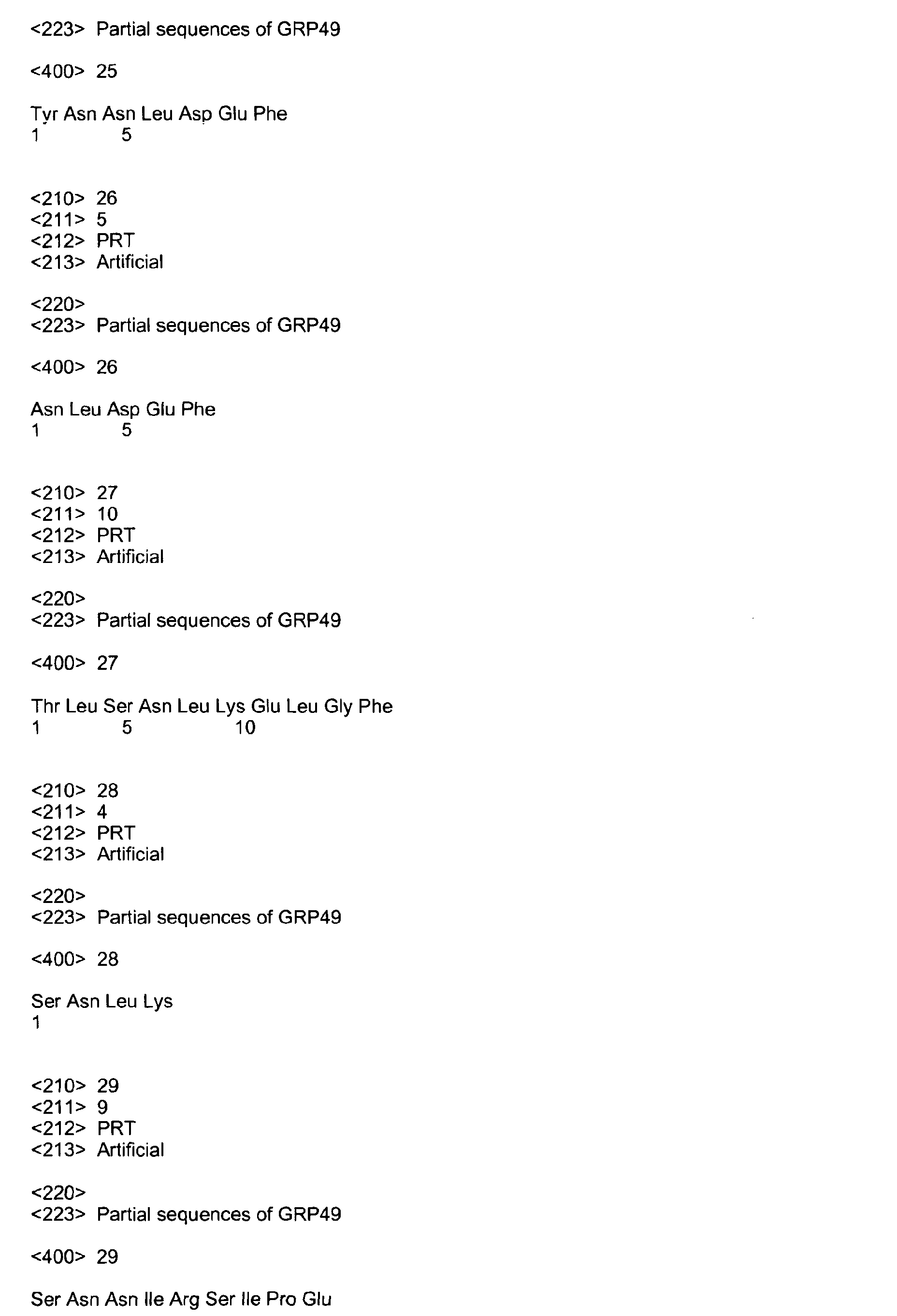

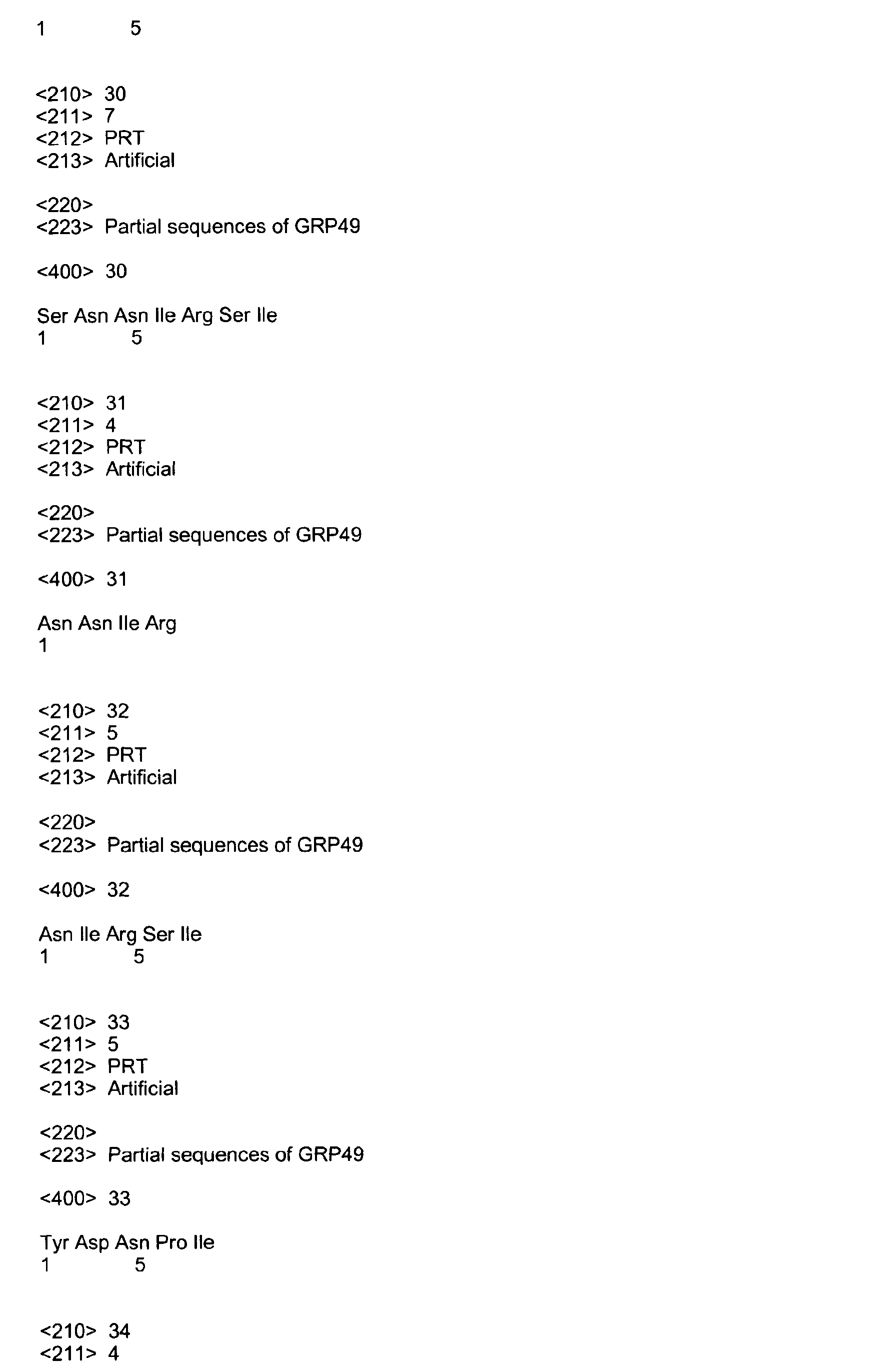

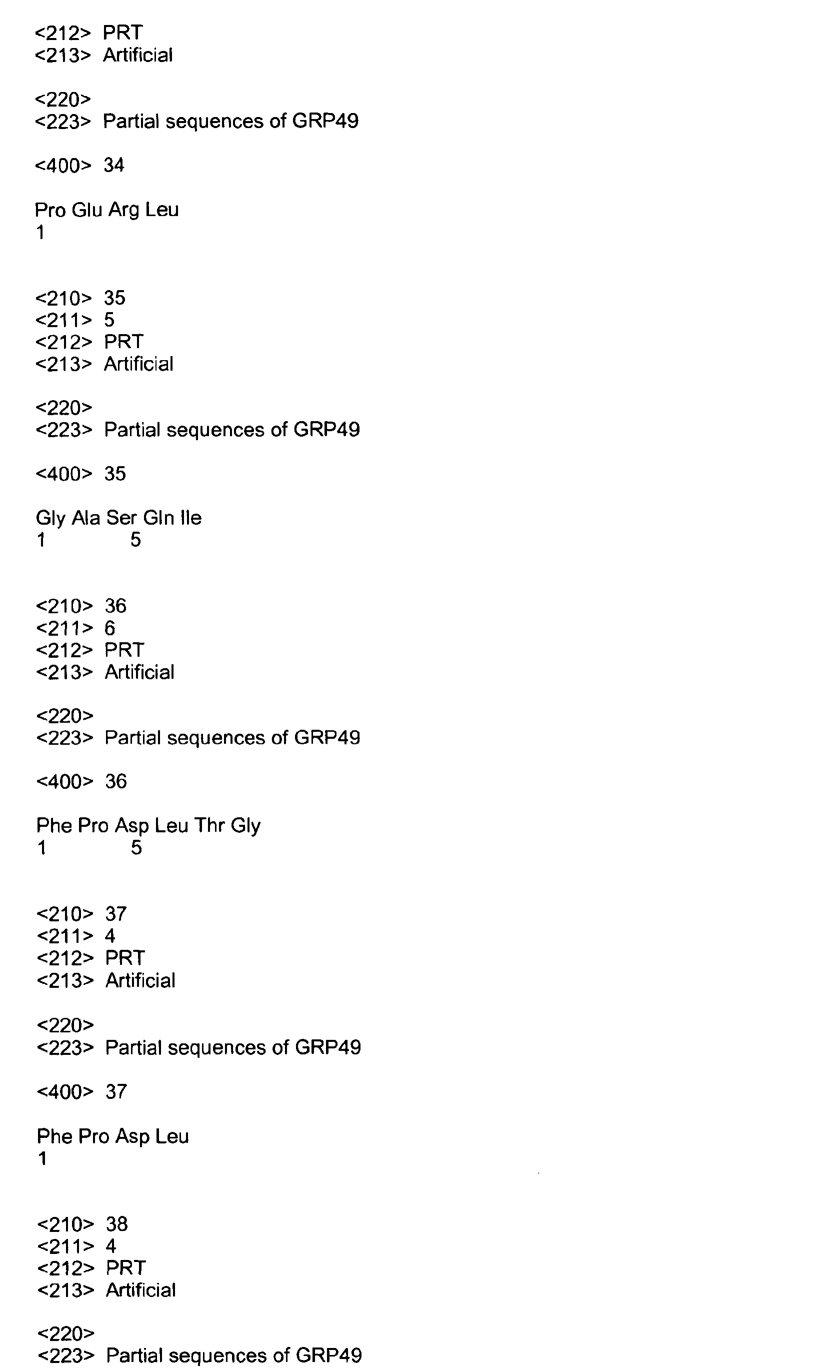

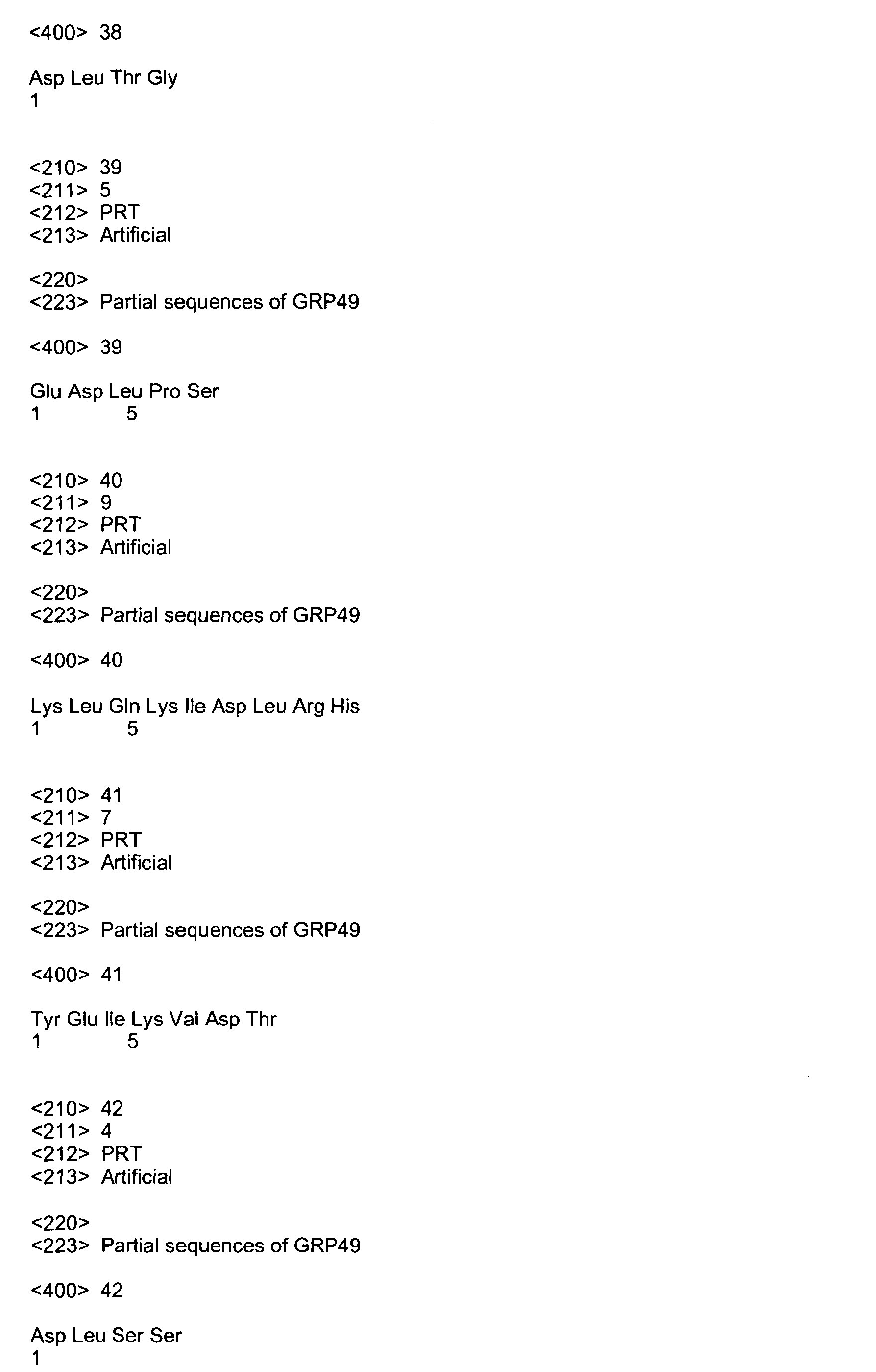

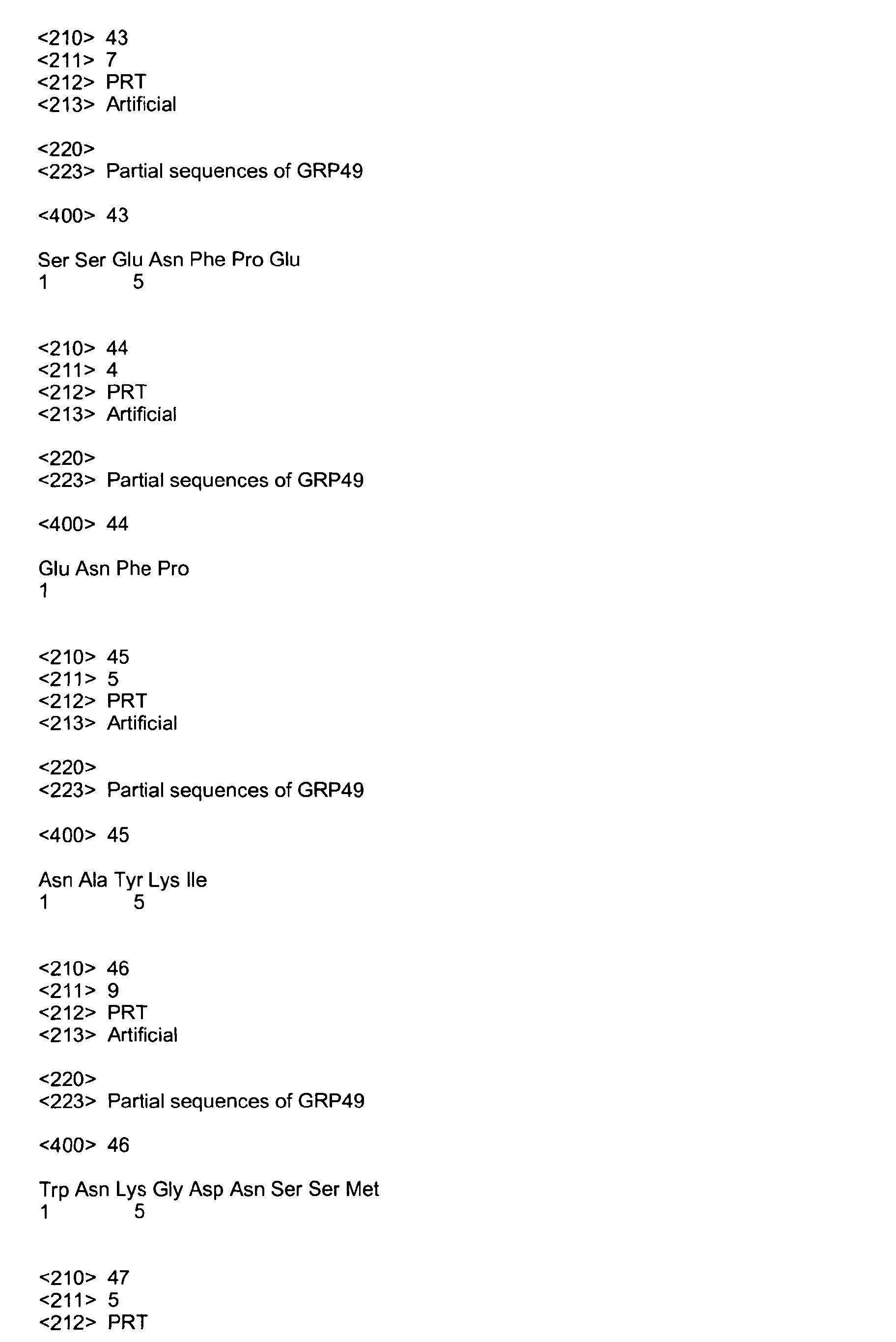

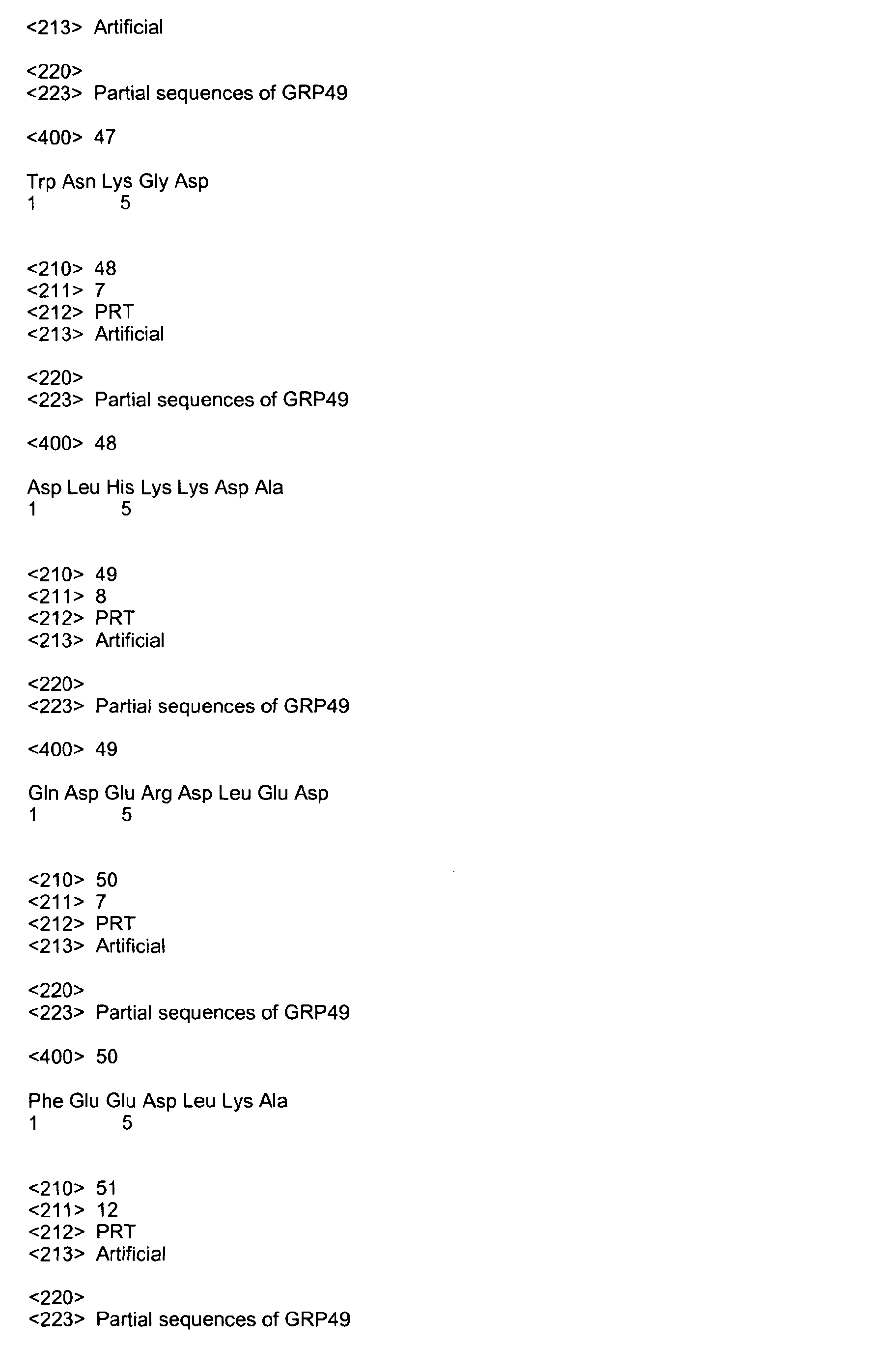

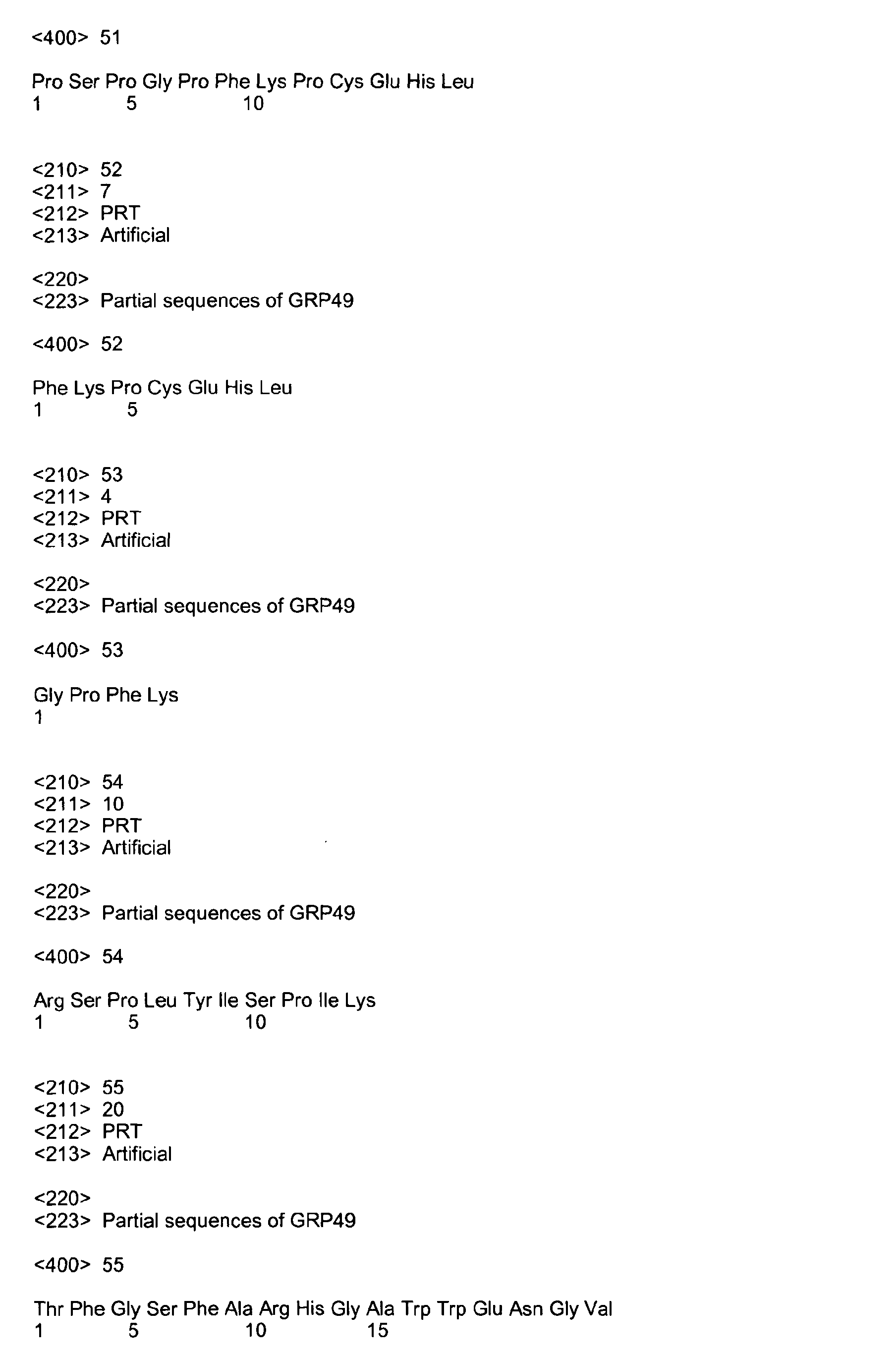

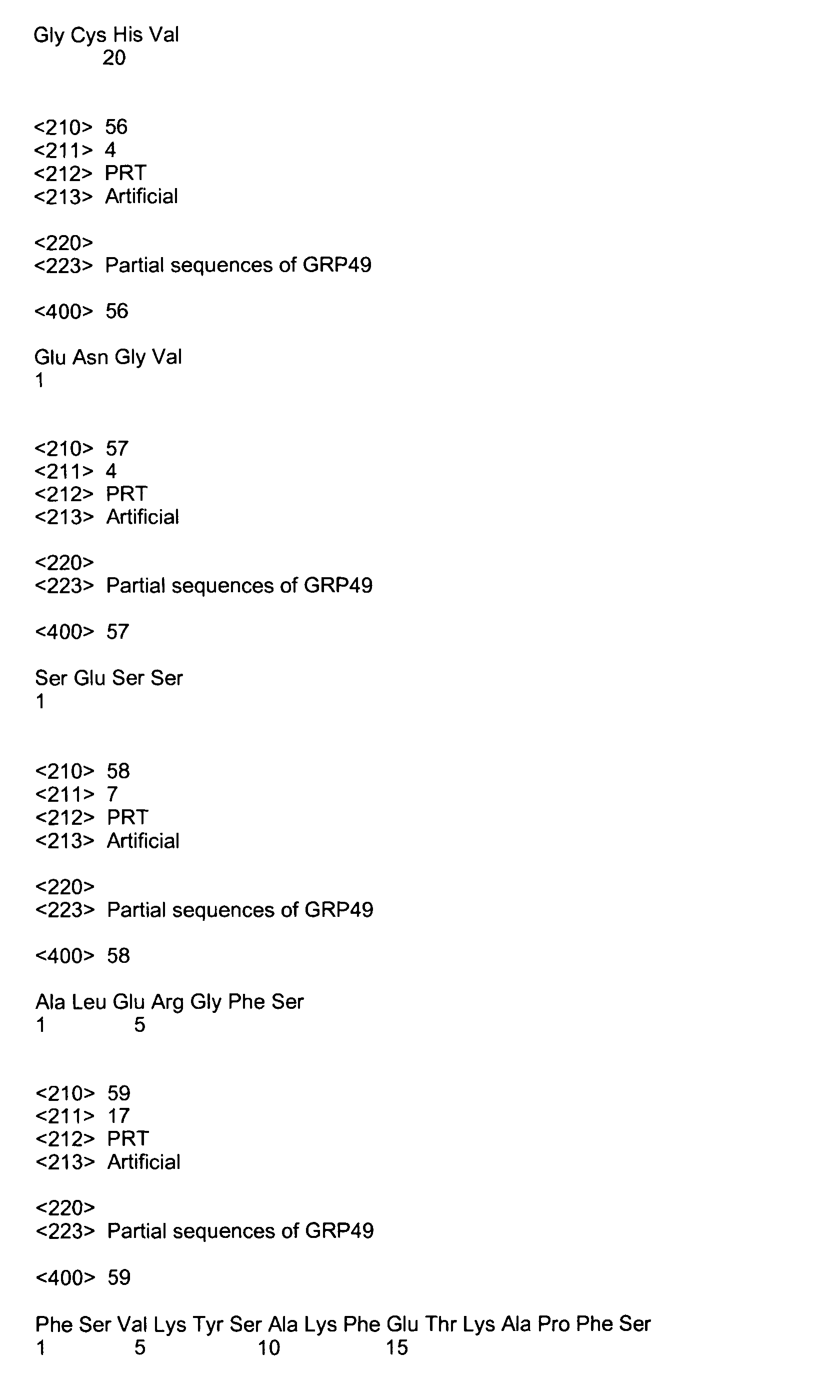

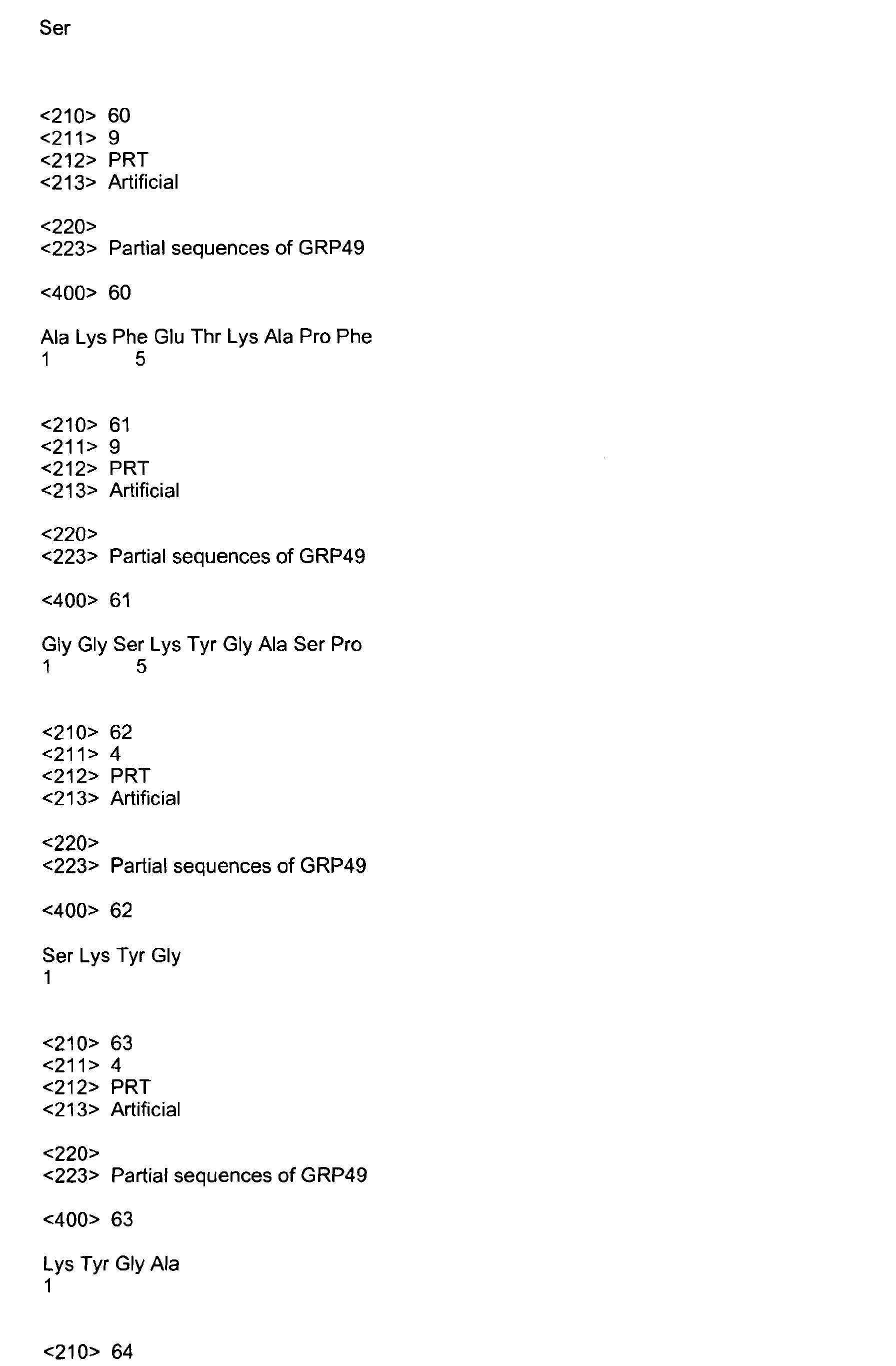

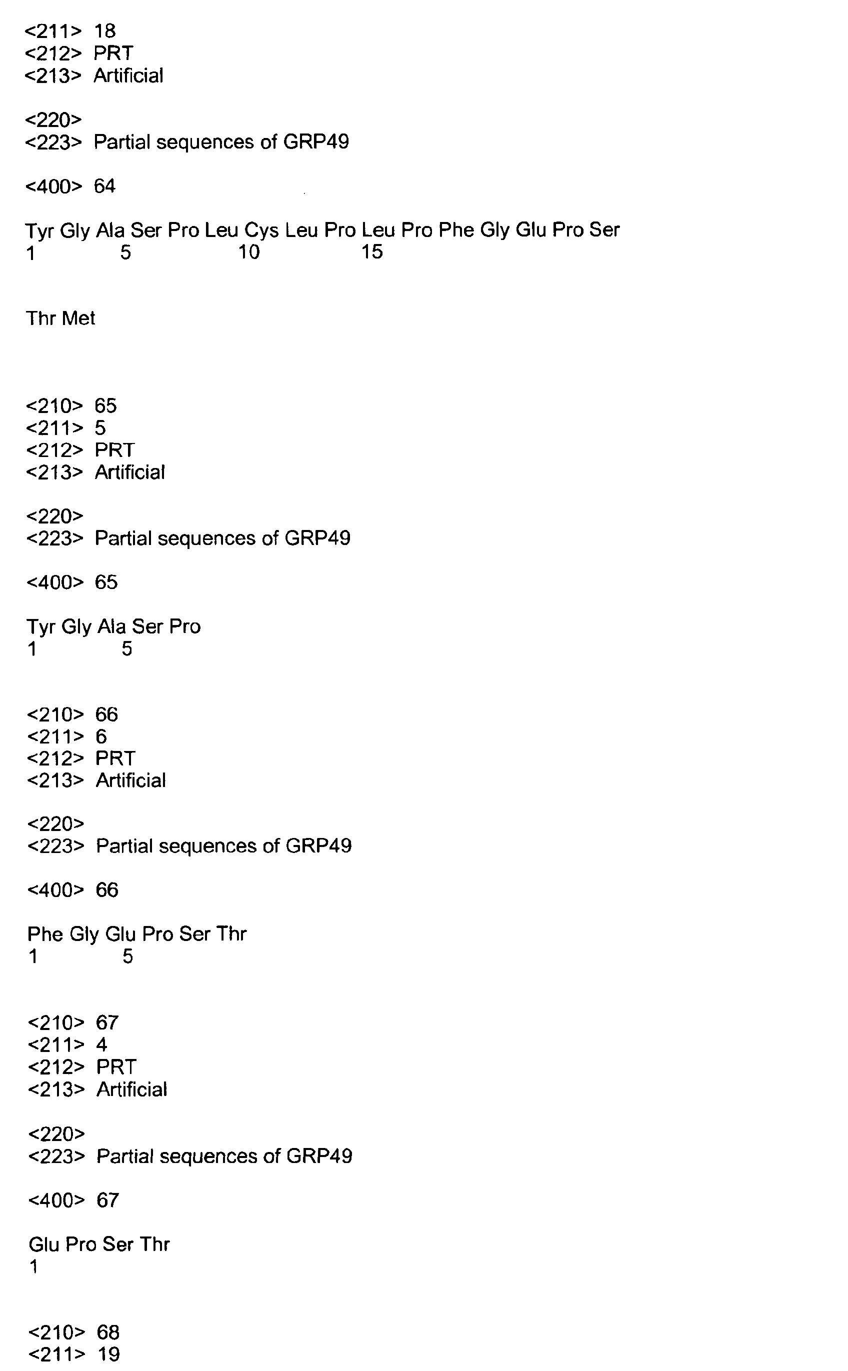

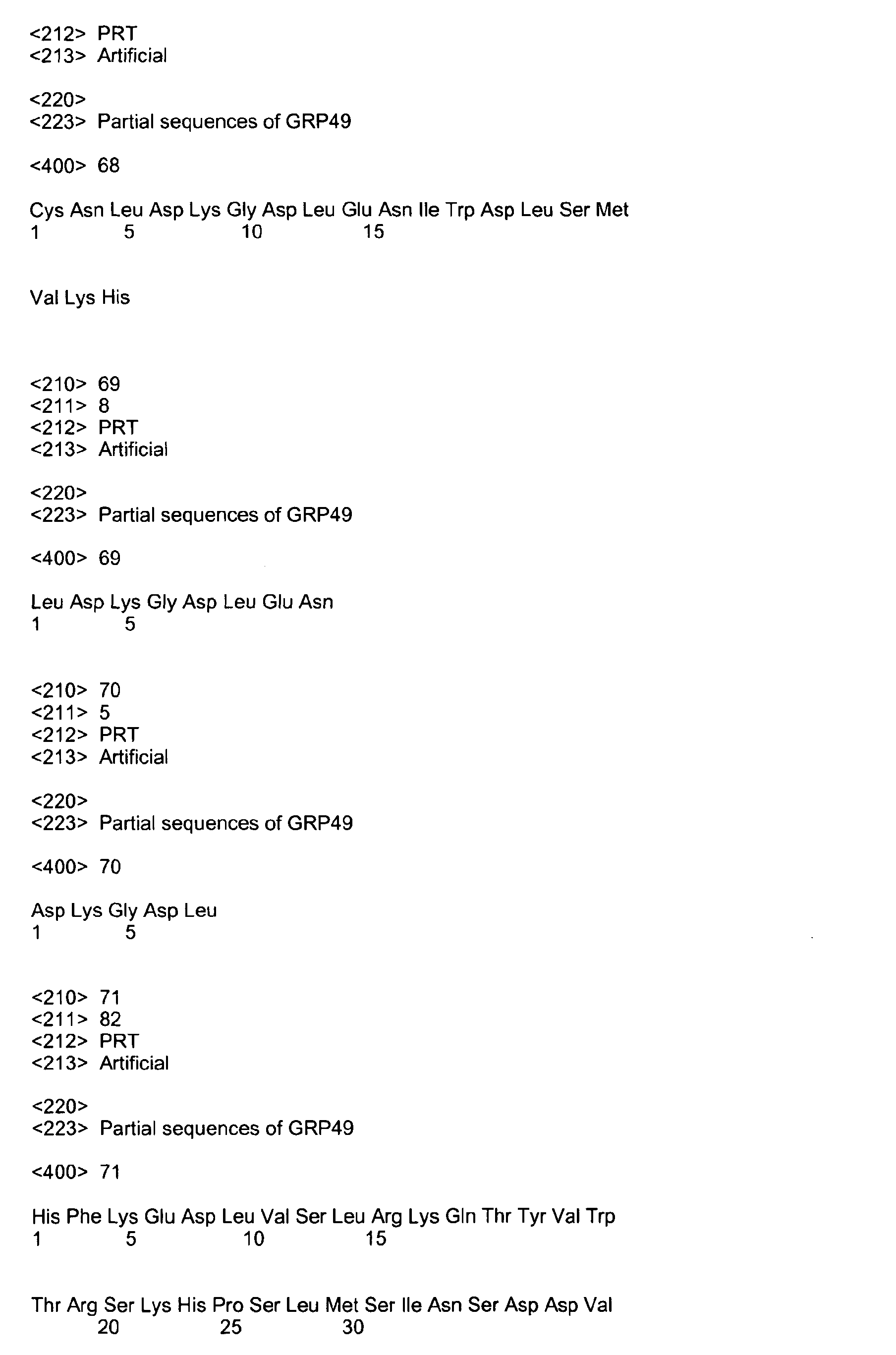

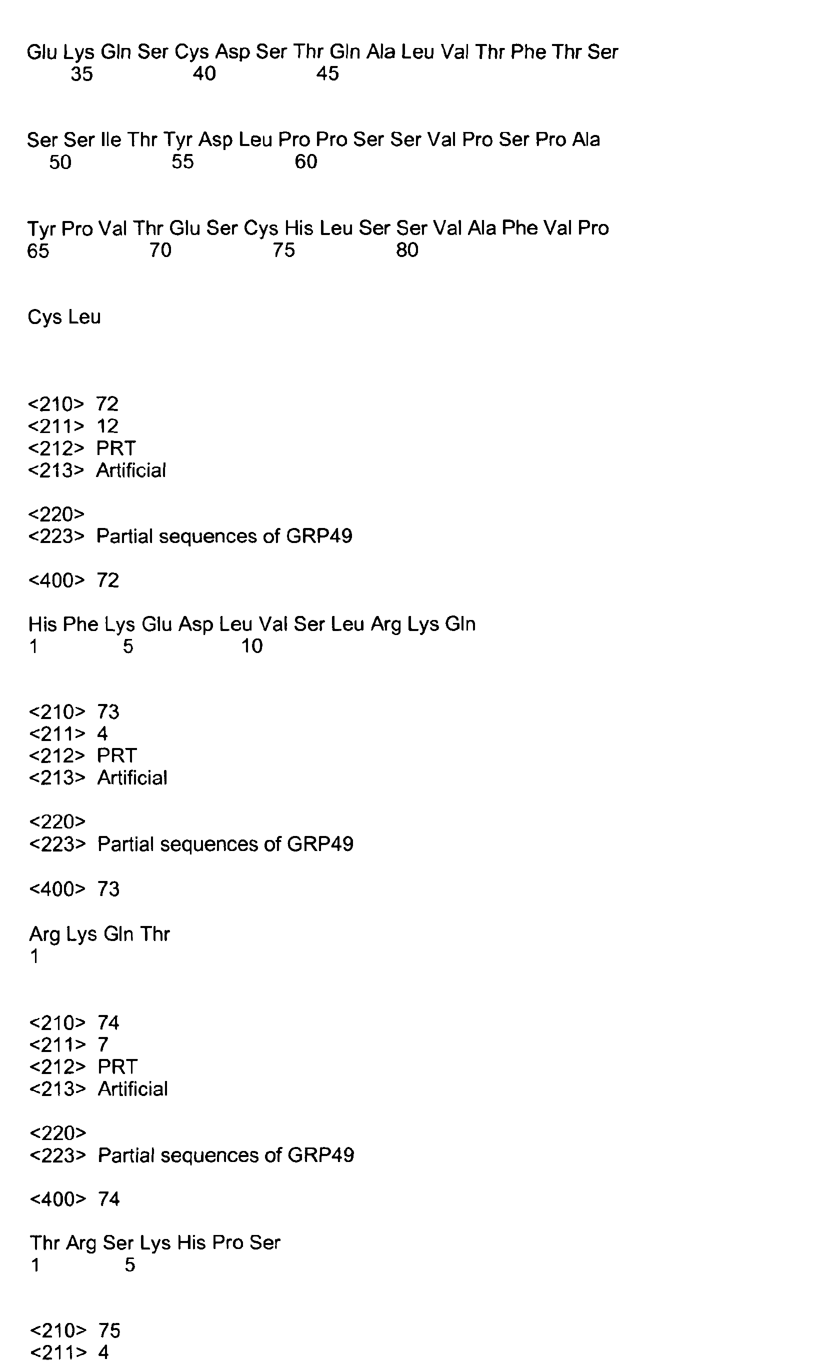

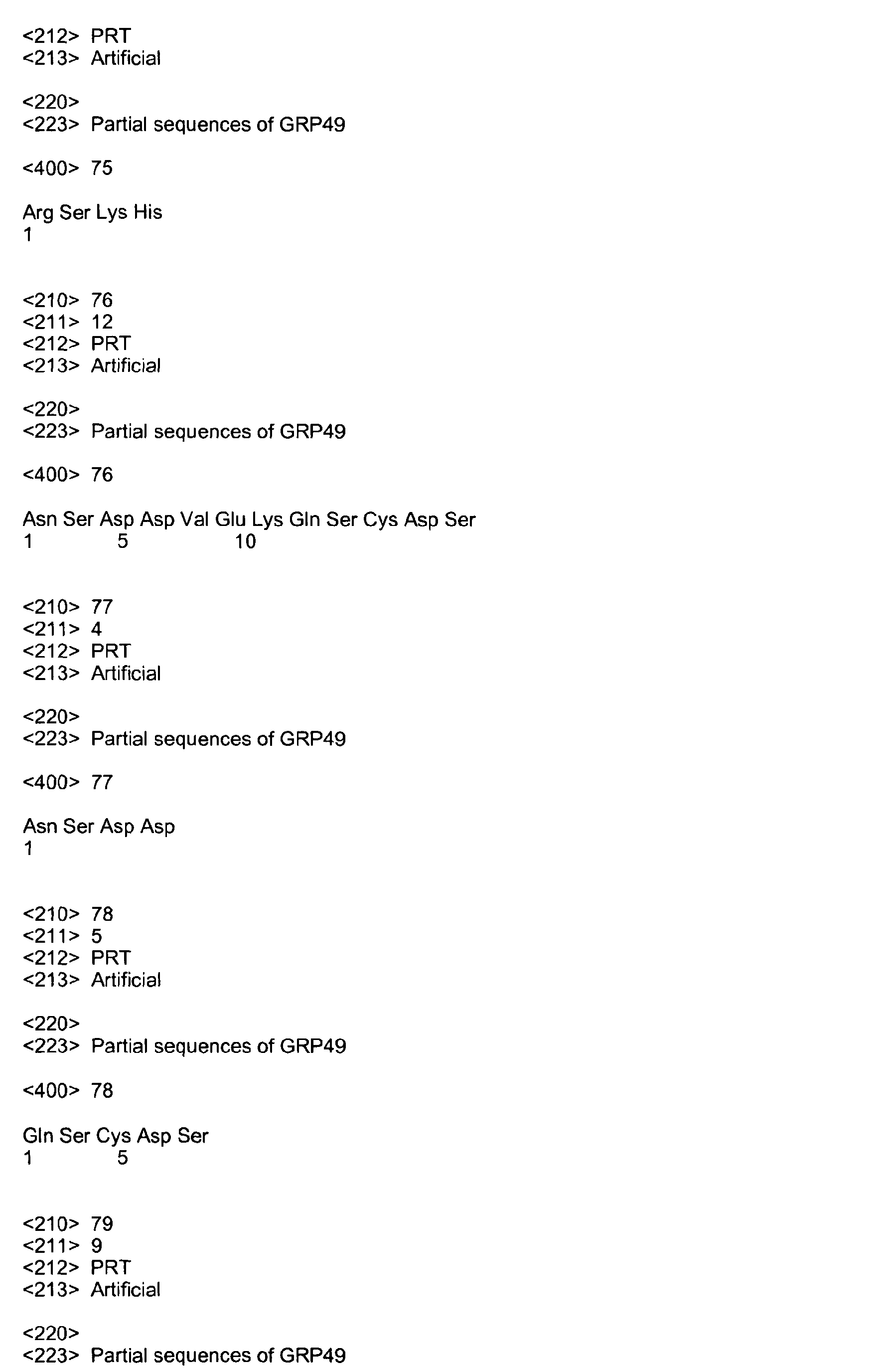

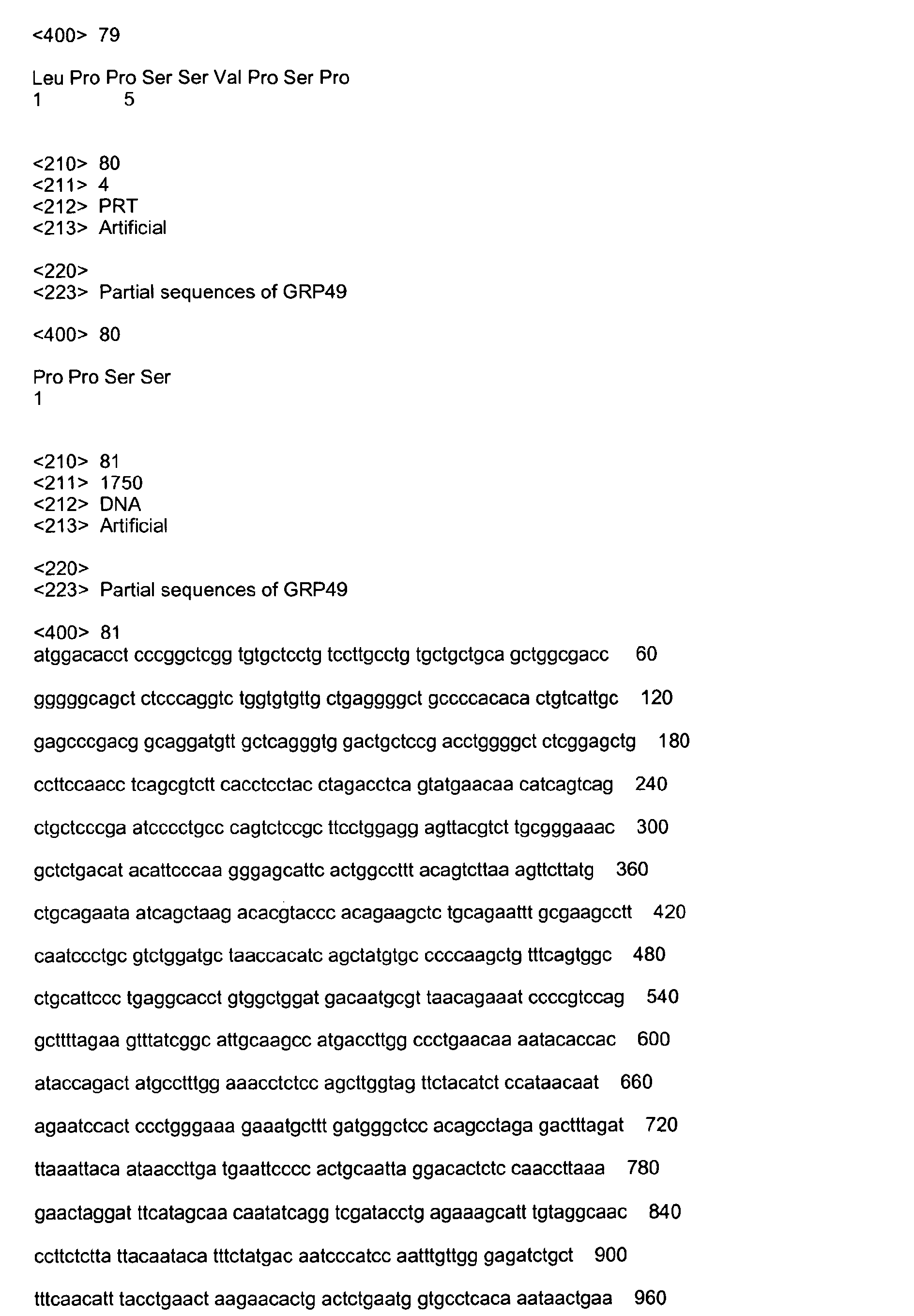

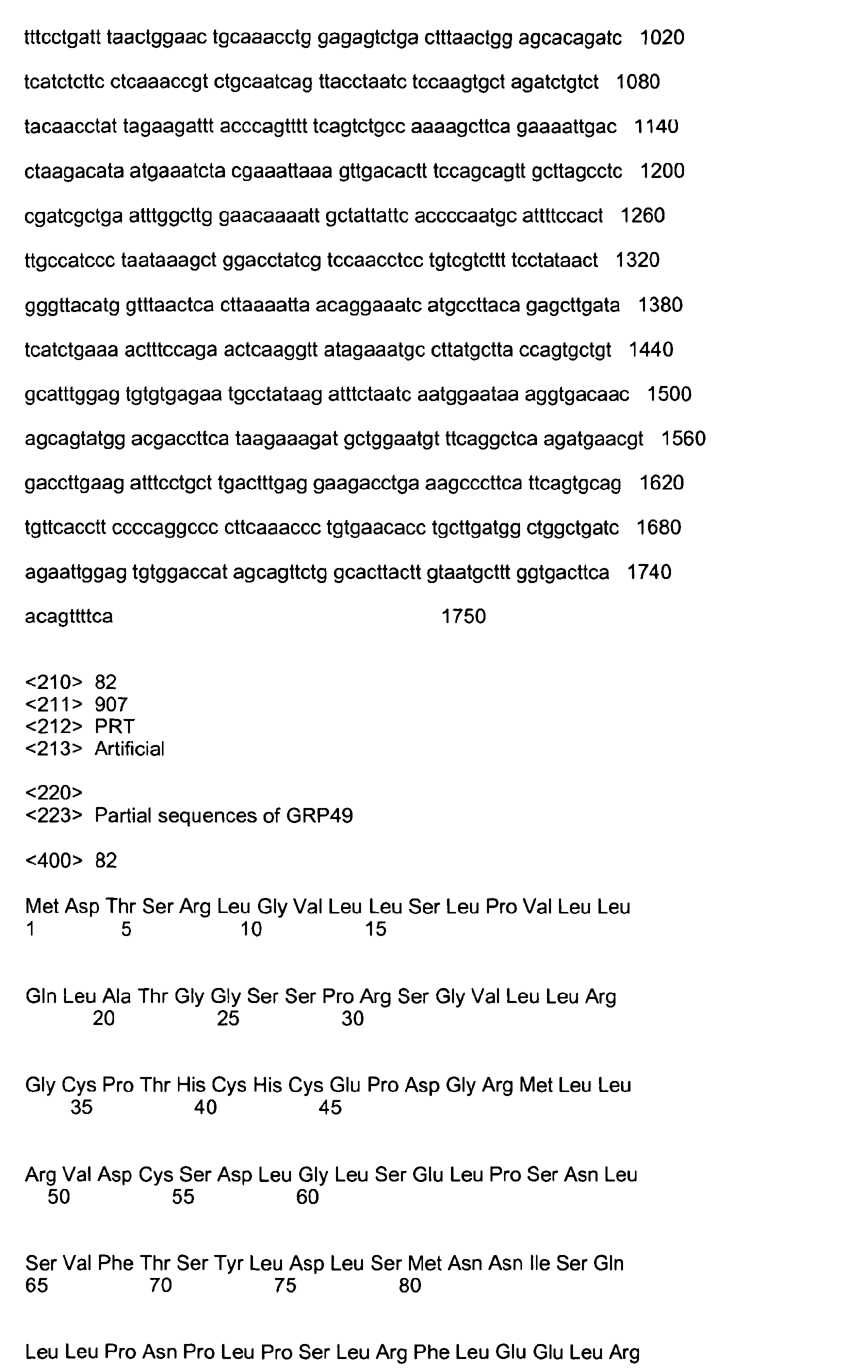

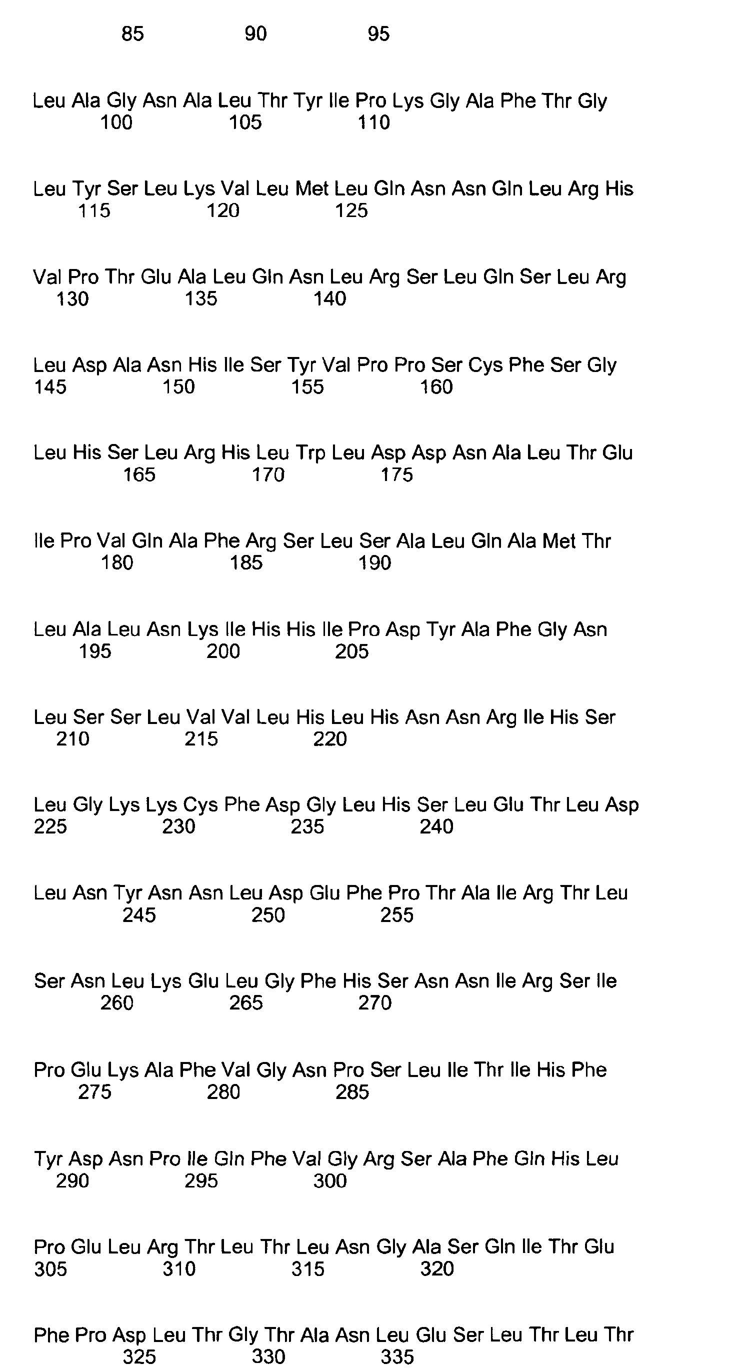

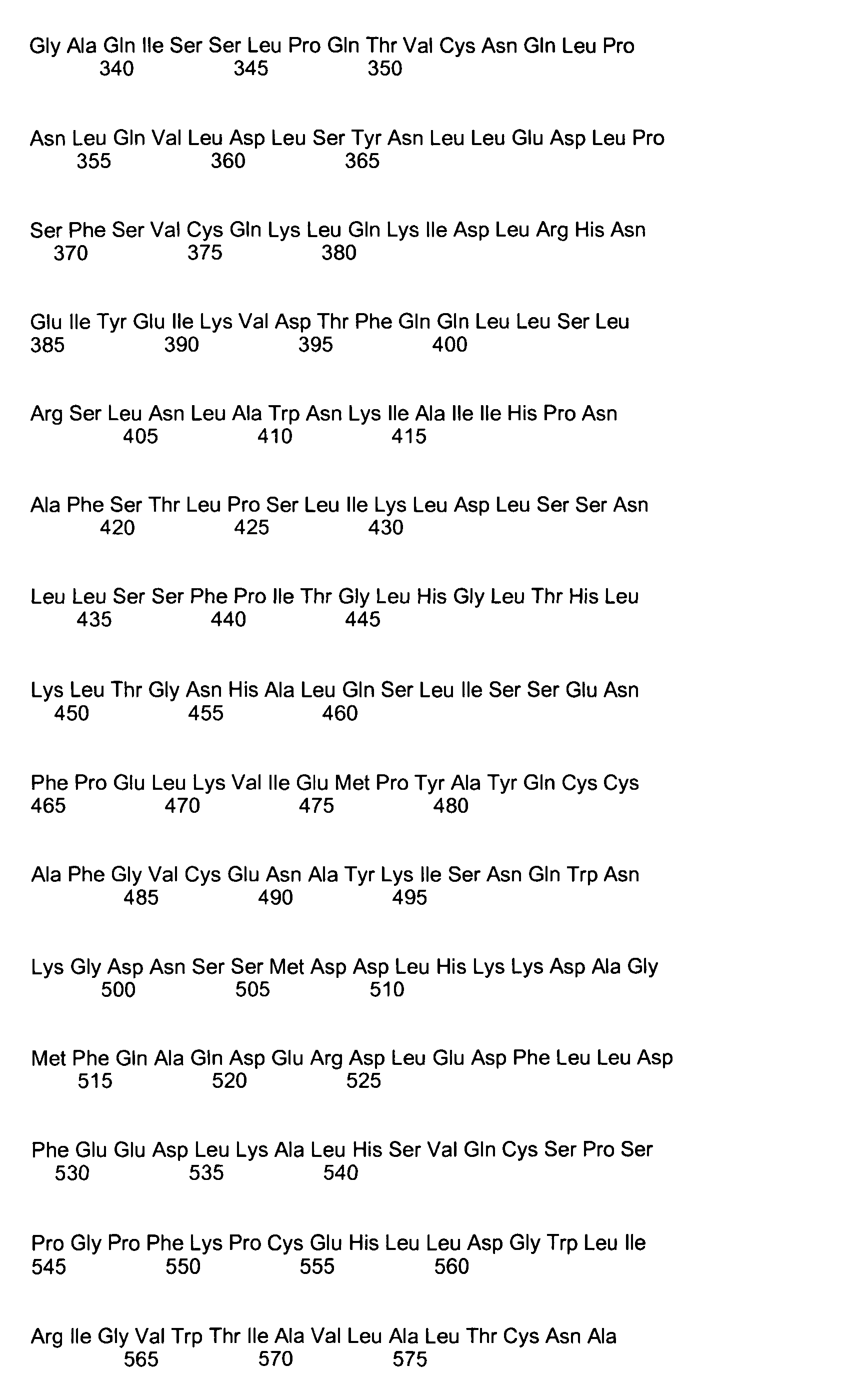

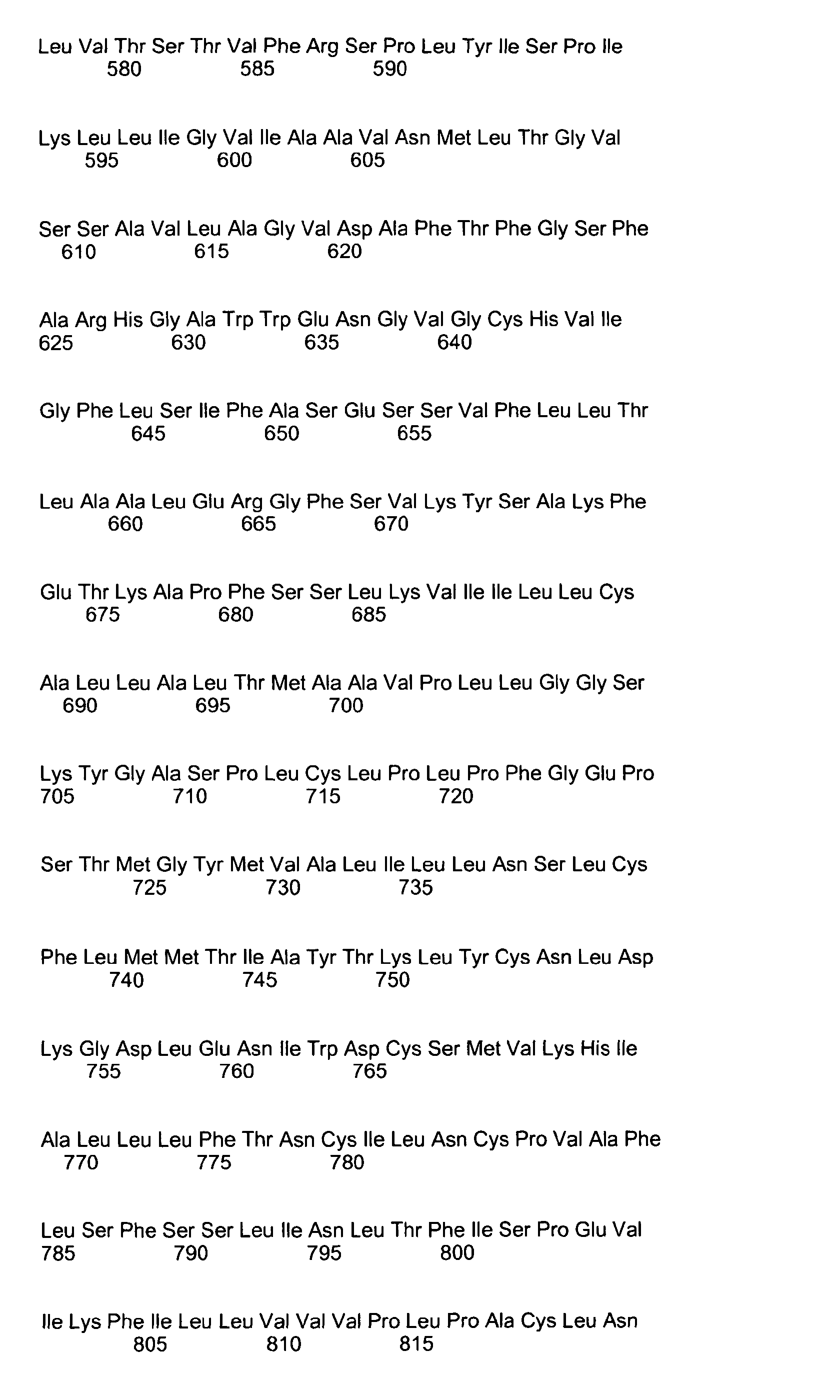

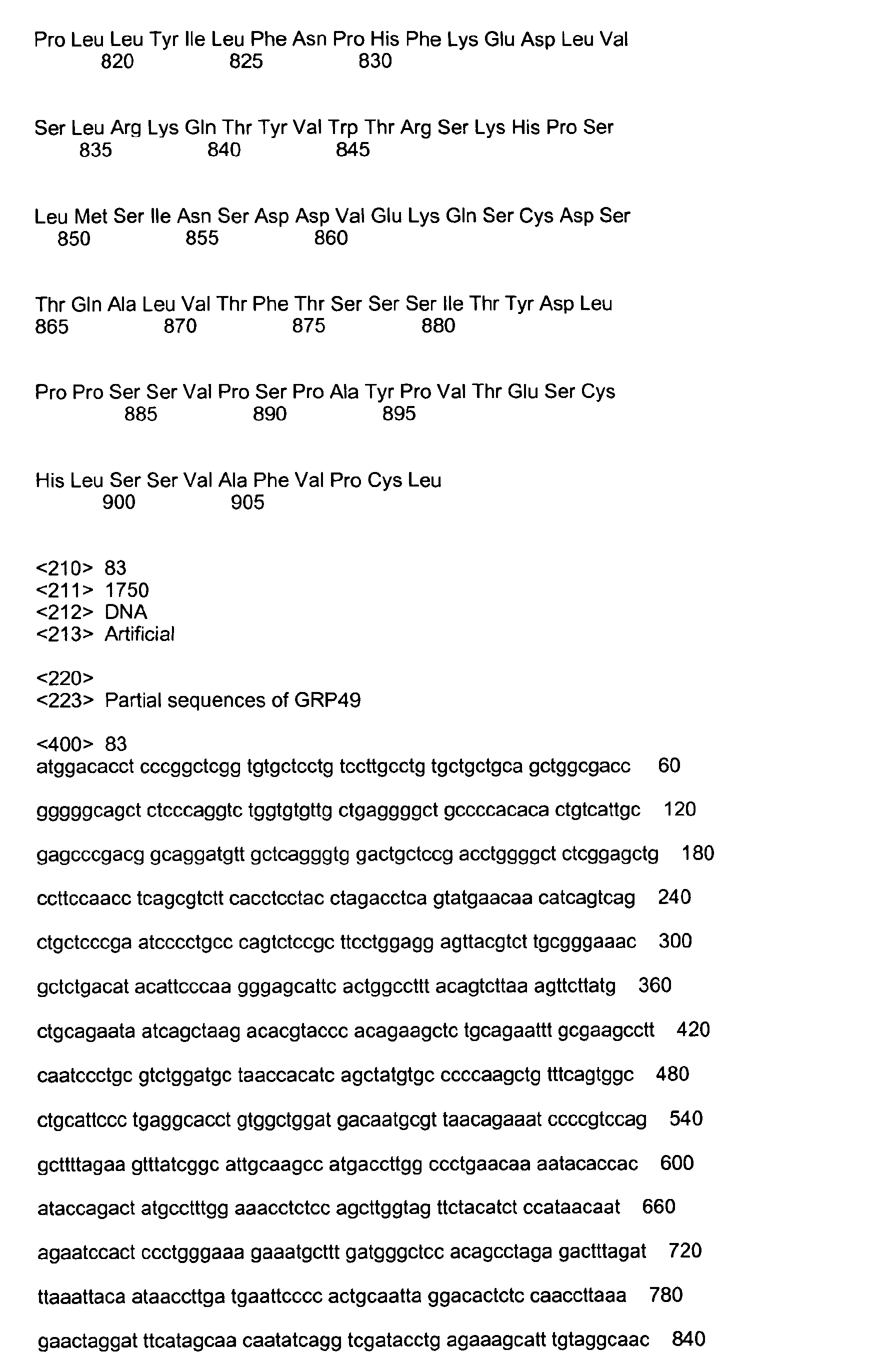

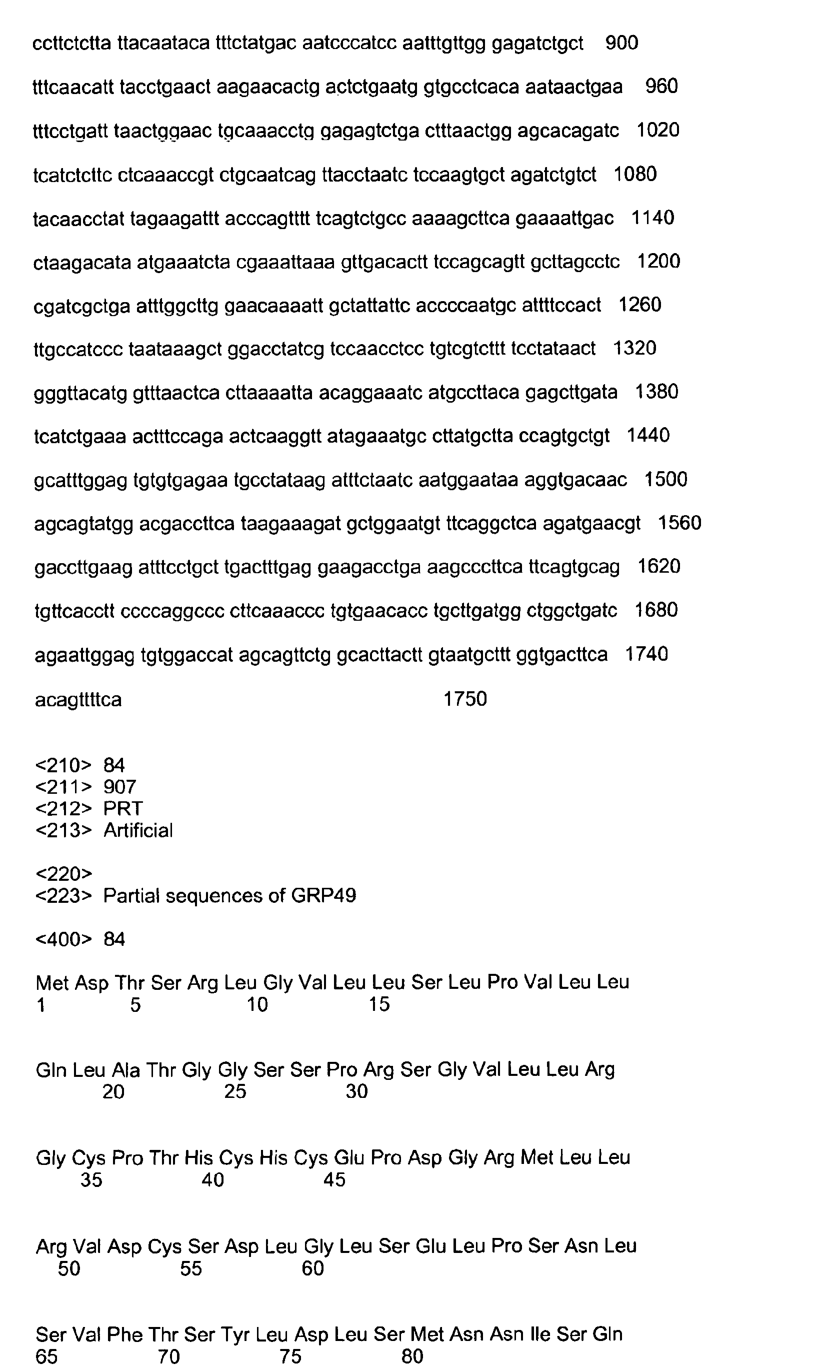

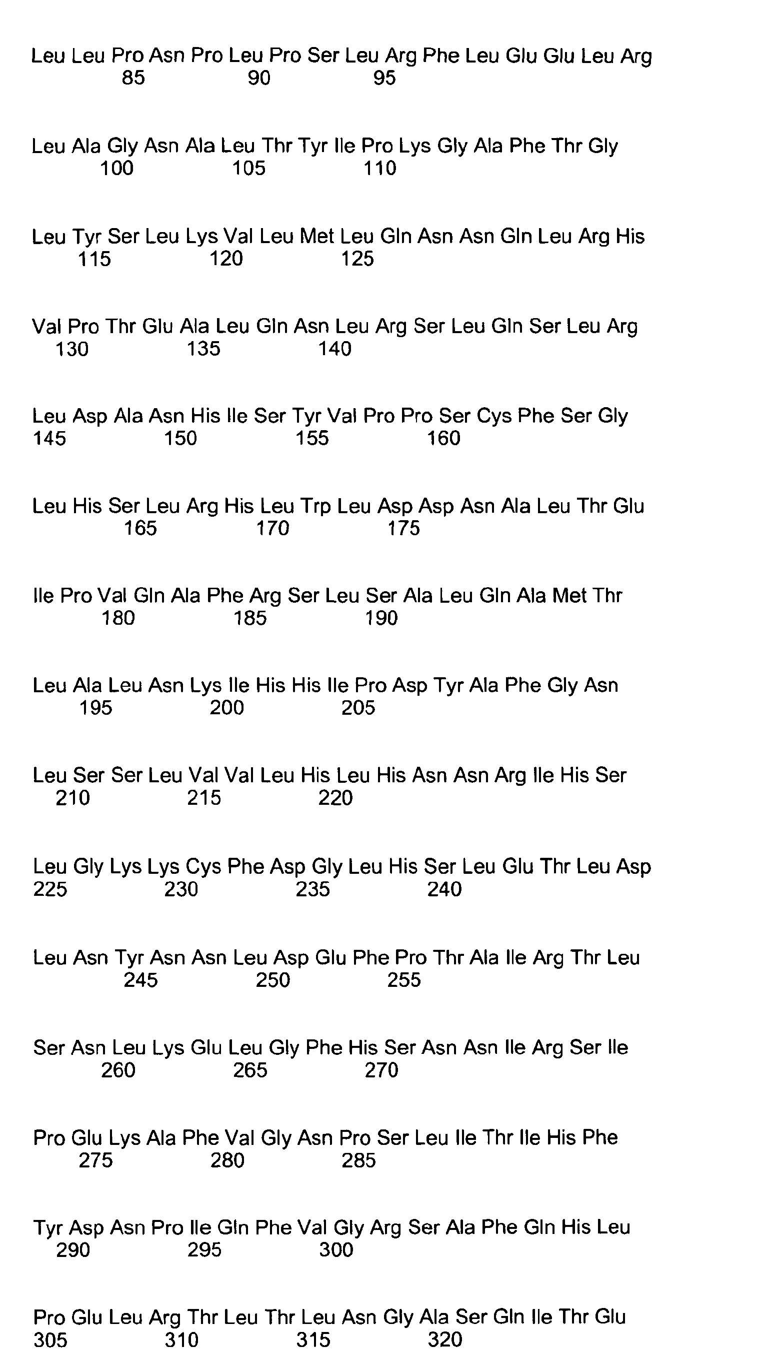

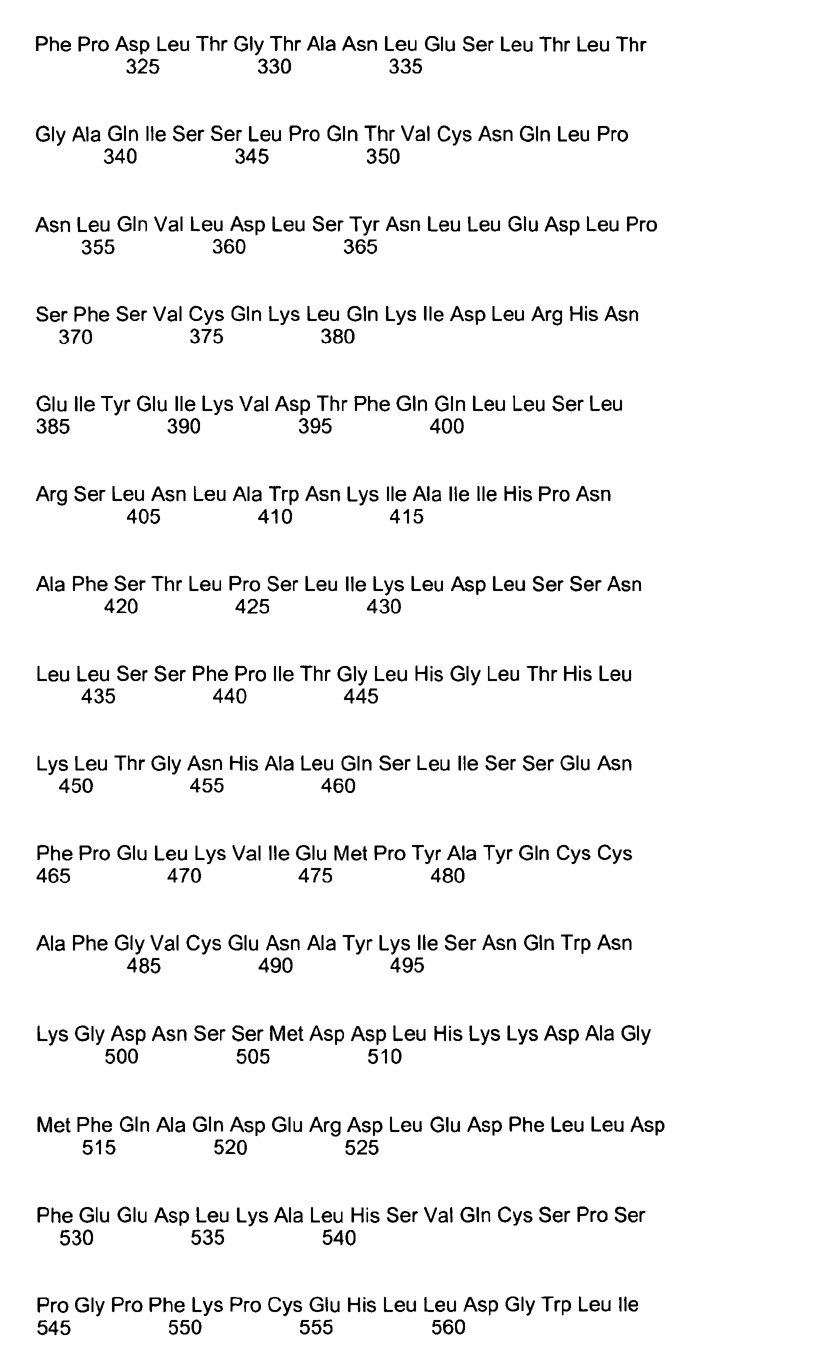

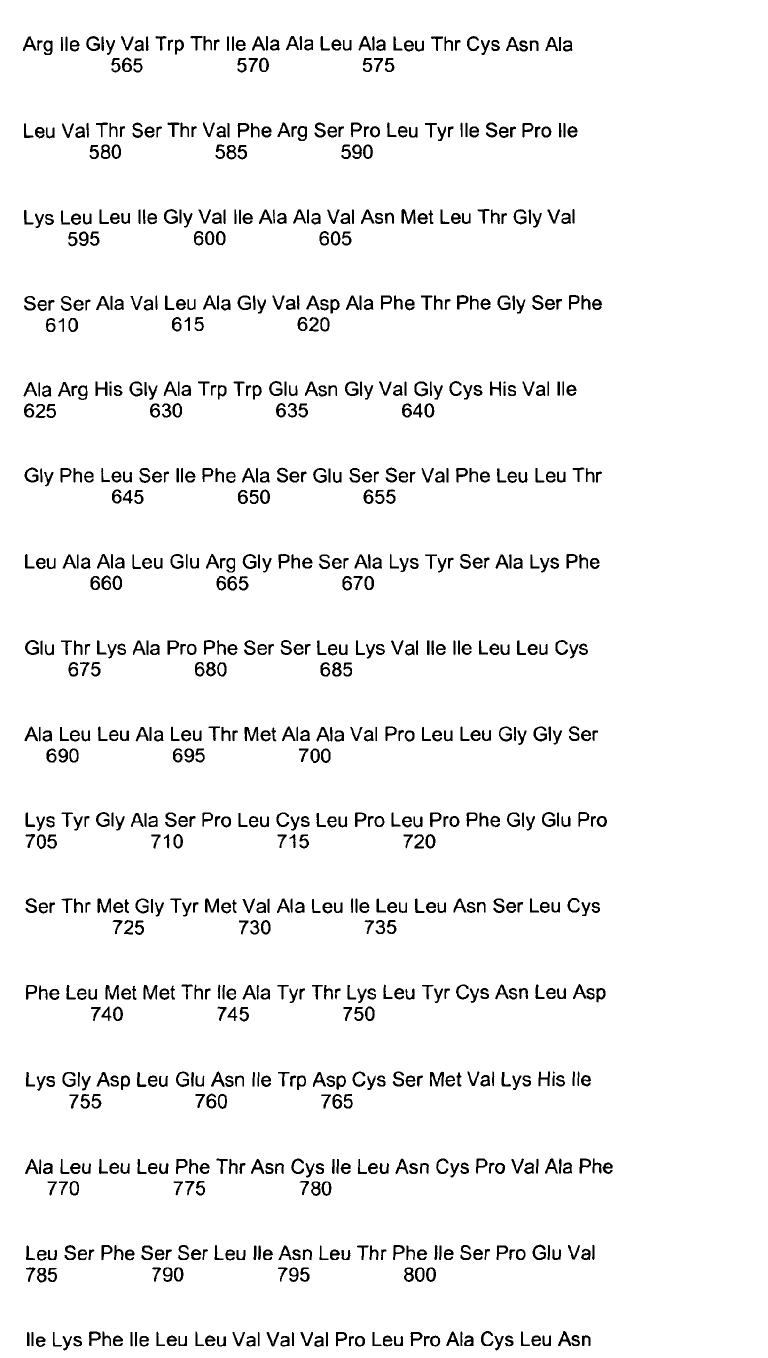

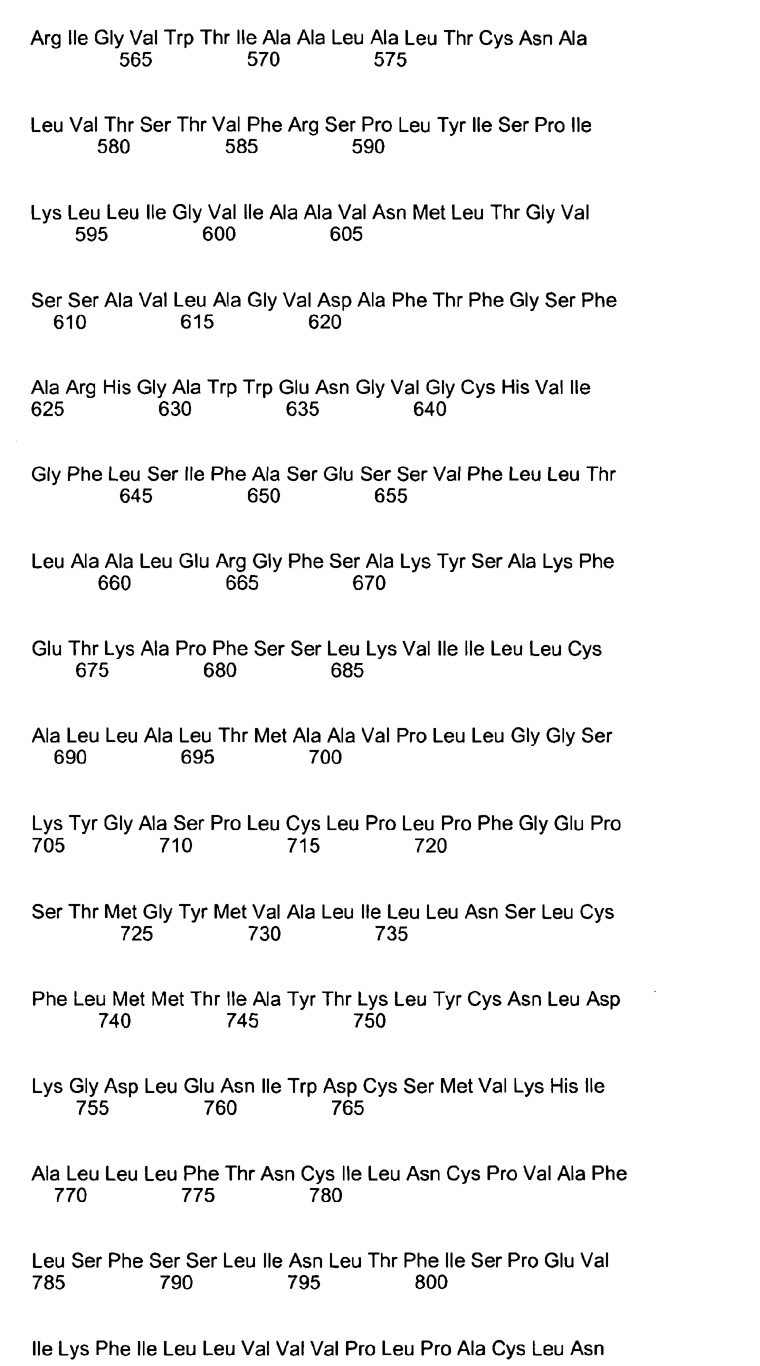

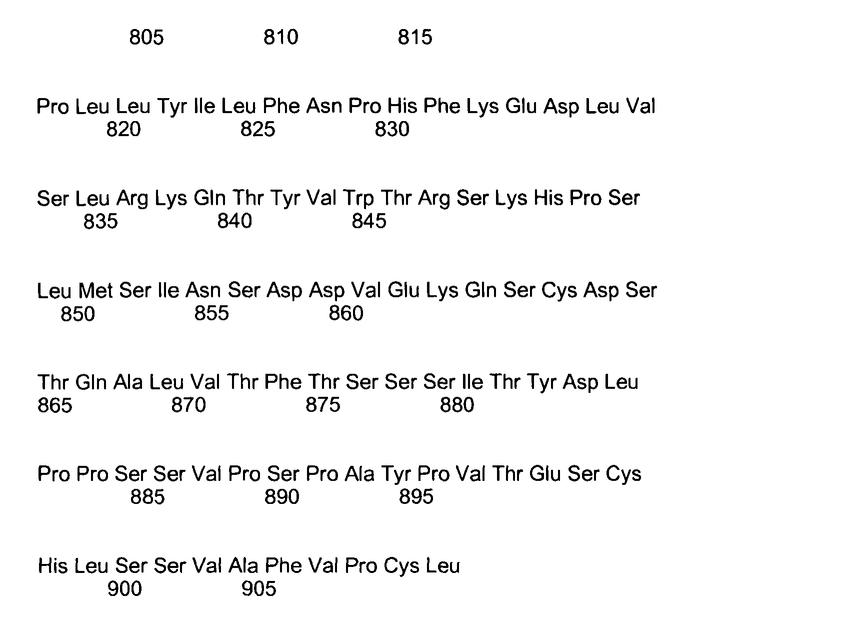

## Patentansprüche

1. Verwendung einer für ein GPR49 Peptid oder Protein codierenden Nukleinsäure und/oder eines GPR49 Peptids oder Proteins zur Detektion von Krebs, insbesondere Colon-, Uterus- und/oder Rectumtumoren, oder zur Detektion eines Risikos der Erkrankung an einem solchen Tumor, wobei eine Gewebeprobe, insbesondere eine Colon-, Uterus- und/oder Rectum-Gewebeprobe, auf Transkription oder Übertranskription von GPR49 RNA oder auf Expression oder Überexpression eines GPR49 Proteins untersucht wird.

2. Verwendung nach Anspruch 1, wobei eine an für GPR49 codierende Nukleinsäure oder eine an GPR49 Protein oder Peptid bindende Detektorsubstanz, vorzugsweise enthaltend eine Reportergruppe, verwendet wird, wobei Bindung besagter Nukleinsäure und/oder besagten Proteins oder Peptids an die Detektorsubstanz halbquantitativ oder quantitativ detektiert wird.

3. Verwendung einer GPR49 RNA oder eines GPR49 Proteins oder Peptids zum Screenen nach daran bindenden Substanzen, insbesondere nach prospektiven Wirkstoffen zur Inhibierung von besagter RNA oder besagtem Protein oder Peptid oder nach prospektiven Detektorsubstanzen, wobei eine prospektive Substanz oder eine Mischung solcher prospektiver Substanzen mit besagter RNA oder besagtem Protein oder Peptid kontaktiert wird, wobei mit einem Bindungsassay Bindungsereignisse festgestellt werden, und wobei eine bindende prospektive Substanz, ggf. nach Dekonvolutierung, selektiert wird.

4. Verwendung einer GPR49 inhibierenden oder daran bindenden Substanz zur Herstellung einer pharmazeutischen Zusammensetzung zur Behandlung von Krebs, insbesondere von Colon-, Uterus- und/oder Rectumtumoren, oder zur Herstellung einer pharmazeutischen Zusammensetzung zur Diagnose eines solchen Tumors oder zur Diagnose eines Progressionsrisikos eines solchen Tumors.

5. Verwendung nach Anspruch 4, wobei die Substanz ein Antikörper ist, welcher beispielsweise durch Immunisierung eines nicht-menschlichen Säugetiers mit einem GPR49 Peptid oder Protein, mit GPR49 transfizierten Zellen, oder einer hierfür für codierenden cDNA, erhältlich ist, oder ein Phage-Display Antikörper ist.

6. Verwendung nach Anspruch 4, wobei die Substanz eine Mimikriverbindung eines Antikörpers gegen ein GPR49 Peptid oder Protein ist.

7. Verwendung nach Anspruch 4, wobei die Substanz, ein Aptamer, eine antisense RNA, eine siRNA, oder ein Ribozym ist.

8. Verwendung nach einem der Ansprüche 4 bis 7, wobei die Substanz zusätzlich eine zytotoxische und/oder immunstimulierende Komponente trägt.

9. Verwendung nach einem der Ansprüche 4 bis 8, wobei die pharmazeutische Zusammensetzung zur lokalen Applikation in Tumorzellen enthaltendem Gewebe hergerichtet ist.

10. Verfahren zur Diagnose einer Krebserkrankung, insbesondere eines Colon-, Uterus- und/oder Rectumkarzinoms, oder des Risikos der Erkrankung an einem solchen Rumor, wobei eine an GPR49 bindende Detektorsubstanz in einer Ausführungsform mit einer Reportergruppe in zu untersuchendes Gewebe appliziert wird, wobei das zu untersuchende Gewebe dann einer Detektionsverfahrenstufe unterworfen wird, welche sensitiv für die Reportergruppe ist, und wobei im Fall der Detektion eines definierten Mindestwertes der Reportergruppe im Gewebe das Gewebe als Tumorzellen enthaltend oder als erkrankungsgefährdet qualifiziert wird.

11. Verfahren zur Behandlung von Krebs, insbesondere eines Colon-, Uterus- und/oder Rectumkarzinoms, wobei eine pharmazeutische Zusammensetzung nach einem der Ansprüche 4 bis 9 in einer physiologisch wirksamen Dosis und galenisch für die anzuwendende Darreichungsform hergerichtet einem Patienten dargereicht wird.

12. Protein oder Peptid enthaltend oder bestehend aus einer Sequenz Seq.-ID 1, 2, 4 bis 80, oder 83, oder einer Teilsequenz der Mindestlänge von 4, 5, 6, 7, 8, 9, oder 10 AS aus besagten Sequenzen, nicht jedoch enthaltend oder bestehend aus Seq.-ID 82, oder Nukleinsäure codierend für ein vorstehend definiertes Protein oder Peptid.
